# EUROPÄISCHE PATENTANMELDUNG

(11) **EP 1 757 608 A1**
(43) Veröffentlichungstag der Anmeldung: **28.02.2007**
(21) Anmeldenummer: 06025028.9
(22) Anmeldetag: 21.04.1997
(51) Int. Cl.: C07D 491/10, A61K 31/55, C07D 491/14, A61P 25/28, C07D 317/00, C07D 223/00

(54) **Neue Benzazepinderivate, diese enthaltende Arzneimittel und Verwendung derselben zum Herstellen von Arzneimitteln**

(30) Priorität: 19.04.1996 AT 71696
(62) Teilanmeldung aus: 97916263.3
(71) Anmelder: Sanochemia Pharmazeutika Aktiengesellschaft, 1090 Wien (AT)
(72) Erfinder: Czollner, Laszlo, 2491 Neufeld (AT); Fröhlich, Johannes, 1050 Wien (AT); Jordis, Ulrich, 1190 Wien (AT); Küenburg, Bernhard, 1190 Wien (AT)
(74) Vertreter: Dungler, Karin

(57) **Zusammenfassung**

Es werden neue Derivate des Benzazepins, insbesondere des Benzofuro[3a,3,2,ef][2]benzazepins der allgemeinen Formel (I) 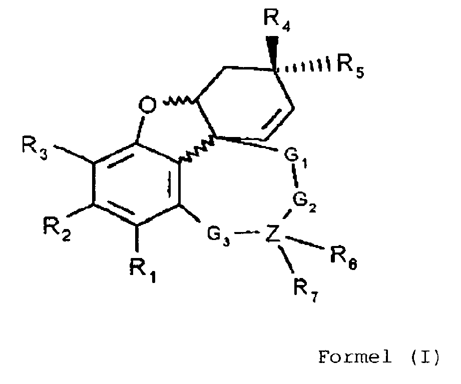 sowie auch Arzneimittel enthaltend diese Verbindungen beschrieben, die zur Behandlung der Alzheimer'schen Krankheit und verwandter Demenzzustände sowie für die Behandlung des Langdon-Down-Syndroms erfolgreich eingesetzt werden können.

## Beschreibung

Die Erfindung betrifft neue Verbindungen und Arzneimittel enthaltend die neuen Verbindungen als pharmazeutische Wirkstoffe.

Die Erfindung bezieht sich in gleicher Weise auf die Verwendung der neuen Verbindungen zur Herstellung von Arzneimitteln für die Behandlung der Alzheimer'schhen Krankheit und verwandter Demenzzustände, sowie für die Behandlung des Langdon-Down-Syndroms (Mongolismus, Trisomie 21).

Die Säureadditionssalze von Galanthamin, das die chemische Strukturformel 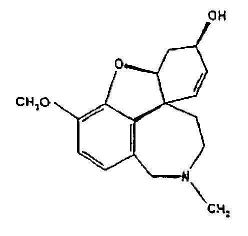 hat, sowie einige seiner Analoga sind als pharmazeutische Wirkstoffe mit inhibitorischer Wirkung auf das synaptische Enzym Acetylcholinesterase bekannt. Galanthamin wird daher bei Lähmungserscheinungen im Gefolge von Poliomyelitis und bei verschiedenen Erkrankungen des Nervensystems pharmakologisch angewandt.

Galanthamin und einige seiner Derivate werden auch bei der symptomatischen Behandlung der Alzheimer'sehen Krankheit und verwandter Demenzzustände eingesetzt (EP 236 684 B1).

Galanthamin ist chemisch gesehen ein Alkaloid der Morphingruppe, das aus Schneeglöckchen (Galanthus woronowii, G. nivalis usw.) und anderen Amaryllidaceen gewonnen werden kann.

Neben der Gewinnung von Galanthamin aus pflanzlichen Quellen sind auch chemische Syntheseverfahren für Galanthamin und dessen Analoga einschließlich ihrer Säureadditionssalze bekannt geworden (WO 95/27715).

Das Down-Syndrom ist auf eine Verdreifachung des Chromosoms Nr. 21 zurückzuführen, d.h. die Patienten haben einen Satz von 47 statt 46 Chromosomen, was zytologisch relativ einfach nachzuweisen ist. Trisomie 21 ist mit mittelgradiger bis schwerer geistiger Behinderung und einer Reihe körperlicher Dysmorphiezeichen verbünden. Eine ursächliche Therapie ist derzeit nicht möglich. Die bestehenden Behinderungen lassen sich durch gezielte therapeutische Maßnahmen beeinflussen, jedoch bleibt eine Hilfsbedürftigkeit in der Regel bestehen.

Die neuen Verbindungen gemäß der Erfindung sind neue Benzazepin-Derivate, insbesondere Derivate des Benzofuro[3a,3,2,ef] [2]benzazepines.

Die neuen Verbindungen gemäß der Erfindung sind neue Benzazepin-Derivate, insbesondere Derivate des Benzofuro[3a,3,2,ef] [2]benzazepines.

Es handelt sich um Verbindungen der allgemeinen Formel (I) 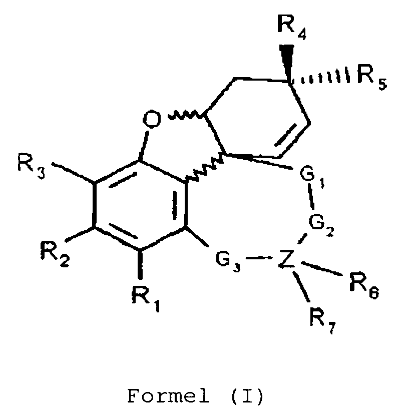 wobei
R₁, R₂ entweder gleich oder verschieden sind und
• Wasserstoff, F, Cl, Br, J, CN, NC, OH, SH, NO₂, SO₃H, NH₂, CF₃ oder eine niedere (C₁-C₆) gegebenenfalls verzweigte, gegebenenfalls substituierte (Ar)Alkyl- oder (Ar)Alkyloxygruppe oder
• eine Aminogruppe, die durch ein oder zwei gleiche oder verschiedene niedere (C₁-C₆), gegebenenfalls verzweigte, gegebenenfalls substituierte (Ar)Alkyl- oder (Ar)Alkylcarbonyl- oder (Ar)Alkyloxy-carbonylgruppen substituiert ist oder
• eine COOH, COO(Ar)Alkyl, CONH, CON(Ar)Alkyl Gruppe oder
• -(CH₂)ₙ -Cl, -(CH₂)ₙ -Br,-(CH₂)ₙ-OH, -CH₂)ₙ -COOH, -(CH₂)ₙ -CN, -(CH₂)ₙ -NC, darstellen, wobei
• Rᵣ-R₂ auch gemeinsam als -CH=CH-CH=CH-, -O-(CH₂)ₙ-O-, mit n = 1-3 definiert sein können.
R₃ = R₁, insbesondere OH und OCH₃, weiters
R₂-R₃ gemeinsam: -O-(CH₂)ₙ-O- bilden können, wobei n = 1 - 3
R₄, R₅: entweder beide Wasserstoff oder wechselweise jede Kombination von Wasserstoff oder eines (Ar)Alkyl-, (Ar)Alkenyl-, (Ar)Alkinyl-mit
• S-R₈, wobei R₈ Wasserstoff oder eine niedere (C₁-C₁₀) gegebenenfalls verzweigte, gegebenenfalls substituierte (Ar)Alkylgruppe ist
• SO-R₈, SO₂R₈
• OH, O-Schutzgruppe (wie TMS, TBDMS),
• O-CS-N-R₈ (Thiourethane),
• O-CO-N-R₉, wobei R₉ die folgenden Bedeutungen hat: 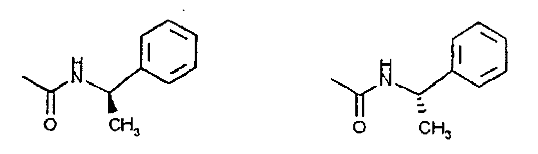
• O-CO-R₈ (Ester, R₈ siehe oben), insbesondere auch Ester mit dem Substitutionsmuster von Aminosäuren, wie 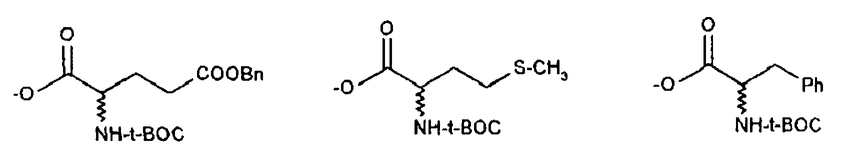
• weiters : R₄, R₅ = gemeinsam Hydrazone (=N-NH-R₁₀, =N-N(R₁₀, R₁₁), Oxime (=N-O-R₁₁) wobei R₁₀ Wasserstoff, eine niedere (C₁-C₆) gegebenenfalls verzweigte, gegebenenfalls substituierte (Ar)Alkyl- oder (Ar)Alkylcarbonyl -oder (Ar)Alkylcarbonyloxygruppe sowie Sulfonsäure- wie z.B. Tosyl und Mesylgruppe ist und R₁₁ Wasserstoff, eine niedere (C₁-C₆), gegebenenfalls verzweigte, gegebenenfalls substituierte (Ar)Alkyl- oder (Ar)Alkylcarbonylgruppe sowie Sulfonsäure- wie z.B. Tosyl- und Mesylgruppe ist.
• sowie Substituenten vom Typ: 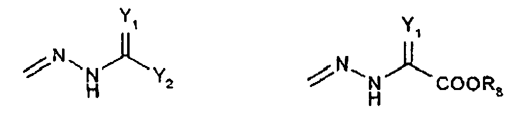 Y₁ , Y₂ = O, S, NH oder N-R₁₀ (überzählige Valenzen sind jeweils -H)
• wobei für den Fall, dass R₄ ≠ H darstellt R₅ auch OH bzw. für den Fall dass R₅ ≠ H darstellt R₄ auch OH sein kann.
G₁, G₂: gemeinsam oder verschieden die Bedeutung haben:
- -C(R₁₃, R₁₄) -, wobei R₁₃, R₁₄ Wasserstoff, OH, eine niedere gegebenenfalls verzweigte, gegebenenfalls substituierte (Ar)Alkyl-, Aryl, (Ar)Alkyloxy- oder Aryloxygruppe oder gemeinsam eine Alkylspirogruppe (C₃ bis C₇- Spiroring) sein können.
- Weiters G₁ und G₂ gemeinsam 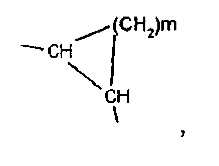 mit m = 1 bis 7 darstellt.
G_{3:} -CH₂- oder =CO darstellt.
R₆ eine Gruppe -(G₄)ₚ - (G₅)_{q} - G₆ mit p,q = 0 - 1 darstellt, in der G₄ folgende Definitionen erfüllt:
- (CH₂)ᵣ-, -C(R₁₅,R₁₆)-(CH₂)ᵣ-, mit r = 1-6 und R₁₅, R₁₆ = Wasserstoff, niedere, gegebenenfalls verzweigte, gegebenenfalls substituierte (Ar)Alkyl-, Cycloalkyl-, Arylgruppe,
- -O-, oder -NR₁₅- 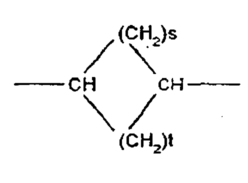 mit s = 1-4, t = 0-4 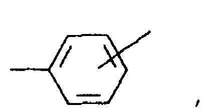 also ein ortho, meta, oder para disubst. Aromat 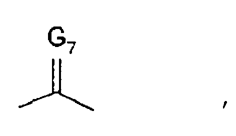 wobei G₇ = NR₁₅, 0 oder S darstellt,
G₅ gleich oder verschieden von G₄ sein kann und für den Fall, dass p = 1 ist zusätzlich -S- darstellt,
G₆ folgende Definitionen erfüllt: 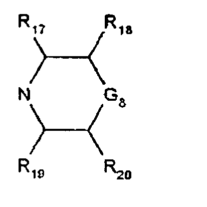 mit
- P₁₇, R₁₈, R₁₉, und R₂₀ sind einzeln oder gemeinsam, gleich oder unterschiedlich Wasserstoff, niedere, gegebenfalls verzweigte, gegebenenfalls substituierte (Ar)Alkyl-, Cycloalkyl-, oder Arylgruppen, wobei R₁₇ und R₁₈ bzw. R₁₉ und R₂₀ gemeinsam eine Cycloalkylgruppe (Ringgröße 3-8) bilden können.
- G₈ = O, S, NH, NR₂₁, -(CH₂)ₙ-,
- R₂₁ = CHO, COOR₇, oder ein unsubstituierter, oder durch eine oder mehrere mehrere F, Cl, Br, J, NO₂, NH₂, OH, Alkyl, Alkyloxy, CN, NC oder CF₃, CHO, COOH, COOAlkyl, SO₃H, SH, S-Alkyl - Gruppen gleich oder unterschiedlich substituierter (Hetero)Arylrest, (mit Heteroaryl insbesondre 2-Pyridyl, 4-Pyridyl, 2-Pyrimidinyl) oder
- eine Methylgruppe, welche durch 1-3 unsubstituierte, oder durch ein oder mehrere F, Cl, Br, J, NO₂, NH₂, Alkyl, Alkyloxy, CN, NC oder CF₃ Gruppen gleich oder verschieden substituierte Phenylgruppe(n) substituiert ist,
G₆ kann weiters sein: 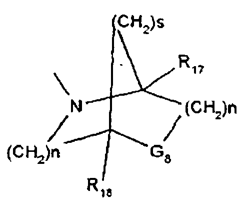 bzw. 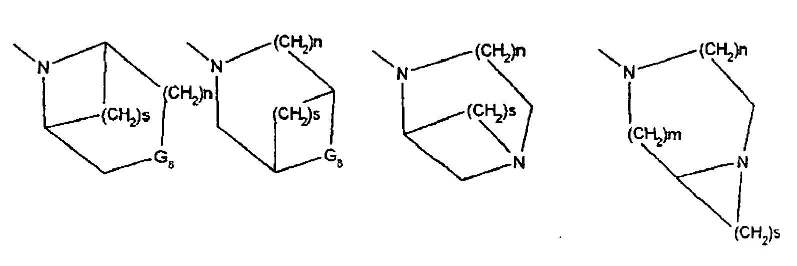 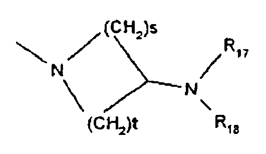
• -CHO, COOR₁₇, -CONR₁₇
• eine niedrige, gegebenenfalls verzweigte, gegebenenfalls substituierte (Ar)Alkyl-, (AR)Alkenyl-, (AR)Alkinyl-, Cycloalkyl-, oder Arylgruppe,
• -O-R₁₇, -NR₁₇R₁₈, Phthalimido, -CN, oder -NC.
R₇ ist gleich R₆ oder stellt -O⁽⁻⁾ (N-Oxid) oder ein freies Elektronenpaar (e-Paar) dar, wobei R₆ und R₇ auch einen gemeinsamen Ring der Größe 3-8 bilden können, und
• [X] nur dann existiert und ein Ion einer pharmakologisch verwendbare anorganischen und organischen Säure darstellt, wenn R₅ und R₆ vorhanden sind und somit der Stickstoff eine positive Ladung trägt.
• Z = N, bzw. N⁺ für den Fall, dass R₆ und R₇ gemeinsam vorhanden sind und R₇ ungleich O⁻ ist.
Einen Sonderfall der neuen Verbindungen der allgemeinen Formel (I), sind die Verbindungen der allgemeinen Formel (II) 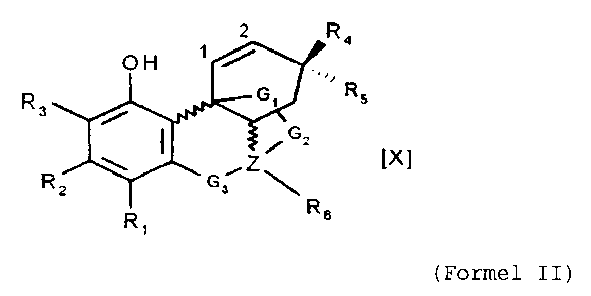 wobei die Reste die bei Formel (I) beschriebenen Bedeutungen haben. Diese Formel entsteht formal aus Formel (I), indem die Bindung von C₁ zum "Furan" Sauerstoff gebrochen und stattdessen von C₁ direkt an Z gebildet wird.

Weiters umfasst die Erfindung die neue, substituierte überbrückte Basen der allgemeinen Formel (III) und deren Herstellung, insbesondere 2,5-Diazabicyclo[2.2.1]heptane: 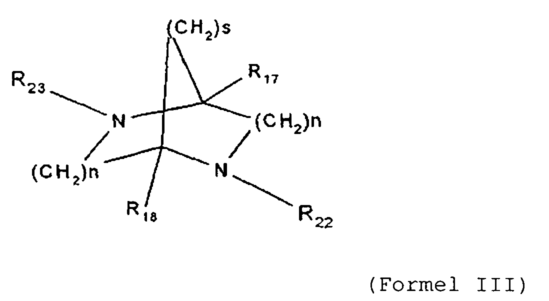 wobei R₂₂
- ein unsubstituierter, oder durch eine oder mehrere F, Cl, Br, J, NO₂, NH₂, OH, Alkyl, Alkyloxy, CN, NC oder CF₃, CHO, COOH, COOAlkyl, SO₃H, SH, S-Alkyl -Gruppen gleich oder unterschiedlich substituierter (Hetero)Arylrest oder eine Methylgruppe, welche durch 2 unsubstituierte, oder durch ein oder mehrere F, Cl, Br, J, NO₂, NH₂, OH, Alkyl, Alkyloxy, CN, NC oder CF₃, CHO, COOH, COOAlkyl, SO₃H, SH, S-Alkyl -Gruppen gleich oder verschieden substituierte Phenylgruppe(n) substituiert ist,
R₁₇, R₁₈, n, s, die bei der allgemeinen Formel (I) genannten Bedeutungen haben und
R₂₃ = - (G₅)_{q} - (G₄)ₚ - G₉
wobei G₄ und G₅ die bei der allgemeinen Formel (I) genannten Bedeutungen haben und G₉ definiert ist als:
Wasserstoff, F, Cl, Br, J, OH, O-Ts, O-Ms, O-Triflat, COOH, COCl CHO, -O-R₁₇, -NR₁₇R₁₈, Phthalimido, -CN, oder -NC oder andere für nukleophile Substitutionen, Additionsreaktionen, Kondensationsreaktion etc. geeignete Gruppen.

Beispiele für diese Verbindungstypen:

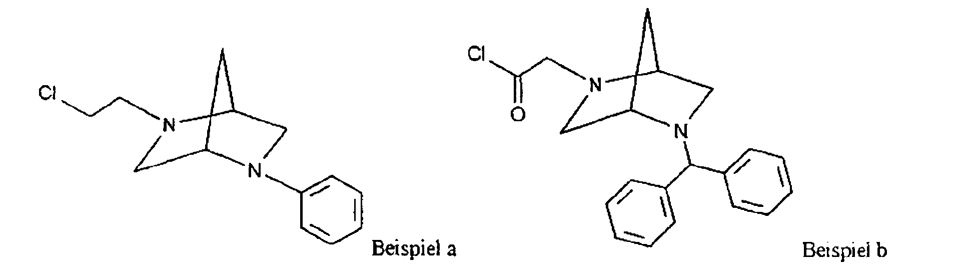

Diese Verbindungen der allgemeinen Formel (III) stellen nicht nur pharmazeutisch eine interessante Verbindungsklasse dar, sondern finden auch als Substituenten in einer Vielzahl von Grundkörpern Anwendung. Beispiele stellen die Verbindungen 105 bis 109 dar.

Die erfindungsgemäßen Arzneimittel können für die Behandlung der Alzheimer'schen Krankheit und verwandter Demenzzustände, sowie für die Behandlung des Langdon-Down-Syndroms erfolgreich eingesetzt werden.

Die Erfindung betrifft in gleicher Weise die Verwendung der vorstehend genannten Verbindungen zur Herstellung eines Arzneimittels für die Behandlung der Alzheimer'schen Krankheit und verwandter Demenzen, sowie für die Behandlung des Langdon-Down-Syndroms.

Besonders bevorzugt sind erfindungsgemäß die in der nachstehenden Übersicht genannten Verbindungen. In der Übersicht sind auch die für die Wirksamkeit mit maßgeblichen ACHE-Hemmwerte (IC⁵⁰, das ist die 50% Hemm-Konzentration) der erfindungsgemäßen Verbindungen angegeben.

Die Hemmung Acetylcholinesterase wurde nach einer modifizierten Methode von Ellmann (Lit. 44) bestimmt, wobei als Serum Humanserum aus einem pool von 10 Probanden verwendet wurde. Methode: 520 µl Lösung der Testsubstanz (es wurden Konzentrationen von 10⁻⁴ bis 10⁻⁷, in Ausnahmefällen bis 10⁻⁹ Mol/Liter verwendet) in 0.02 M Tris(hydroxymethyl)aminomethanlösung, mit HCl auf pH = 7.8 gepuffert und 400 µl m-Nitrophenollösung (Sigma Diagnostics, Art. 420-2) wurden in der Halbmikroküvette bei 37°C mit 40 µl Cholinesterase - Lösung (Sigma Diagnostics, Art. 420-MC, im Verhältnis 1:15 mit Wasser verdünnt) und 160 µl Serum inkubiert und über 5 Min. die Änderung der Absorption gegen eine Vergleichsprobe auf einem Beckmann DU-50 Spektralphotometer mit einem Kinetics Programm vermessen. Die Werte wurden in % gegen die Vergleichsprobe angegeben und aus dem Kurvenverlauf 50% Hemmkonzentration (IC⁵⁰) berechnet.

Übersicht der neuen Verbindungen vom Typ der allgemeinen Formel:

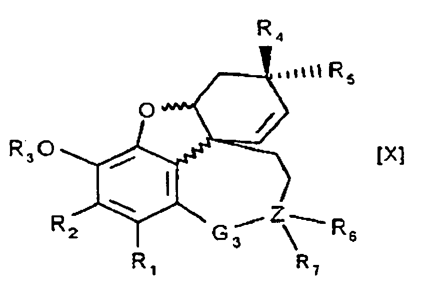

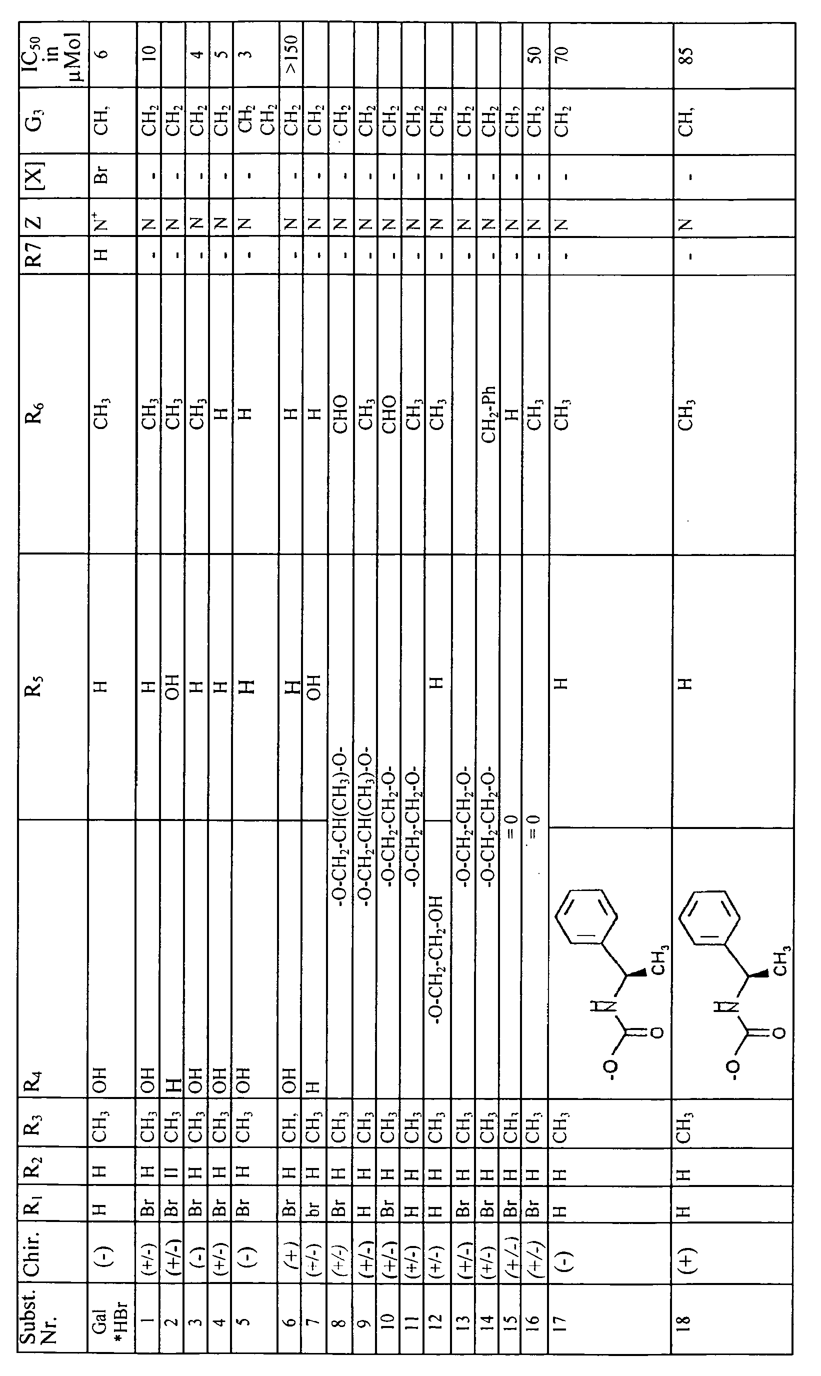

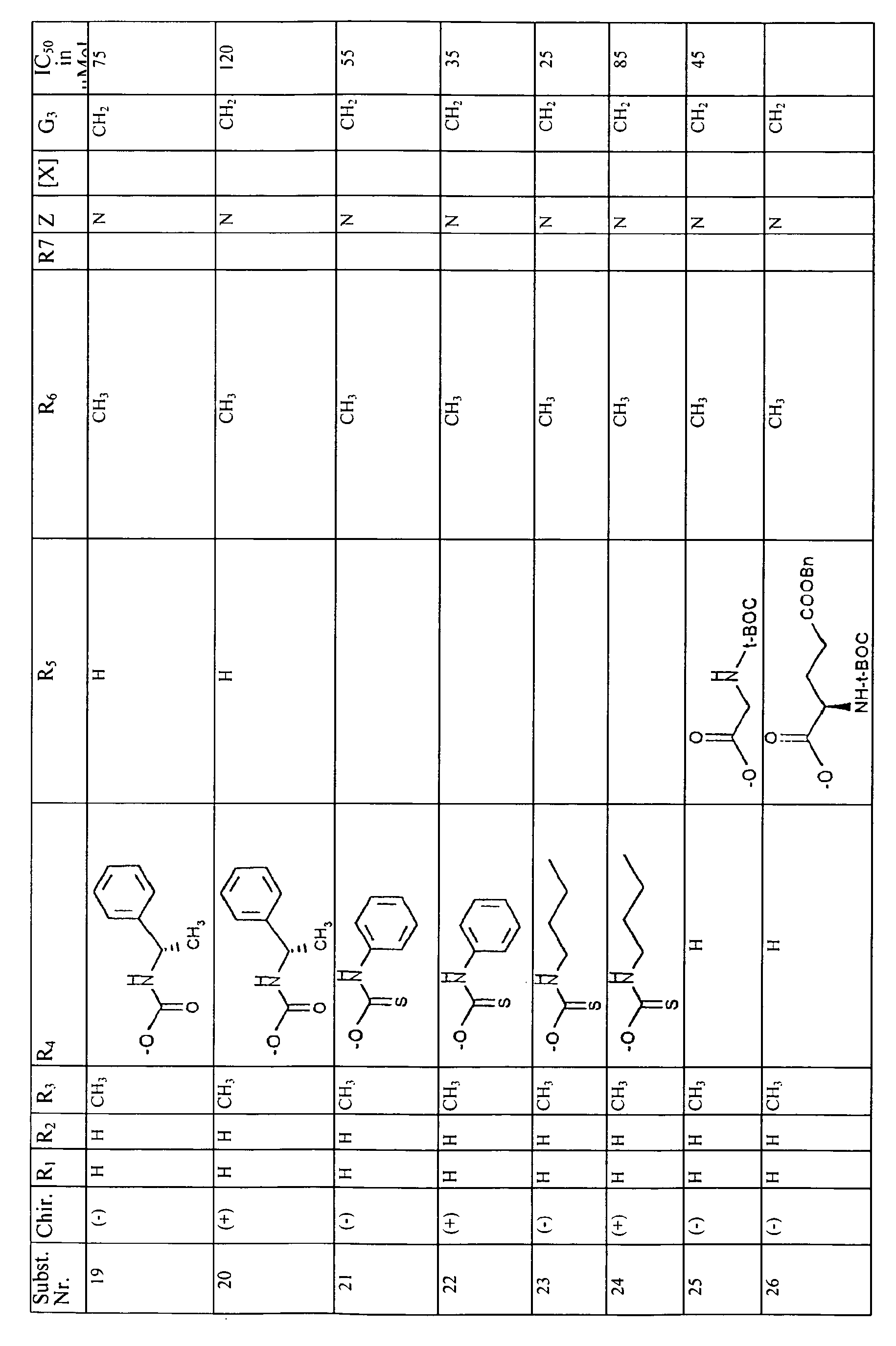

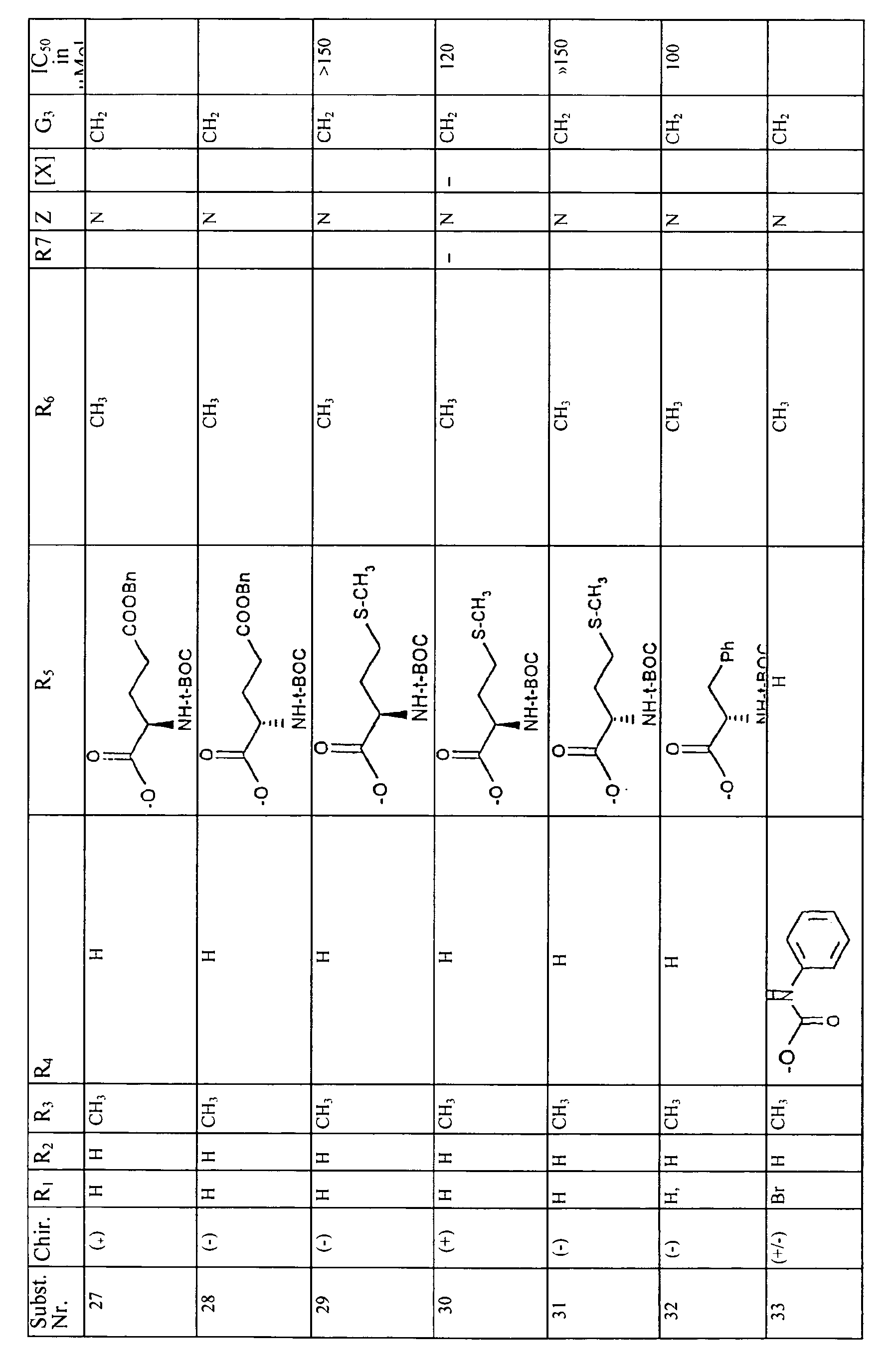

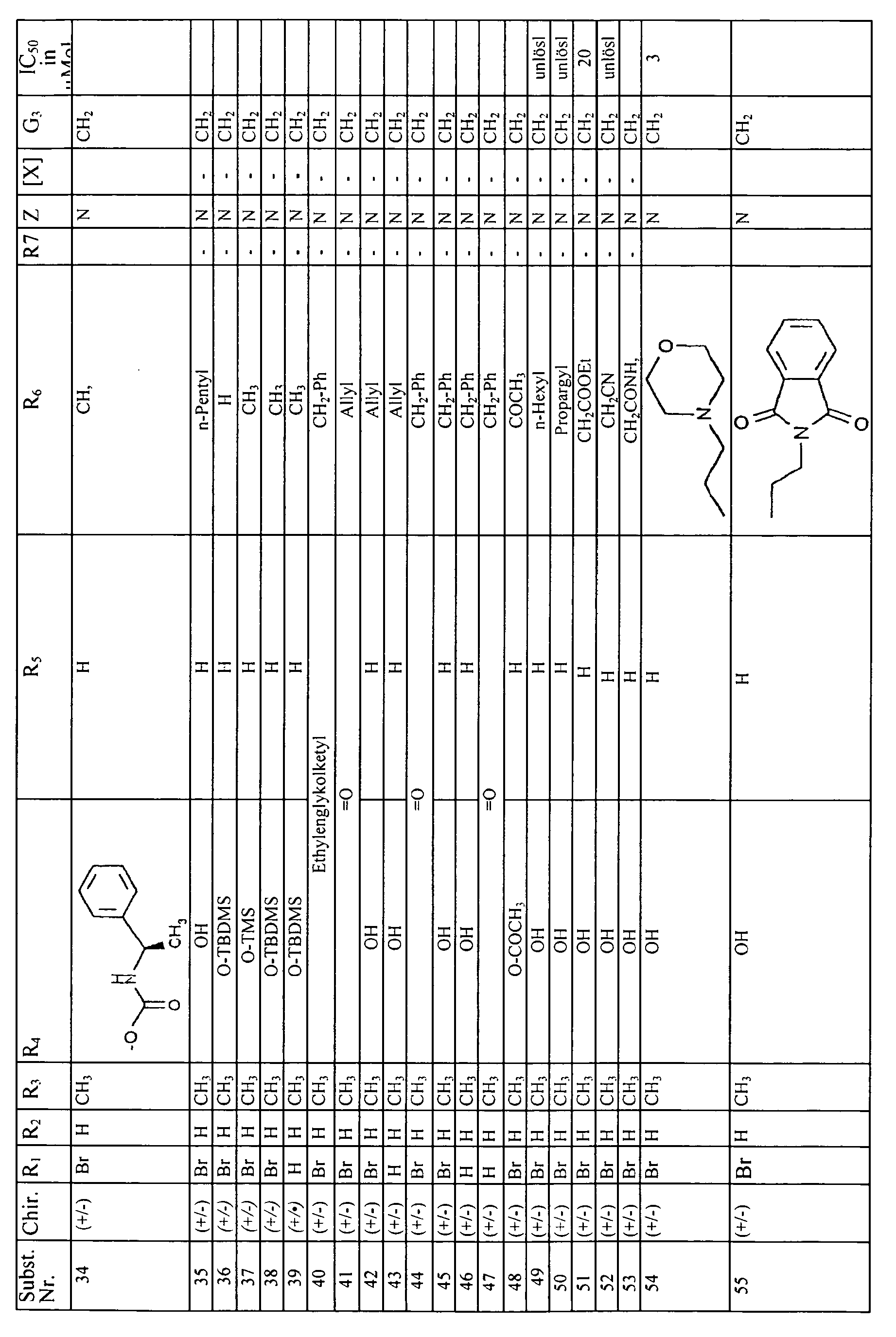

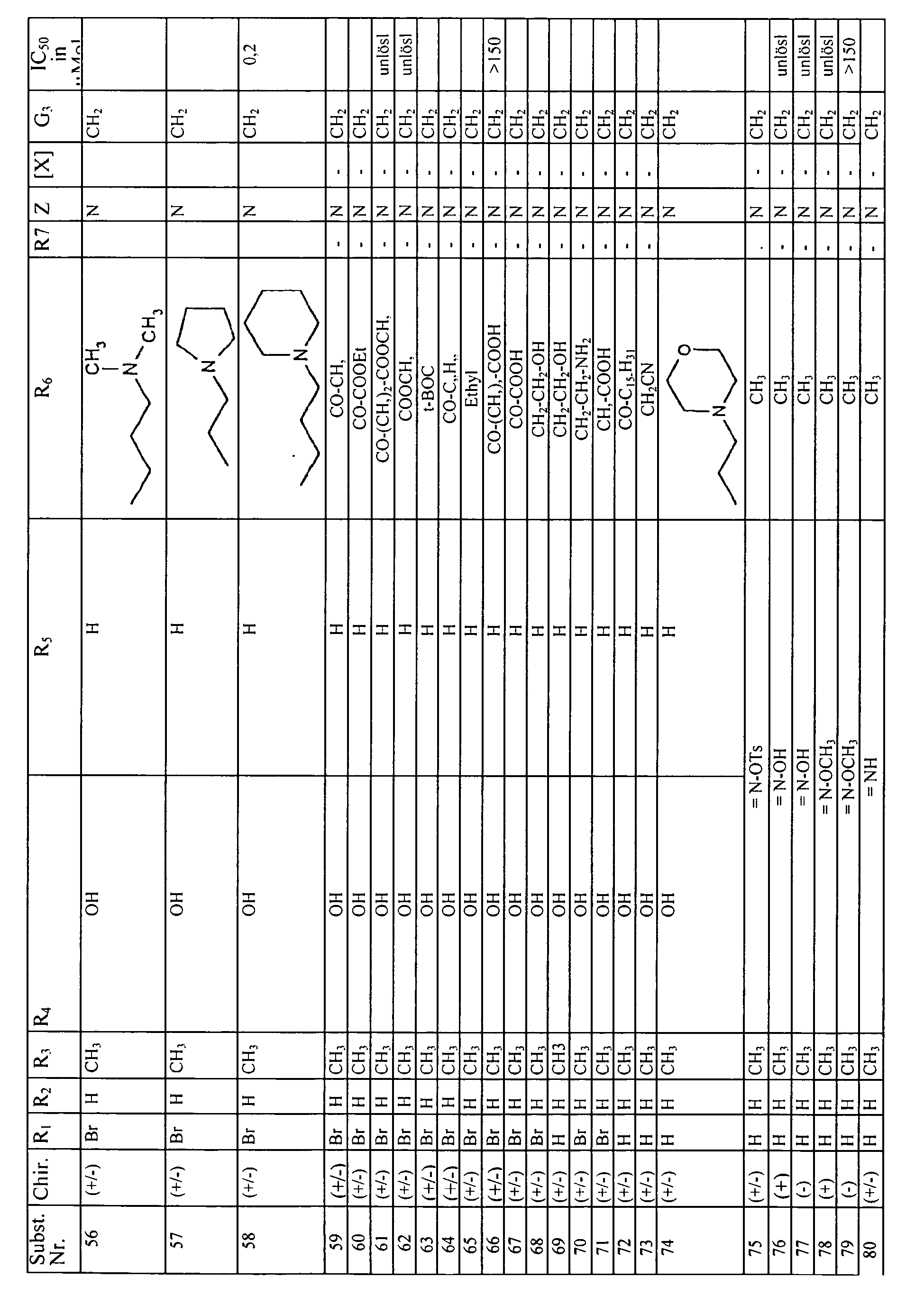

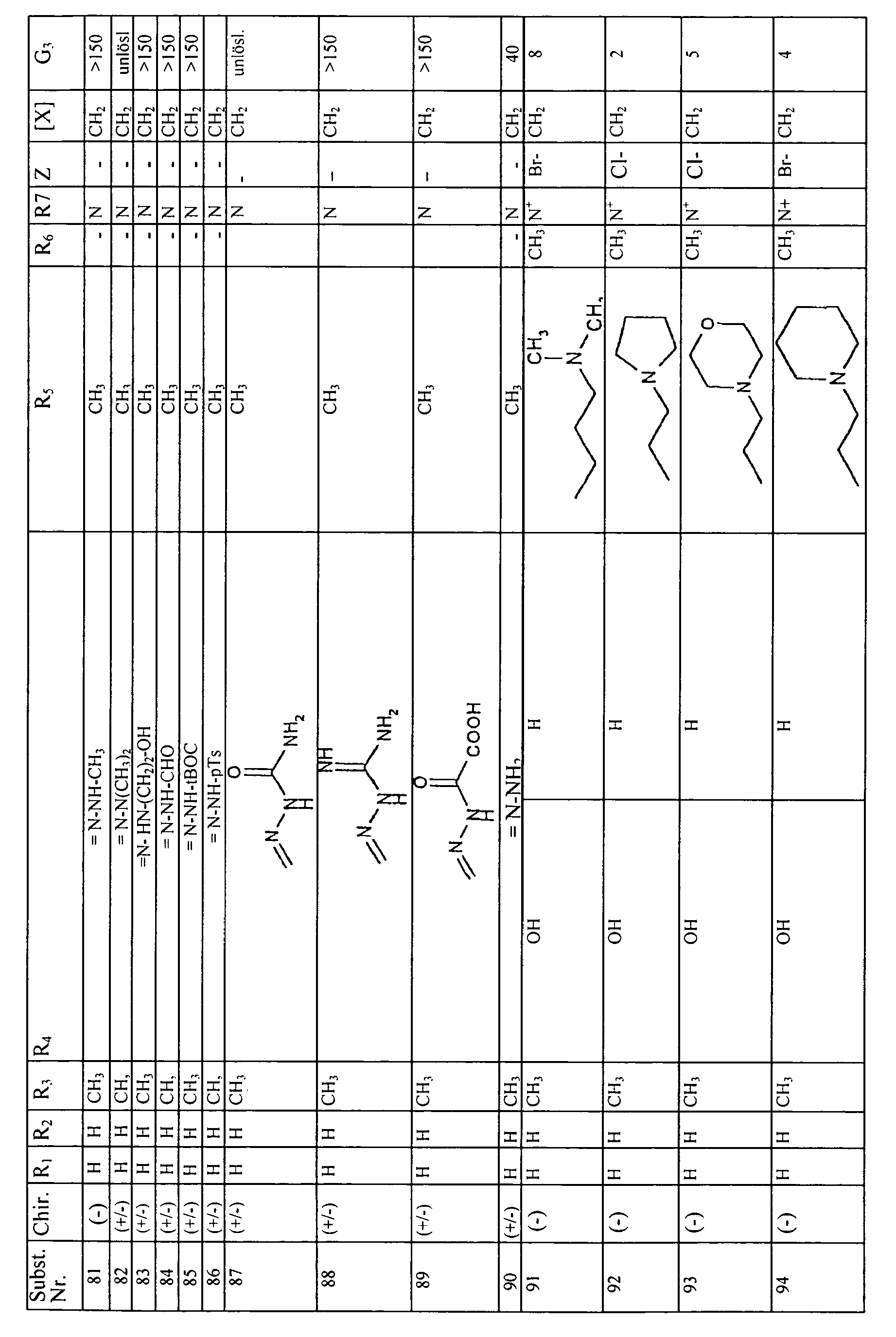

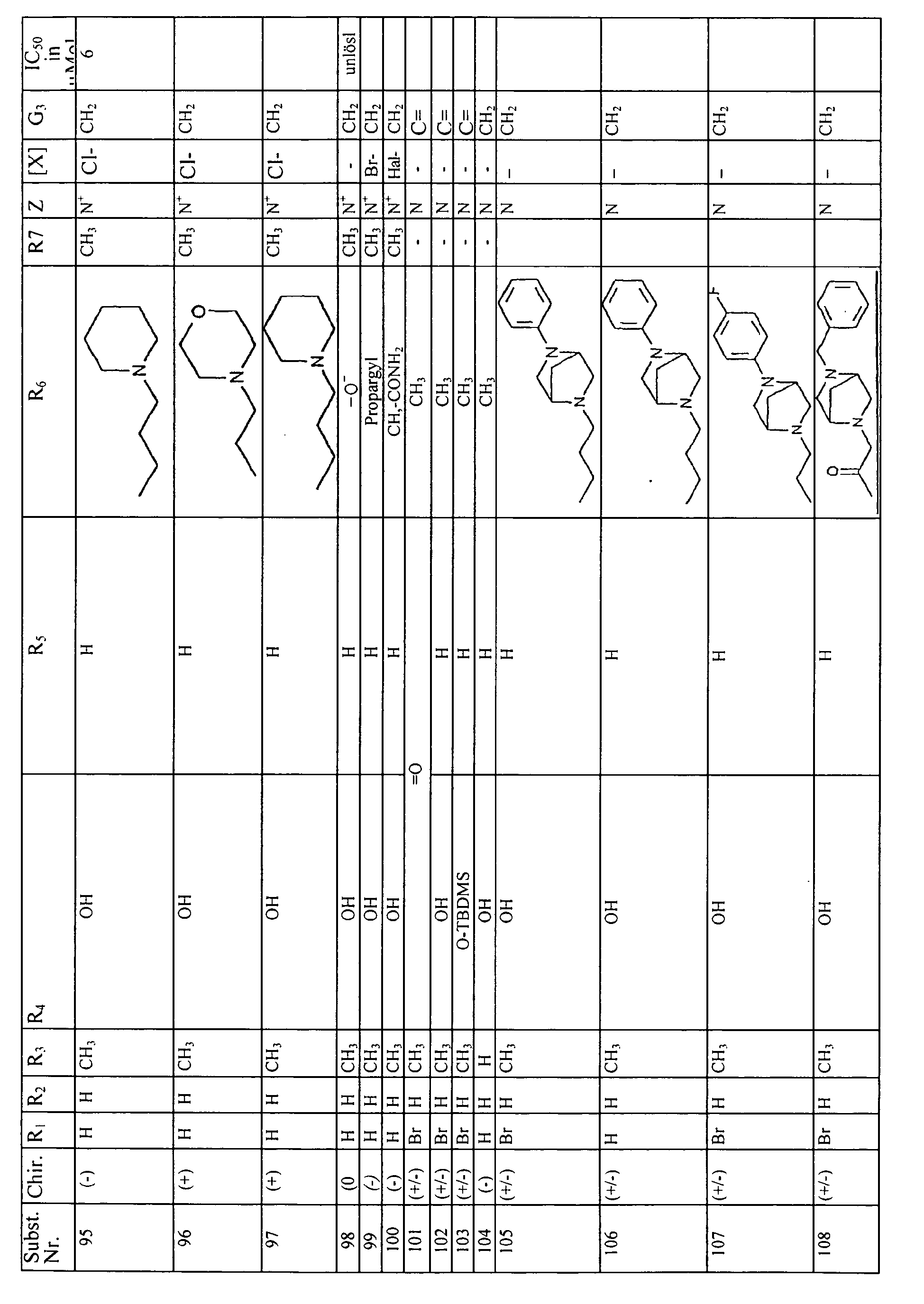

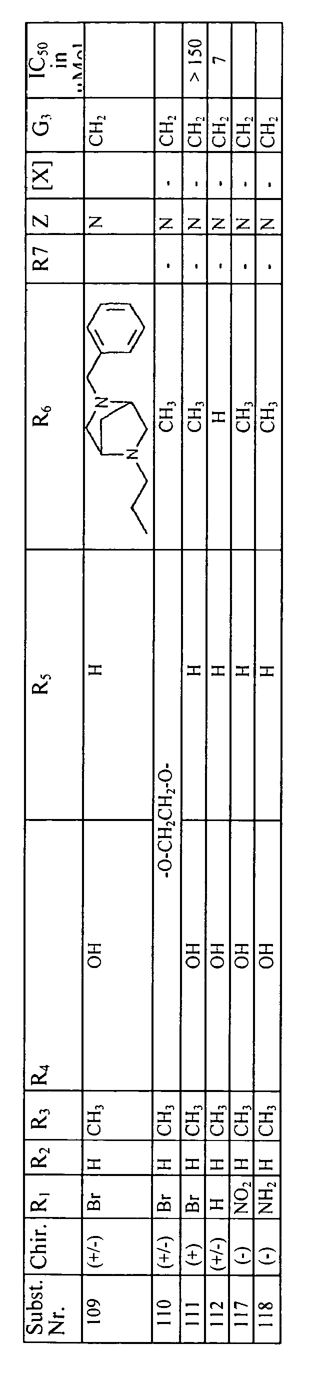

Sonderfall der allgemeinen Formel (I) ist die allgemeine Formel (II):

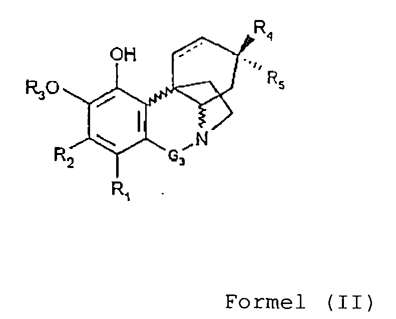

| Pat II Nr. | Chiral | R₁ | R₂ | R₃ | R₄ | R₅ | G₃ | DB* | IC₅₀ |
|---|---|---|---|---|---|---|---|---|---|
| 113 | (+/-) | Br | H | CH₃ | = O | | CH₂ | ja | 5 |
| 114 | (+/-) | Br | H | CH₃ | OH | H | CH₂ | ja | |
| 115 | (+/-) | H | H | CH₃ | OH | H | CH₂ | ja | >150 |
| 116 | (+/-) | Br | H | CH₃ | OH | H | CH₂ | nein | 50 |

| | | | | | | | | | |
|---|---|---|---|---|---|---|---|---|---|
| * DB = Doppelbindung | | | | | | | | | |

Anm.: "Chiral" weist in der gesamten Tabelle auf die Chiralität des jeweiligen Eduktes hin. Drehwerte der Produkte sind im experimentellen Teil erfasst.

Die erfindungsgemäß in Arzneimitteln enthaltenen Verbindungen können in jeder geeigneten chemischen, z.B. als Säureadditionssalz, oder physikalischen Form verabreicht werden. Beispielsweise können sie als das Hydrobromid, Hydrochlorid, Methylsulfat oder Methiodid verabreicht werden.

Die erfindungsgemäßen Arzneimittel können Patienten oral oder durch subkutane oder intravenöse Injektion oder intracerebroventrikulär mittels eines implantierten Behälters verabreicht werden. Es kann notwendig sein, mit niedrigeren Dosen zu beginnen, als sie letztlich wirksam sind.

Typische Dosierungsraten bei Verabreichung von Arzneimitteln enthaltend die erfindungsgemäß vorgeschlagenen Wirkstoffe hängen von der Natur der verwendeten Verbindung und vom Zustand des Patienten ab. So liegen typische Dosierungsraten im Bereich von 0,01 bis 1,0 mg pro Tag und Kilogramm Körpergewicht in Abhängigkeit vom Alter, physischem Zustand und sonstiger Medikation des Patienten.

Die erfindungsgemäßen Arzneimittel können in den folgenden spezifischen Formulierungen vorliegen:
Tabletten oder Kapseln enthaltend 0,5 bis 50 mg
Parenterale Lösung enthaltend 0,1 bis 30 mg/ml
Flüssige Formulierung zur oralen Verabreichung in einer Konzentration von 0,1 bis 15 mg/ml.

Die erfindungsgemäßen Verbindungen können auch ein transdermales System sein, in welchem 0,1 bis 10 mg/Tag freigesetzt werden.

Ein transdermales Dosiersystem besteht aus einer Vorratsschicht, welche 0,1-30 mg der Wirksubstanz als freie Base oder Salz allenfalls zusammen mit einem Penetrationsbeschleuniger, z.B. Dimethlylsulfoxid oder einer Carbonsäure, z.B. Octansäure, und einem hautneutralen Polyacrylat, z.B. Hexylacrylat/Vinylacetat/Acrylsäure Copolymer samt Weichmacher, z.B. Isopropylmyristat enthält. Als Abdeckung dient eine wirkstoffundurchlässige Außenschicht, z.B. ein metallbeschichtetes, siliconisiertes Polyethylenpflaster mit einer Dicke von beispielsweise 0,35 mm. Zur Erzeugung einer klebenden Schicht dient z.B. ein Dimethylamino-methylacrylat/Methylacrylat Copolymer in einem organischen Lösungsmittel.

Nachstehend werden Beispiele für Verfahren, nach welchen erfindungsgemäße Verbindungen hergestellt werden können, angegeben:

### Experimenteller Teil

### Allgemeine Hinweise:

- Dünnschichtchromatographie mit Kieselgel 60 F₂₅₄ (Fa. Merck, Art. Nr. 5554)
- Verwendete Abkürzungen:
   NH₄OH konzentrierter wässriger Ammoniak
   PE Petroläther (40 - 60 ° C)
- p-Ts = p-Tos = p-Toluolsulfonyl-
- CE = Capillarelektrophorese
- Drehwerte sind generell in der Konzentration von c = 0.1 aufgenommen.
- Die Schmelzpunkte wurden mit Hilfe eines Heiztischmikroskopes nach Kofler bestimmt, die Werte sind unkorrigiert.
- Der Glasautoklav stammt von der Fa. Büchi (TinyClave, MiniClave).
- Der Wassergehalt der Lösungsmittel wurde, falls angegeben, nach Karl Fischer bestimmt.
- Die Mikroelementaranalysen wurden im mikroanalytischen Laboratorium des Institutes für Physikalische Chemie der Universität Wien unter der Leitung von Herrn Mag. J. Theiner angefertigt.
- NMR-Spektren wurden auf einem Bruker 200 FS FT-NMR-Spektrometer aufgenommen. Als Lösungsmittel wurden CDCl₃ oder DMSO-d₆ verwendet
¹H-NMR: Messfrequenz 200.13 MHz, interner Standard: CDCl₃ (δ = 7.26 ppm) oder DMSO-d₆ (δ = 2.50 ppm)
¹³C-NMR: Meßfrequenz 50.32 MHz, interner Standard: CDCl₃ (δ = 77.0 ppm) oder DMSO-d₆ (δ = 39.5 ppm)
Die Aufspaltungen in der NMR-Spektroskopie werden wie folgt bezeichnet:
s = Singulett d = Duplett t = Triplett
q = Quartett m = Multiplett

Die Multiplizitäten der ¹³C-Spektren wurden wo notwendig durch DEPT-Experimente ermittelt, die Zuordnungen der ¹H-Spektren gegebenenfalls durch COSY-Experimente.
Mit * sind nicht gesicherte Zuordnungen gekennzeichnet.
• Experimenteller Teil:
   (+/-) 8-Bromgalanthamin (1), (+/-) 8-Brom-epigalanthamin (2):
   Zu einer Suspension von 4,0 g (10,5 mmol) Brom-N-formylnarwedin in 60 ml Toluol wird 24 ml (36 mmol) 1M DIBAL-H-Lösung in Toluol bei 0 °C zugetropft. Die Reaktion wird 1 Std. bei RT gerührt, das restliche Reduktionsmittel mit H₂O zersetzt und anschließend 20 ml cc NH₄OH zugegeben. Nach 20 minütigem Rühren bei Raumtemperatur wird das ausgefallene Material abfiltriert, die org. Phase abgetrennt und die wässrige Phase mit 50 ml Toluol gewaschen. Die vereinigten org. Phasen werden über Na₂SO₄ getrocknet, filtriert und das Lösungsmittel im Vakuum entfernt. Der Rückstand wird mittels Säulenchromatographie getrennt. Ausbeute: 0,9 g (23,3 %) 1 und 0,8 g (20,7 %) 2

Daten von Bromgalanthamin (1):
- Molgewicht: C₁₇H₁₉BrNO₃: 365.23
- IR (KBr): 689,03m; 778,57m; 839,37m; 989,86m; 1050, 66s; 1212,43s; 1279,87s; 1434,08s; 14,72s; 1613.99s; 2667,39m; 3370-3778br.
- ¹H -NMR (CDCl₃) : 6,9 (s, 1 H); 6.06 (m, 2 H); 4,60 (d, 1 H); 4,15, (t, 1 H); 3,92 (d, 1 H); 3,82 (s, 3 H); 3,24 (m, 1 H); 2,98 (dt, 1 H); 2,68 (dd, 1 H); 2,42 (s, 3 H); 2,05 (m, 2 H); 1,60 (dt, 1 H).
- ¹³C-NMR (CDCl₃) : 145,32 s; 144,00 s 133,96 s; 127,95 d; 127,68 s; 126,51 d; 115,61 d; 114,22 s; 88.56 d; 61,58 d; 58,56 t; 55,95 q; 53,26 t; 48,56 s; 42,06 q; 33,47 t; 29, 69 t.

Daten von Epibromgalanthamin (2):
- Molgewicht: . C₁₇H₁₉BrNO₃: 365.23
- IR(KBr): 667,95w; 752m; 836,68m; 1040,31s; 1208,39s; 12,82m; 1435,25m; 1485,72m; 1512,94w; 1558,27w; 1615,19m; 1667,14w; 2943,24w; 3360-3575br.
- ¹H-NMR (CDCl₃): 6,85 (s, 1 H); 5,96 (AB, 22); 4,69 (m, 2 H); 4,28 (d, 1 H); 3,90 (d, 1 H); 3,83 (s, 1H); 3,25 (m, 1 H); 2, 95 (m, 1 H); 2,85 (dt, 1 H); 2,36 (s 3 H); 2,15 (td, 1 H), 1, 69 (m, 2 H).
- ¹³C-NMR (CDCI3 +_{DMSO}-d₆): 145,84 s; 143,49 s; 133,89 s; 133,14 d; 126,12 s; 124,35 d; 115,04 s; 113,01 s; 88,26 d; 61,10 d; 57,441; 55,58 q; 52,84 t; 47,86 s; 41,20 q; 33,35 t; 31,43 t.

### (+/-) Bromgalanthamin (1):

### Methode-1:

Zu einer Lösung von 2,0 g (5,6 mmol) (4) in 20 ml H₂O werden 5 ml 89%iger HCOOH, 5 ml 37%iger CH2O gegeben und unter Rückfluss gekocht. Nach 15-minütigem Kochen wird die Reaktionsmischung mit H₂O verdünnt, der pH-Wert mit 25%iger NH4OH auf 9 gestellt und mit 3x20 ml CH₂Cl₂ extrahiert. Die vereinigten org. Phasen werden mit Na₂SO₄ getrocknet, filtriert und das LM. in Vakuum eingedampft. Chromatographische Reinigung des Rückstandes (150 g SiO₂ Kieselgel CHCl₃:MeOH=97:-95:5) ergibt farblosen Schaum. Ausbeute: 2,0 g (96,4 %)

### Methode-2:

Zu einer Suspension von 10 g (26,4 mmol) Brom-N-formylnarwedin in 200 ml THF wird 100 ml (100 mmol) 1 M Lösung von L-Selectrid bei 0 °C in 30 min zugetropft. Nach 60-minütigem Rühren bei 0 °C wird der Reagenz mit H₂O zersetzt und die Reaktionsmischung mit 100 ml 25 %ier NH₄OH-Lösung versetzt. Nach 30-minütigem Rühren bei RT wird das LM. i.Vak. auf die Hälfte konzentriert, in einen Schütteltrichter überfuhrt, mit 100 ml 25 %iger NH₄OH versetzt und mit 3x200 ml CH₂CI₂ extraliiert. Die vereinigten org. Phasen werden über Na₂SO₄ getrocknet, filtiert und das LM. in Vakuum eingedampft. Zum Rückstand werden 50 ml H₂O, 30 ml 98 %ige HCOOH, 30 ml 37%ige CH₂O-Lösüng gegeben und die Reaktionsmischung unter Rückflussss gekocht. Nach 15-minütigem Kochen wird die Reaktion mit NH₄OH neutralisiert und mit 3x200 ml CH₂Cl₂ extrahiert. Die vereinigten org. Phasen werden über Na₂SO₄ getrocknet, filtriert und das LM. in Vakuum eingedampft. Chromatographische Reinigung des Rückstandes (600 g SiO₂ Kieselgel CHCl₃:MeOH=9:1-8:2) ergibt farblosen Schaum. Ausbeute: 6,4 g (66,2 %)

### Methode zur Herstellung von: rac., (-) oder (+)-Bromgalanthamin (1, 3, 111) :

### Methode A:

Zu einer Lösung von 4.00 g (10.8 mmol) Nivalin in 40 ml 30 %iger Ameisensäure werden 40 ml 30 %ge Wasserstoffperoxidlösung zugegeben und das Reaktionsgemisch rasch auf 100°C erhitzt. Nach 20 min. wird das Reaktionsgemisch rasch auf Raumtemp. abgekühlt, mit konzentriertem wässrigen Ammoniak basisch gemacht und dreimal mit je 50 ml Essigsäureethylester extrahiert*. Die organische Phase wird einmal mit gesättigter wässriger Natriumchloridlösung gewaschen, getrocknet (Na₂SO₄), filtriert und eingedampft, wodurch 2.55 g (64 % d. Th.) farblose Kristalle mit einem Schmp. 76 - 77°C und einem Drehwert von α_{D}²⁰[CHCl₃] = -93° an 3 erhalten werden.

DC: CHCl₃:MeOH = 9:1

### Methode B:

Eine Lösung von 1.0 g (2.84 mmol) rac. N-Demethylbromgalanthamin (4) in 1 ml 37 %igem Formalin, 2 ml Ameisensäure und 5 ml Wasser wird 3 Stdn. bei 70°C gerührt. Nach dem Abkühlen wird mit konzentriertem wässrigem Ammoniak basisch gemacht und 20 Stdn. bei 4°C kristallisieren gelassen. Der Niederschlag wird abgesaugt und bei 50°C/20 mm getrocknet, wodurch 0.85 g (82 % d. Th) farblose Kristalle vom Schmp. 76 -77 °C an 1 erhalten werden.

DC: CHCl₃:MeOH = 9:1

### Methode C:

siehe allgemeine Arbeitsvorschrift zur Reduktion mit L-Selectride NMR-Daten von (1, 3, 111)

¹H-NMR (CDCl₃; δ (ppm)):
1.60 (ddd, 1H, H-9, J_{(9,9')} = 14 .2 Hz); 1.90-2.15 (m, 2H, H-9' /5, J_{(5,5")} = 15.1 Hz); 2.20 (b, 1H tauscht D₂O, OH); 2.45 (s, 3H, NCH₃) ; 2.65 (ddd, 1H, H-5', J_{(5, 5')} , = 15.1 Hz); 2.95 (ddd, 1H, H-10, J_{(10,10')} = 15.6 Hz); 3.25 (ddd, 1H, H-10', J(_{10,10'}) = 15.6 Hz); 3.80 (s, 3H, OCH₃); 3.95 (d, 1H, H-12, J(_{12,12'}), = 16.0 Hz); 4.15 (dd, 1H, H-6); 4.30 (d, 1H, H-12', J(_{12,12'}) = 16.0 Hz); 4.60 (b, 1H, H-4a); 5.95 - 6.10 (m, 2H, H-7/8); 6.90 (s, 1H, H2)

¹³C-NMR (CDCl₃; δ (ppm)): 29.7 (t, C-5); 33.5 (t, C-9); 42.1 (q, NCH₃); 48.6 (s, C-8a); 53.3 (t, C-10); 55.9 (q, OCH₃); 58.7 (t, C-12); 61.6 (d, C-6); 88.6 (d, C-4a); 114.2 (s, C-1); 115.6 (d, C-8); 126.5 (t, C-2); 127.6 (s, C-12a); 127.9 (r, f-7); 134.0 (s, C-12b); 144.0 (s, C-3a); 145.3 (s, C-3)

### N-Demethylbromgalanthamin (4):

### Methode A

50.0 g (132 mmol) N-Formylbromnarwedin werden in 250 ml absolutem Tetrahydrofuran suspendiert und bei -25 bis -20°C mit 430 ml (430 mmol) einer 1 N Lösung von L-Selectrid in Tetrahydrofuran versetzt. Nach 3 Stdn. wird mit Ethanol-Tetrahydrofuran (1:1) hydrolysiert, die Reaktionsmischung auf ca. 200 ml eingeengt, mit 400 ml Ethanol versetzt und abermals auf 200 ml eingeengt, um Borsäureester abzutrennen. Der Rückstand wird in 500 ml Ethanol aufgenommen, mit 62 %iger wässriger Bromwasserstoffsäure versetzt, bis pH 1 erreicht ist und 24 Stdn. bei Raumtemp. gerührt. Der entstandene Niederschlag wird abgesaugt und mit etwas Ethanol nachgewaschen. Nach dem Trocknen des Niederschlages wird er in 500 ml Wasser gelöst. Die wässrige Phase wird langsam unter Kühlung und gutem Rühren mit konzentriertem wässrigem Ammoniak basisch gemacht, sodass das Produkt ausfällt. Der Niederschlag wird im Kühlschrank kristallisieren gelassen und dann abgesaugt. Durch Extraktion des Filtrates mit Essigsäureethylester wird eine zweite Fraktion an Produkt gewonnen, wodurch insgesamt 33.5 g (72 % d. Th.) farblose Kristalle an 4 erhalten werden.

DC: CHCl₃ : MeOH = 95:5

### Methode B:

siehe allgemeine Arbeitsvorschrift zur Reduktion mit L-Selectride

¹H-NMR (CDCl₃; δ (ppm)):
1.65 - 1.85 (m, 2H, H-9/9'), 1.98 (ddd, 1H, H-5); 2.25 (b, 2H tauschen D₂O, NH/OH); 2.62 (ddd, 1H, H-5'); 3.05 - 3.35 (m, 2H, H-10/10') ; 3.80 (s, 3H, OCH₃) ; 3.85 (d, 1H, H-12, J(_{12,12'}) = 14.7 Hz) ; 4.10 (dd, 1H, H-6); 4.48 (d, 1H, H-12', J(_{12,12'}) = 14.7 Hz); 4.56 (b, 1H, H-4a); 5.90 -6.05 (m, 2H, H-7/8); 6.85 (s, 1H, H-2)

¹³C-NMR (CDCl₃; δ (ppm)):
29.7 (t, C-5); 39.8 (t, C-9); 96.6 (t, C-10); 99.3 (s, C-8a); 52.7 (t, C-12); 56.0 (q, OCH₃); 61.7 (d, C-6); 88.4 (d, C-4a); 113.0 (s, C-1); 115.5 (d, C-8); 126.8 (d, C-2); 127.9 (d, C-7); 131.6 (s, C-12a); 134.1 (s, C-12b); 144.0 (s, C-3a); 145.8 (s, C-3)

### (+/-) N-Demethyl-bromgalanthamin (4), (+/-) N-Demethyl-epibromgalanthamin (7):

Zu einer Suspension von 1,0 g (2,6 mmol) Brom-N-formylnarwedin in 5 ml THF wird 3,0 g (11,8 mMol) LiAlH(^{t}BuO)₃ in 15 ml THF 0 °C in 30 min zugetropft. Nach 30-minütigem Rühren bei 0 °C wird die Reaktionsmischung unter Rückfluss gekocht. Nach 22-stündigem Kochen wird der mit dem Reagenz gebildete Komplex mit H₂O zersetzt und die Reaktionsmischung mit 10 ml 25 %iger NH₄OH-Lösung versetzt. Nach 30-minütigem Rühren bei RT wird das LM. i.Vak. auf die Hälfte konzentriert, in einen Schütteltrichter überführt, mit 10 ml 25%iger NH₄OH -Lösung versetzt und mit 3x20 ml CH₂Cl₂ extrahiert. Die vereinigten org. Phasen werden mit Na₂SO₄ getrocknet, filtriert und das LM. in Vakuum eingedampft. Chomatographische Reinigung des Rückstandes (60 g SiO₂ Kieselgel CHCI₃ :MeOH=95:5-9:1 -8:2) ergibt zwei Produkte: 300,0 mg (32,2 %) N-Demethyl-bromgalanthamin (4) als farblosen Schaum und 270 mg (29,0 %) N-Demethyl-epibrom-galanthamin (7) als farblosen Schaum.

Daten von N-Demethyl-epibrom-galanthamin (7):
- Molgewicht: C₁₆H₁₈BrNO₃: 352,21
- IR(KBr): 781,60w; 834,28w; 976,63w; 1050,28m; 1179,73m; 1211,87m; 1280,07m; 1435,24m; 1486,10m; 1616,37m; 2923,54w; 3700-2900mbr.
- ¹H-NMR (CDCl₃) : 6,86 (s, 1H) ; 5,92 (AB, 2H); 4,56 (m, 2H); 4,50 und 3,82 (AB, 2H); 3,80 (s, 3H); 3,28, (m, 2H); 2,52, (m, 1H); 2,20-1,70 (m, 3H).
- ¹³C-NMR (CDCl₃): 146,73s; 143,91s; 134,10s; 132,17s;132,17d; 131,48d; 126,34d; 115,34d; 112,44s; 88,51d; 62,81d; 56,10q; 52,34t; 49,25s; 46,82t; 40,52t; 32,07t.

(-)-N-Demethylbromgalanthamin (5) und (+)-N-DemethyIbromgalanthamin (6):

### (-)-N-Demethylbromgalanthamin (5):

Zu einer Lösung von 10.0 g (28.4 mmol) rac. N-Demethylbromgalantliamin (4) in 30 ml Methanol wird eine Lösung von 4.4 g (11.4 mmol) (-)-0,0-Di-p-Toluoylweinsaure in 5 ml Methanol getropft und anschließend mit 1 ml Methanol nachgewaschen. Die Lösung wird mit einem Impfkristall versetzt (ohne Impfkristall kann die Kristallbildung mehrere Wochen dauern) und zwei Tage bei 4°C stehen gelassen. Dann wird mit einem Glasstab gut durchgerieben und weitere zwei bis fünf Tage bei 4°C stehen gelassen, wobei immer wieder mit einem Glasstab gut durchgerieben wird. Anschließend wird das ausgefallene Salz abgesaugt, dreimal mit eiskaltem Methanol nachgewaschen und in 100 ml Wasser aufgenommen. Die wässrige Phase wird mit konzentriertem wässrigem Ammoniak basisch gemacht und dreimal mit je 60 ml Essigsäureethylester extrahiert. Die vereinigten organischen Phase werden einmal mit gesättigter wässriger Natriumchloridlösung gewaschen, getrocknet (Na₂SO₄, Aktivkohle), filtriert und eingedampft, wodurch 1.90 g (38 % d. Th) farblose Kristalle mit einem Drehwert von α_{D}²⁰ [CHCl₃] = -104° (ee nach CE > 99.9 %) an 5 erhalten werden. Die methanolische Mutterlauge wird eingedampft, der Rückstand in 100 ml Wasser aufgenommen und wie das reine Salz oben behandelt, wodurch 7.13 g (88 % d. Th.) Rohprodukt rückgewonnen werden können, die zur Gewinnung von 6 verwendet werden.

### (+)-N-Demethylbromgalanthamin (6):

Zu einer Lösung von 7.13 g (20.2 mmol) aus der 5-Gewinnung rückgewonnenes N-Demethylbromgalanthamin (dieses leicht angereicherte Produkt bildet schneller Kristalle als rac. (4) in 10 ml Methanol wird eine Lösung von 3.12 g (8.1 mmol) (+)-0,0-Di-p-Toluoylweinsäure in 4 ml Methanol getropft, wobei mit 1 ml Methanol nachgewaschen wird. Die Lösung wird mit einem Impfkristall versetzt (ohne Impfkristall kann die Kristallbildung mehrere Wochen dauern) und wie bei der Gewinnung von 5 behandelt, wodurch 2.02 g (57 % d. Th.) farblose Kristalle mit einem Drehwert von α_{D}²⁰[CHCl₃] =+102°(ee nach CE: > 99.9 %) an 6 erhalten werden.
C₁₆H₁₈BrNO₃ * 1.05 C₂₀H₁₈O₈ * 1.01 H₂O (JOS 1500) 776.11 g/mol
ber.: C 57.26 H 5.05 N1.80
gef: C 57.28 H 5.12 N1.82

### (+/-) Brom-N-formyl-narwedin-propylenglycolketal. (8):

100 g Brom-N-formylnarwedin, 100 g Propylenglycol und 0.5 g H₂S0₄ wurden in 800 ml Toluol (bei RT 2-phasig) unter heftigem mech. Rühren auf Rückflusstemperatur erwärmt (ab ca. 90°C homogen) und 14 Stunden unter Wasserabscheidung gekocht. Nach dem Abkühlen wurden die Phasen getrennt (wobei die Toluolphase die obere Phase ist), die Propylenglycolphase 2 mal mit 100 ml Toluol extrahiert, die vereinten Toluolphasen mit 2 x 200 ml ges. NaHCO₃ Lösung geschüttelt, über Na₂SO₄ getrocknet und eingedampft: Ausbeute: 115.3g gelblicher Schaum (8) (100 % d.Th. roh), der über Nacht kristallisierte. Säulenchromatographie von 1.0 g (60 g Kieselgel 60, CHCl₃/ 1-2% MeOH) ergab: 0.80 g farbloser Schaum, der aus EtOAc kristallisierte.

Schmp.: 170-171 °C

Molgew.: C₂₀H₂₂BrNO₅: 436.28

¹H-NMR (CDCI3):
8.12 (d, H), 6.88 (s, H), 5.96-6.17 (m, H), 5.75 (dd, H), 5.68 (d, H/2), 5.10 (d, H/2), 4.53 (b, H), 4.48 (d, H/2), 4.31 (d, H/2), 3.12-4.38 (m, 5H), 3.82 (s, 3H), 2.56-2.80 (m, H), 2.05-2.35 (dd, H), 1.83 - 2.05 (m, 2H), 1.22-1.47 (m, 3H).

¹³C-NMR (CDCl3) :
162.48, 161.72, 147.17, 144.89, 144.64, 132.16, 129.04, 128.51, 128.57, 127.82, 127.70, 127.61, 115.70, 115.48, 127.09, 126.77, 126.5, 113.20, 111.66,102.38, 102.22,87.25, 87.07, 73.38, 72.46, 71.67, 71.41, 71.23, 70.55, 70.28, 55.92, 51.52,46.18, 48.43, 40.77, 39.29, 36.07, 35.97, 34.58, 33.68, 33.44, 33.13, 18.68, 17.59, 17.45.

Anmerkung NMR, Diastereomere: Aufgrund des zusätzlich eingeführten chiralen Zentrums mittels der (+/-) Propylengruppe kommt es zur Bildung von Diastereomeren, die eine zusätzliche Signalaufspaltung zu jener, von der Formylgruppe verursachten, bewirken.

### (+/-) Narwedin-propylenglycolketal (9):

37.5 g LiAlH₄, wurden unter Argon in einen vorgetrockneten 4-1 Mehrhalskolben gefüllt und 800 ml THF aus einem Tropftrichter zulaufen gelassen, wobei unter heftigem Schäumen die Temp. auf ca. 45 °C anstieg (hängt vom Wassergehalt des THF und des Reaktionskolbens ab). Nun wurde eine Suspension aus 114 g (8) (roh) in 400 ml THF über 15 min. zugetropft, wobei die Temperatur auf Rückflusstemp. (ca. 65-68°C) anstieg. Nun wurde unter mechanischem Rühren 10 Stunden auf Rückflusstemperatur erwärmt, abgekühlt und tropfenweise unter Kühlen 100 ml Wasser in 100 ml THF zugegeben.

Entnahme von 10 ml, Alkalischmachen mit NH₄OH und Extraktion mit EtOAc (3x 20 ml) ergab nach Eindampfen öliges Produkt (9). Säulenchromatographie (5 g Kieselgel 60, CHCl₃/3-5% MeOH) von 0.17 g ergab: 0.1 g farblosen Schaum. Aufarbeitung des Restes: analog, jedoch ohne Säulenchromatographie.

Ausbeute: 87.5 % d.Th.)

Molgew.: (C₂₀H₂₅NO₄) : 343.42

¹H-NMR (CDCI3):
6.60 (dd, 2H), 6.16 (dt, H), 5.68 (dd, H), 4.55 (m, H), 4.38-4.00 (m, 3H), 3.80 (s, 3H), 3.68-2.95 (m, 4H), 2.78-2.60 (m, H), 2.35 (s, 3H), 2.24-2,02 (m, 2H), 1.62 (bd, H) 1.28 (t, 3H).

¹³C-NMR(CDCl3) :
146.59, 143.92, 132.04,131.90, 129.57, 129.16, 128.86, 128.76, 128.39, 127.44, 126.92, 126.12, 126.02, 121.16, 111.05, 110.90, 110.77, 102.87, 102.73, 87.23, 73.15, 72.24, 71.43, 71.12, 70.44, 70.17, 60.28, 55.59, 55.53, 55.45, 53.83,47.87, 47.80,47.75,41.80,41.70, 34.84, 33.95, 33.66, 33.37, 18.66, 17.62, 17.43.

Anmerkung NMR, Diastereomere: Aufgrund des zusätzlich eingeführten chiralen Zentrums mittels der (+-/-) Propylengruppe kommt es zur Bildung von Diastereomeren, die eine zusätzliche Signalaufspaltung zu jener, von der Formylgruppe verursachten, bewirken.

N-Formylbromnarwedinethylenglycolketal (10): 10.0 g (26.5 mmol) N-Formylbromnarwedin werden in 20 g Ethylenglykol und 200 ml Toluol mit 0.1 ml konzentrierter Schwefelsäure am Wasserabscheider auf Rückfluss erhitzt. Nach 24 Stdn. wird die Toluol-Phase abdekandiert und die Ethylenglykol-Phase einmal mit Toluol ausgekocht. Die vereinigten Toluol-Phasen werden zweimal mit gesättigter wässriger Natriumhydrogencarbonatlösung gewaschen und eingedampft, wodurch quantitativ farblose Kristalle vom Schmp 192 - 193 °C an 10 erhalten werden.

DC: EtOAc:MeOH = 99:1

¹H-NMR (CDCl₃; δ (ppm) :
1.75-2.10 (m, 2H, H-9/9'); 2.15 (dd, 1H, H-5, J₍₅,5) =16.5 Hz); 2.65 (dd, 1H, H-5', J_{(5,5)} = 16.5 Hz); 3.60 (ddd, 1H, H-10); 3.80 (s, 3H, OCH₃) ; 3.90 - 4.10 (m, 5H, H-10', 0-CH₂-CH₂-0) ; 4.30 (d, 1H, H-12_{Konformeres A,} J_{(12,12')} = 17.8 HZ) ; 4.50 (d, 1H, H-12_{Konfomeres B}); 4.55 (b, 1H, H-4a) ; 5.10 (d, 1H, H-12' _{Konfomeres A}, J(_{12,12')}= 17.8 Hz); 5.65 (d, 1H. H-12' _{Konformeres B)}; 5.70 (d, 1H, H-8); 6.10 (t, 1H, H-7); 6.85 (s, 1H, H-2); 8.10, 8.15(2*s, 1H, CHO_{KonformeresA/B})

¹³C-NMR (CDCl₃; δ (ppm)):
32.9 (t, C-5) ; 36.0 (t, C-9) ; 39.3, 40.7 (2* t, C-10_{KonfomeresA/B)}; 48.4 (s, C-8a) ; 46.1, 51.4 (2* t, C-12_{Konformeres A/B}); 55. 9 (q, OCH₃) ; 64.2, 65.1 (2* t, 0-CH₂-CH₂-0) ; 86.9, 87.1 (2* s, C-4a_{Konformeres A/B}); 102.0 (s, C-6); (d, C-2) ; 115.4, 115.7 (2* d, C-8_{Konformeres A/B)}; 126.4 (s, C-12a) ; (s, C-1) ; 127.5, 127.7 (2* t, C-7_{KonformeresA/B)}; 132.0, 132.1 (2* S, C-12b_{KonformeresA/B}); 144.6, 144.8 (2* S, C-3a_{Konformeres A/B}); 147.1 (s, C-3); 161.6, 162.4 (2* S,CHO_{Konformeres A/B})

### Narwedinethylenglycolketal (11):

### Methode A:

Zu einer Suspension von 2.0 g (4.74 mmol) 10 in 50 ml absolutem Tetrahydrofuran werden bei 0°C 20 ml einer 0.9 Molaren Lithiumaluminiumhydrid-Lösung in Diethylether zugetropft. Anschließend wird das Reaktionsgemisch auf Raumtemp. aufwärmen gelassen und schließlich auf Rückfluss erhitzt (Siedetemp.52°C). Nach 50 Stdn. wird nach Abkühlen mit 3 ml eines 2:1-Gemisches aus Tetrahydrofuran und Wasser hydrolysiert. Dann werden 50 ml Wasser und 50 ml konzentrierter wässriger Ammoniak zugegeben und die wässrige Phase dreimal mit je 50 ml Essigsäureethylester extrahiert. Die vereinigten organischen Phasen werden einmal mit gesättigter Natriumchloridlösung gewaschen, getrocknet (Na₂SO₄) und eingedampft. Durch Reinigung über MPLC mit EtOAc : MeOH = 8:2 werden 820 mg (52.5 % d. Th.) farblose Kristalle vom Schmp. 109 - 110°C an 11 erhalten.

DC: CHCl₃:MeOH = 9:1

### Methode B:

1.0 g (3.5 mmol) (-)-Narwedin werden in 2.0 g Ethylenglykol und 20 ml Toluol mit 0.05 ml konzentrierter Schwefelsäure am Wasserabscheider auf Rückfluss erhitzt. Nach 24 Stdn. wird die Toluol-Phase abdekandiert und die Ethylenglykol-Phase einmal mit Toluol ausgekocht. Die vereinigten Toluol-Phasen werden zweimal mit gesättigter wässriger Natriumhydrogencarbonatlösung gewaschen und eingedampft, wodurch quantitativ farblose Kristalle an 11 erhalten werden.

DC: CHCl₃:MeOH = 9:1

¹H-NMR (CDCl₃; δ (PPM)):
1.65 (ddd, 1H, H-9, J_{(9,9')}, = 13.4 Hz); 2.10 (ddd, 1H, H-9' J_{(9,9')} = 13.4 Hz); 2.15 (dd, 1H, H-5, J_{(5,5')} = 14.2 Hz); 2.40 (s, 3H, NCH₃) ; 2.65 (dd, 1H, H-5', J_{(5,5')}= 14.2 Hz); 3.05 (ddd, 1H, H-10); 3.20 (ddd, 1H, H-10'); 3.60 (d, 1H, H-12, J_{(12,12')} = 16.0 Hz); 3.80 (s, 3H, OCH₃) ; 3. 90 - 4.05 (m, 4H, O-CH₂-CH₂-O); 4.10 (d, 1H, H-12', J_{(12,12')} = 16.0 Hz); 4.55 (dd, 1H, H-4a); 5.65 (d, 1H. H-8, J_{(7,8)} = 9.8 Hz); 6.15 (d, 1H, H-7, J_{(7,8)} = 9.8 Hz); 6.55, 6.60 (AB, 2H, H-1/2)

¹³C-NMR (CDCl₃; δ (ppm)) :
33.2 (t, C-5); 33.8 (t, C-9); 41.7 (q, N-CH₃); 47.8 (t, C-10); 53.8 (s, C-8a); 55.5 (q, OCH₃); 60.2 (t, C-12); 64.0, 65.0 (2* t, O-CH₂-CH₂-0); 87.1 (d, C-4a); 102.5 (s, C-6); 110.9 (d, C-8); 121.1 (d, C-2); 125.9 (d, C-7); 128.7 (s, C-12a); 128.9 (s, C-12b); 131.8 (d, C-1); 143.8 (s, C-3a); 146.5 (s, C-3)

### (+/-) Galanthamin-2-hydroxyethylether (12):

1.0 g Edukt (10) wurden in 25 ml THF gelöst, auf 0°C abgekühlt, 9 ml LiAlH₄ /THF (IM) über 5 Minuten zugetropft und 30 Minuten bei 0°C gerührt. Nun wurde 48 Stunden auf Rückflusstemperatur erwärmt, abgekühlt, 25 ml NH₄OH (25%) tropfenweise zugegeben und 4-mal mit 20 ml EtOAc extrahiert. Die organischen Phasen wurden über Na₂SO₄ getrocknet und eingedampft: Ausbeute: 0.76 g gelbliches Öl (12) (92.9 % d.Th.). Säulenchromatographie (40 g Kieselgel 60, CHCl₃/ 2-7% MeOH) ergab: 0.62 g farblosen Schaum.

Molgew. (C₁₉H₂₄NO₄) : 330.40

### N-Demethylbromnarwedinethylenglycolketyl (13):

9.0 g (21.3 mmol) N-Formylbromnarwedinethylenglycolketal (10) werden in 100 ml absolutem Tetrahydrofuran suspendiert, bei -15 bis maximal -10°C mit 28.4 ml (25.6 mmol) einer 0.9 N Lithiumalumiumhydridlösung in Diethylether versetzt und bei dieser Temperatur gerührt. Nach 20 min. werden weitere 10 ml einer 0.9 N Lithiumaluminiumhydridlösung in Diethylether zugetropft und weitere 20 min bei -15 bis -10°C gerührt. Anschließend wird mit 15 ml Tetrahydrofuran : Wasser 2:1 hydrolysiert, die Lösung einrotiert und der Rückstand in 200 ml Wasser aufgenommen und dreimal mit je 100 ml Essigsäureethylester extrahiert. Die vereinigten organischen Phasen werden mit gesättigter wässriger Natriumchloridlösung gewaschen, getrocknet (Na₂SO₄) und eingedampft, wodurch 6.53 g (78 % d. Th.) farblose Kristalle an 13 erhalten werden.

DC: CHCl₃:MeOH = 95:5
EtOAc:MeOH = 9:1

¹H-NMR (CDCl₃; δ (ppm) ) :
1.70- 1.85 (b, 1H tauscht D₂0,NH); 1.80 (dd, 1H,H-9); 1.90 (dd, 1H, H-9') ; 2. 15 (dd, 1H, H-5, J_{(5,5')} = 16.0 Hz); 2.65 (dd, 1H, H-5' J_{(5,5')}= 16.0 Hz); 3.20 (ddd, 1H, H-10); 3.80 (s, 3H, OCH₃); 3.85 - 4.10 (m, 6H, H-10712, HO-CH₂-CH₂-0); 4.50 (d, 1H, H-12', J_{12,12')} = 14.2 Hz); 20 4.60 (dd, 1H, H-4a); 5.65 (dd, 1H, H-8, J_{(7,8)} = 9.8 Hz); 6.15 (dd, 1H, H-7, J_{(7,8)} = 9.8 Hz); 6.85 (s, 1H, H-2)

### N-Benzyl-bromnarwedinethylenglycolketal (14):

250 mg (0.63 mmol) N-Demethylbromnarwedinethylenglycolketal (13) und 63 mg (0.63 mmol) Triethylamin werden in 15 ml absolutem Tetrahydrofuran vorgelegt, 108 ml (0.63 mmol) Benzylbromid werden bei Raumtemp. zugegeben und anschießend wird 24 Stdn. gerührt. Das Reaktionsgemisch wird mit 50 ml Wasser versetzt und die wässrige Phase dreimal mit je 20 ml Essigsäureethylester extrahiert. Die vereinigten organischen Phasen werden einmal mit gesättigter wässriger Natriumchloridlösung gewaschen, getrocknet (Na₂SO₄) und eingedampft, wodurch 260 mg (85 % d. Th.) farblose Kristalle vom Schmp. 118 - 119°C an 14 erhalten werden.

DC: EtOAc : MeOH = 9:1

1_{H-NMR} (CDCl₃; δ (ppm)) :
1.65 (ddd, 1H, H-9, J_{(9,9')} = 14.2 Hz); 2.05 - 2.30 (m, 2H, H-5, H-9'); 2.65 (dd, 1H, H-5', J_{(5,5')} = 13.4 Hz); 3.00 - 3.30 (m, 2H, H-10/10'); 3.70 (s, 2H, CH₂-Ph) ; 3.80 (s, 3H, OCH₃) ; 3.90 - 4.20 (m, 5H, H-12, O-CH₂-CH₂-O); 4.35 (dd, 1H, H-12' , J_{(12,12')} = 15.1 Hz); 4.60 (ddd, 1H, H-4a); 5.70 (d, 1H, H-8, J_{(7 8)} = 9.8 Hz); 6.25 (d, 1H, H-7, J_{(7,8)} = 9.8 Hz); 6.85 (s, 1H, H-2); 7.25 - 7.30 (m, 5H, Ph)

¹³_{C-NMR} (CDCl₃; δ (ppm)):
33.1 (t, C-5); 33.4 (t, C-9); 48.5 (s, C-8a); 50.7 (t, C-10); 55.8 (q, OCH₃) ; 56.4 (t, C-12) ; 56.9 (t, CH₂-Ph); 64.2, 65.1 (2* t, O-CH₂-CH₂-O); 87.4 (d, C-4a); 102.3 (s, C-6); 113.6 (s, C-1); 115.6 (d, C-8); 126.6 (s, Ph-1); 127.1 (d, C-7); 128.2,128.9 (6* d, Ph-2 - 6, C-2); 133.1 (s, C-12a); 137.9 (s, C-12b); 144.2 (s, C-3a); 146.3 (s, C-2)

### N-Demethylbromnarwedin (15):

### Methode A:

siehe allgemeine Arbeitsvorschrift zur Abspaltung der Ethylenglykolschutzgruppe

### Methode B:

9.0 g (21.3 mmol) N-Formylbromnarwedinketal (10) werden in 100 ml absolutem Tetrahydrofuran suspendiert, bei -25 bis maximal -20°C mit 28.4 ml (25.6 mmol) einer 0.9 N Lithiumaluminiumhydridlösung in Diethylether versetzt und bei dieser Temperatur gerührt. Nach 20 min. werden weitere 10 ml (9.0 mmol) einer 0.9 N Lithiumaluminiumhydridlösung in Diethylether zugetropft und weitere 20 nun bei -25 bis -20°C gerührt. Anschließend wird mit 15 ml Tetrahydrofuran : Wasser 2:1 hydrolysiert, die Lösung einrotiert und der Rückstand in 200 ml 2 N Salzsäure aufgenommen und 15 Mn. gerührt. Die wässrige Phase wird mit 5.71 g (38.1 mmol) L-(+)-Weinsäure versetzt, mit konzentriertem wässrigem Ammoniak basisch gemacht und dreimal mit je 100 ml Essigsäureethylester extrahiert. Die vereinigten organischen Phasen werden mit gesättigter wässriger Natriumchloridlösung gewaschen, getrocknet (Na₂SO₄) und eingedampft, wodurch 6.53 g (78 % d. Th.) farblose Kristalle an 15 erhalten werden.

DC: CHCl₃:MeOH = 95:5
EtOAc:MeOH = 9:1

¹H-NMR (CDCl₃; δ (ppm)):
1.90 - 2.15 (m, 2H, H-9/9'); 2.75, 2.95 (AB, 2H, H-5/5', J_{(5 5')} = 16:0 Hz); 3.10 - 3.35 (m, 2H, H-10/10'); 3.75 (s, 3H, 0-CH₃); 3. 90 (d, 1H, H-12, J_{(12,12)} = 16.4 Hz) ; 4.40 (d, 1H, H-12', J_{(12,12)} = 16.4 Hz); 4.55 (dd, 1H, H-4a); 5.90 (d, 1H, H-8, J_{(7,8)} = 10.7Hz); 6.90 (s, 1H, H-2); 7.05 (d, 1H, H-7, J_{(7,8)} = 10.7Hz)

¹³C-NMR (CDCl₃; δ (ppm)):
36.3 (t, C-5); 37.0 (t, C-9); 45.6 (s, C-8a); 49.5 (t, C-10); 51.3 (t, C-12); 55.9 (q, OCH₃); 87.9 (d, C-4a); 112.5 (s, C-1); 116.0 (d, C-8); 126.6 (d, C-7); 129.6 (s, C-12a); 132.0 (s, C-12b); 143.7 (s, C-3a); 144.8 (d, C-2); 146.6 (s, C-3)

### Bromnarwedin (16):

### Methode A:

siehe allgemeine Arbeitsvorschrift zur Abspaltung der Ethylenlycolschutzgruppe

### Methode B:

9.0 g (21.3 mmol) N-Formylbromnarwedinketal (10) werden in 100 ml absolutem Tetrahydrofuran suspendiert, bei -5 bis maximal 0°C mit 10.0 ml (26.0 mmol) einer 2.6 N Lithiumaluminiumhydridlösung in Tetrahydrofuran versetzt und bei dieser Temperatur gerührt. Nach 20 min. werden weitere 5 ml (13.0 mmol) einer 2.6 N Lithiumaluminiumhydridlösung in Tetrahydrofuran zugetropft und weitere 20 min bei -5 bis 0°C gerührt. Anschließend wird mit 15 ml Tetrahydrofuran:Wasser 2:1 hydrolysiert, die Lösung einrotiert und der Rückstand in 200 ml 2 N Salzsäure aufgenommen und 15 Min. gerührt. Die wässrige Phase wird mit 6.4 g (42.9 mmol) L-(+)-Weinsäure versetzt, mit konzentriertem wässrigem Ammoniak basisch gemacht und dreimal mit je 100 ml Essigsäureethylester extrahiert. Die vereinigten organischen Phasen werden mit gesättigter wässriger Natriumchlondlösung gewaschen, getrocknet (über Na₂SO₄) und eingedampft, wodurch 6.21 g (80 % d. Th.) farblose Kristalle an 16 erhalten werden.

DC: CHCl₃:MeOH = 95:5
EtOAc:MeOH = 9:1

¹H-NMR (CDCl₃; δ (ppm)) :
1.90 (ddd, 1H, H-9, J_{(9,9')}= 12.5 Hz); 2.25 (ddd, 1H, H-9', J_{(9,9')} = 12.5 Hz); 2.45 (s, 3H, NCH₃) ; 2.75 (dd, 1H, H-5, J_{(5, 5')} = 17.8 Hz); 2.95 -3.25 (m, 3H, H-5' /10/10'); 3.85 (s, 3H, OCH₃); 3.95 (d, 1H, H-12, J_{(12,12')} = 16.9 Hz); 4.25 (d, 1H, H-12', J_{(12,12')} = 16.9 Hz); 4.70 (dd, 1H, H-4a); 6.05 (d, 1H, H-8, J_{(7,8)} = 9.8 Hz); 6.95 (s, 1H, H-2); 7.00 (d, 1H, H-7, J_{(7,8')} = 9.8 Hz)

¹³C-NMR (CDCl₃; δ (ppm)):
33.0 (t, C-5); 36.9 (t, C-9); 42.9 (q, NCH₃); 49.2 (s, C-8a); 53.5 (t, C-10); 56.1 (q, OCH₃); 58.9 (t, C-12); 88.0 (C-4a); 114.0 (s, C-1); 116.3 (d, C-2); 127.2 (d, C-8); 127.9 (s, C-12a); 131.6 (s, C-12b); 143.9 (s, C-3a); 144.4 (d, C-7); 146.5 (s, C-3); 193.9 (s, C-6)

Abspaltung der Ethylenglykolschutzgruppe (15,16,Narwedin):

| Substanznr. | Eduktnr. | R₁ | R₆ | SF, MG |
|---|---|---|---|---|
| 15 | 13 | Br | H | |
| Narwedin | 11 | H | CH₃ | C₁₇H₁₉NO₃ [285.35] |
| 16 | 110 | Br | CH₃ | C₁₇H₁₈BrNO₃ [364.25] |

5,0 g Edukt werden in 100 ml 2 N Salzsäure gelöst und für 30 min auf 100°C erhitzt. Nach dem Abkühlen wird mit konzentriertem wässrigem Ammoniak basisch gemacht und das Produkt abgesaugt und bei 50°C/20 mm getrocknet, oder mit Essigsäureethylester extrahiert, getrocknet (Na₂SO₄) und eingedampft.

DC:CHCl₃:MeOH = 9:1

| Substanznr. | Name | Ausbeute | Smp. |
|---|---|---|---|
| 15 | | 91 % farblose Kristalle | 173 - 174°C |
| Narwedin | Narwedin | quantitativ farblose Kristalle | |
| 16 | Bromnarwedin | quantitativ farblose Kristalle | 75 -77°C |

### Narwedin:

¹H-NMR (CCDCl₃; δ (ppm)):
1,85 (ddd, 1H, H-9, J_{(9,9')} = 14,2 Hz) ; 2,25 (ddd, 1H, H-9', J_{(9,9')} = 14,2 Hz); 2,75 (ddd, 1H, H-5, J_{(5,5')} = 17,8 Hz); 3,05 - 3,30 (m, 3H, H-5' /10/10'); 3,70 (d, 1H, H-12, J_{(12,12')} = 12,5 Hz) ; 3, 80 (s, 3H, OCH₃) ; 4,10 (d, 1H, H-12' , J_{(12,12')} = 12,5 Hz) ; 4,70 (b, 1H, H-4a); 6,00 (d, 1H, H-8, J_{(7,8')} = 9,8 Hz); 6. 60 - 6,70 (m, 2H, H-1/2) ; 6,95 (d, 1H, H-7, J_{(7,8)} = 9,8 Hz)

¹³ C-NMR (CDCl₃; δ (ppmm)):
33,3 (t, C-5); 37,3 (t, C-9); 42,5 (q, NCH₃); 49,0 (s, C-8a); 54,1 (t, C-10); 56,0 (q, OCH₃); 60,7 (t, C-12); 88,0 (d, C-4a); 111,9 (d, C-2); 122,0 (d, C-8); 127,1 (d, C-1); 129,4 (s, C-12a); 130,6 (s, C-12b); 144,0 (d, C-7); 144,4 (s, C-3a); 147,0 (s, C-2); 194,4 (s,C-6)

**Allgemeine Arbeitsvorschrift zur Reduktion mit L-Selectride:**

| Substanznr. | Eduktnr. | R1 | R6 | Summenformel, MG |
|---|---|---|---|---|
| 4 | Bromformyl narwedin | Br | 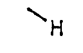 | C₁₆H₁₈BrNO₃ [352.24] |
| 3 | Bromnarwedin | Br | 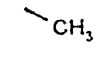 | C₁₇H₂₀BrNO₃ [366.26] |
| 42 | 41 | Br | 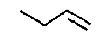 | C₁₉H₂₂BrNO₃ [392.30] |
| 45 | 44 | Br | 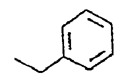 | C₂₃H₂₄BrNO₃ [442.36] |
| 46 | 47 | H | 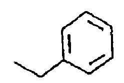 | C₂₃H₂₅NO₃[363.46] |

100 mg Edukt werden in 5 ml absolutem Tetrahydrofuran suspendiert und bei -5 bis 0°C mit 1,2 Äquivalenten einer 1 N Lösung von L-Selectrid in Tetrahydrofuran versetzt. Nach 30 min wird mit Tetrahydrofuran-Wasser 1:1 hydrolysiert, die Reaktionsmischung einrotiert, der Rückstand in 50 ml 2N Salzsäure aufgenommen und über Nacht bei Raumtemperatur gerührt. Die wässrige Phase wird mit 20 ml Diethylether gewaschen und langsam unter Kühlung und gutem Rühren mit konzentriertem wässrigem Ammoniak basisch gemacht, so dass das Produkt ausfällt. Der Niederschlag wird über mehrere Tage im Kühlschrank kristallisieren gelassen und dann abgesaugt. Durch Extraktion des Filtrates mit Essigsäureethylester wird eine zweite Fraktion an Produkt gewonnen. Das Rohprodukt wird durch Säulenchromatographie (15 g Kieselgel, Laufrnittel: CHCl₃: MeOH = 9:1) gereinigt.

DC: CHCl₃ : MeOH = 9:1

| Substanznr. | Name | Ausbeute | Smp. |
|---|---|---|---|
| 4 | (6R)-4a,5,9,10,11,12-Hexahydro-1-brom-3-methoxy-6H-benzofuro[3a,3,2-ef] [2]benzazepin-6-ol | 90% farblose Kristalle | |
| 3 | (6R)-4a,5,9,10,11,12-Hexahydro-1-brom-3-methoxy-11 methyl-6H-benzofuro[3 a,3,2-ef] [2]benzazepin-6-ol | quantitativ farblose Kristalle | 76-77°C |
| 42 | (6R)-4a,5,9,10,11,12-Hexahydro-1-brom-3-methoxy-11-(2-propenyl)-6H-benzofuro[3a,3,2-ef][2]-benzazepin-6-ol | 30% | |
| 45 | (6R)-4a,5,9,10,11,12-Hexahydro-1-brom-3-methoxy-11-(phenylmethyl)-6H-benzofuro[3 a,3,2-ef] [2]-benzazepin-6-ol | 50% | |
| 46 | (6R)-4a,5,9,10,11,12-Hexahydro-3-methoxy- 11 -(phenylmethyl)-6H-benzofuro[3a,3,2-ef] [2]benzazepin-6-ol | 80% | |

**(-)-Galanthamincarbamate und-thiocarbamate**

| Produkt | Summenfonnel | R | Methode | R |
|---|---|---|---|---|
| | C₂₄H₂₆N₂O₄ [406,48] | (-)-Galanthamin-phenylcarbamat | A. | 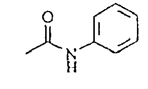 |
| 17 | C₂₆H₂₉N₂O₄ [433,53] | (-)-Galanthamin-R-α-methyl-(-)-Galanthamin-R-α-methyl-benzylcarbamat | A | 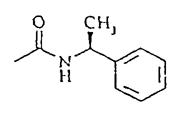 |
| 19 | C₂₆H₂₉N₂O₄ [433,53] | (-)-Galanthamin-S-α-methy]-benzylcarbamat | A | 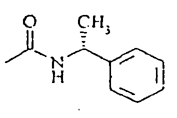 |
| | C₂₈H₂₈N₂O₄ [456,54] | (-)-Galanthamin- α -naphtylcarbamat | B | 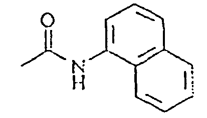 |
| | C₂₂H₃₀N₂O₄ [386,49] | (-)-Galanthamin-n-butylcarbamat | A | 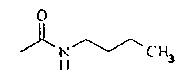 |
| 21 | C₂₄H₂₆N₂O₃S [422,55] | (-)-Galanthamin-phenylthiocarbamat | B | 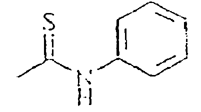 |
| 23 | C₂₂H₃₀N₂O₃S [402,56] | (-)-Galanthamin-n-butylthiocarbamat | B | 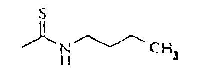 |

### Methode A:

1,2 Äquivalent Isocyanat bzw. Thioisocyanat wurden unter Argon zu einer Lösung von 500 mg (1,74 mmol) (-) Galanthamin in 50 ml absolutem Tetrahydrofuran gegeben und 24 Stunden unter Rückfluss gerührt. Das Reaktionsgemisch wurde eingedampft und der Rückstand durch Säulenchromatographie (Aceton : Methanol = 9 : 1) gereinigt, wodurch farblose Kristalle erhalten wurden.

DC: Toluol:MeOH = 4:1

### Methode B:

68 mg (2,62 mmol) Natriumhydrid (95%ig) wurden unter Argon zu einer Lösung von 500 mg (1,74 mmol) (-) Galanthamin in 15 ml absolutem Dimethylformamid zugegeben und 30 Minuten bei Raumtemperatur gerührt. Dann wurden 1,2 Äquivalent Isocyanat bzw. Thioisocyanat zugetropft und weitere 3 Stunden gerührt. Das Reaktionsgemisch wurde auf 150 ml Wasser gegossen und zweimal mit 150 ml Essigsäureethylester extrahiert. Die organischen Phasen wurden einmal mit 150 ml Wasser gewaschen, über Natriumsulfat getrocknet und eingedampft. Der Rückstand wurde durch Säulenchromatographie (Aceton : Methanol = 9:1) gereinigt, wodurch farblose Kristalle erhalten wurden.

**DC: Toluol:MeOH = 4: 1**

| Produkt | Ausbeute [% d. Th.] | *aₙ(25°C,c= 1) | Schmelzpunkt [°C] |
|---|---|---|---|
| | 94 (Lit.[15]: 80%) | -43.6° | 85-86(Lit. [15]: 85-87) |
| | 58 (Lit. [15]: 60%) | -56,0° | 199-203 (Lit. [15]: 203-204) |
| | 93(Lit. [15]: 100%) | -57,0° | 48-51 (Lit. [15]:47-49) |
| 17 | 96 | -45,5° | 74-77 |
| 19 | 99 | -48,1 | 135-136 |
| 21 | 97 | -22,5° | 175-176 |
| 23 | 71 | -48,5° | 165-167 |

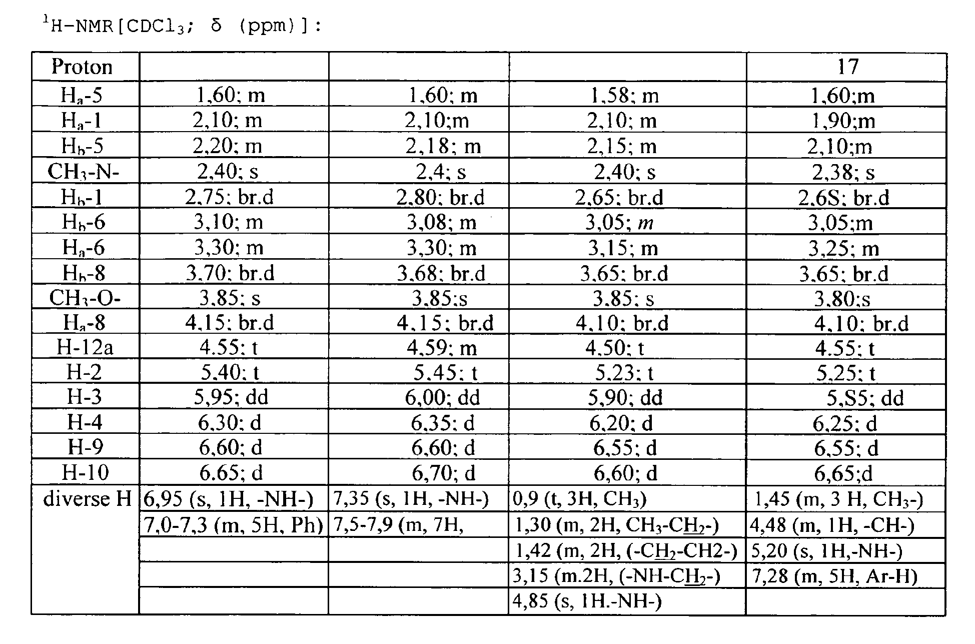

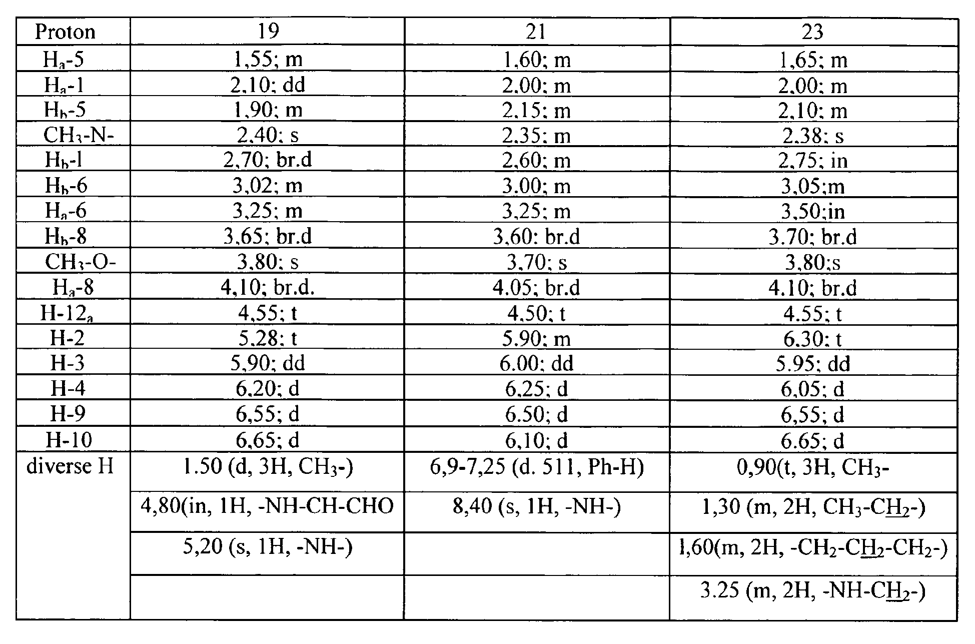

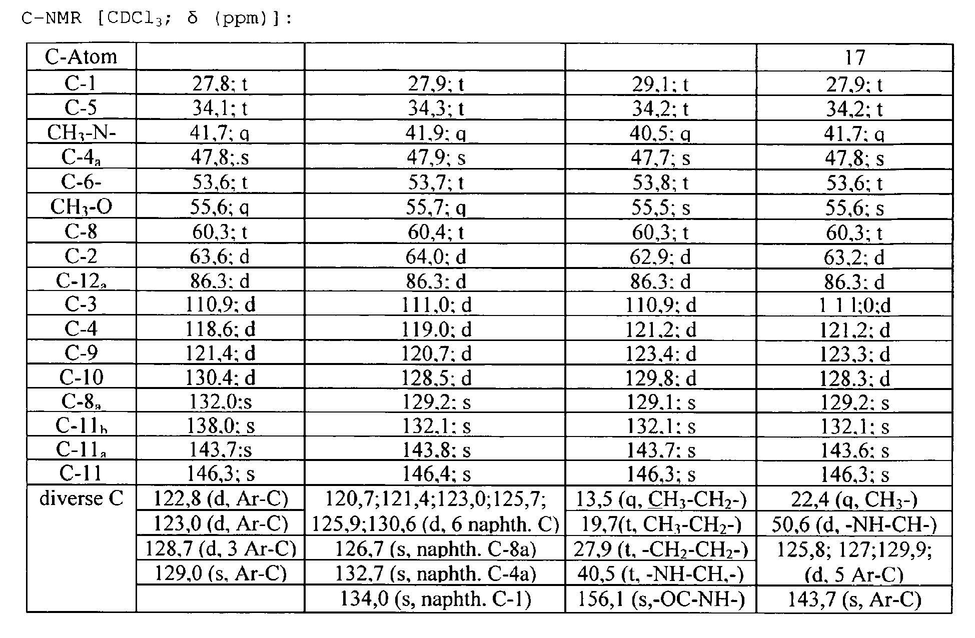

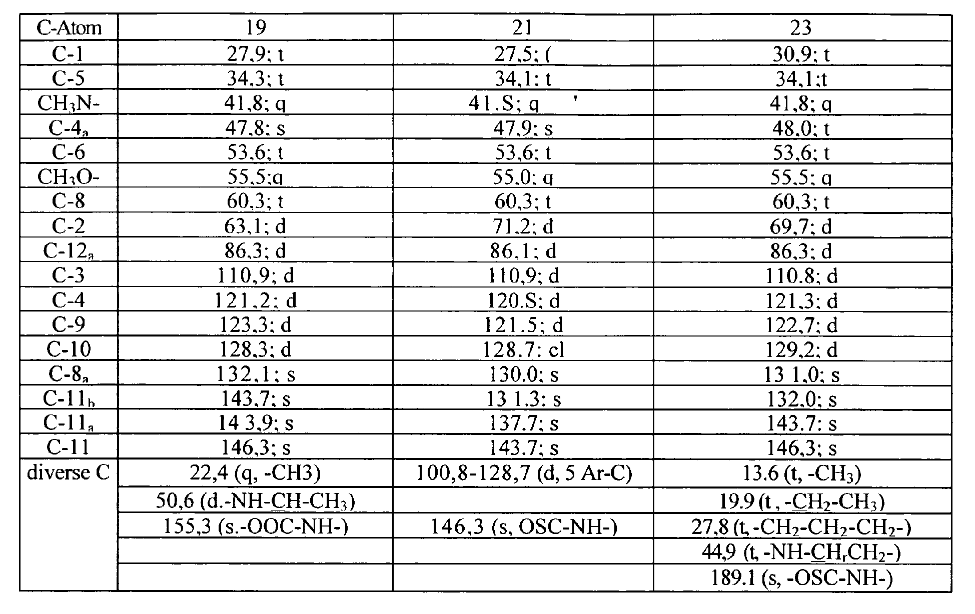

**(+)-GaIanthamincarbamate und -thiocarbamate**

| Produkt | Summenformel | Bezeichnung | R |
|---|---|---|---|
| | C₂₄H₂₆,N₂O₄ [406,48] | (+)-Galanthamin-phenylcarbamat | 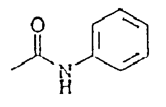 |
| 18 | C₂₆H₂₉N₂O₄ [433,53] | (+)-Galanthamin-R- α -methylbenzylcarbamat | 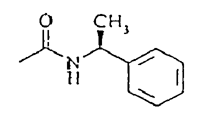 |
| 20 | C₂₆H₂₉N₂O₄ [433,53] | (+)-Galanthamin-S- α -methylbenzylcarbamat | 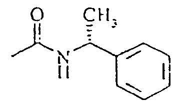 |
| 22 | C₂₄H_{2;6}N₂O₃S [422,55] | (+)-Galanthamin-phenylthiocarbamat | 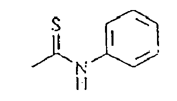 |
| 24 | C₂₂H.₃₀N₂O₃S [402,56] | (+)-Galanthamin-n-butylthiocarbamat | 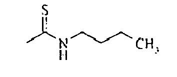 |

### Allgemeine Arbeitsvorschrift:

68 mg (2,62 mmol) Natriumhydrid (95%ig) wurden unter Argon zu einer Lösung von 500 mg (1,74 mmol) (+)-Galanthamin in 15 ml absolutem Dimethylformamid zugegeben und 30 Minuten bei Raumtemperatur gerührt. Dann wurden 1,2 Äquivalent Isocyanat bzw. Thioisocyanat zugetropft und weitere 3 Stunden gerührt. Das Reaktionsgemisch wurde auf 150 ml Wasser gegossen und zweimal mit 150 ml Ethylacetat extrahiert. Die organischen Phasen wurden einmal mit 150 ml Wasser gewaschen, über Natriumsulfat getrocknet und eingedampft. Der Rückstand wurde durch Säulenchromatographie (Aceton : Methanol = 9 : 1) gereinigt, wodurch farblose Kristalle erhalten wurden.

DC: Toluol:MeOH = 4: 1

| Produkt | Ausbeute [% d. Th.] | *α_{D}(25°C, c=1) | Schmelzpunkt [°C] |
|---|---|---|---|
| | 84 | +51,9° | 77-80 |
| 18 | 42 | +55,6° | 58-60 |
| 20 | 47 | +56,5° | 55-57 |
| | 56 | +43,5° | 195-198 |
| | 91 | +42,0° | 52-55 |
| 22 | 61 | + 10,4° | 75-78 |
| 24 | 73 | +31,2° | 122-125 |

**(-)-N-tcrt.-Boc-Aminosaure-epigalanthaminester:**

| Produkt | Summenform | Bezeichnung | R |
|---|---|---|---|
| 25 | C₂₄H₃₂N₂O₆ [444,55] | (-)-N-t-Boc-Glycin-epigalanthaminester | 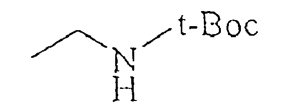 |
| 26 | C₃₃H₄₀N₂O₈ [592,74] | (-)-N-t-Boc-L-Asparaginsäure-β-benzylester-epigalanthaminester | 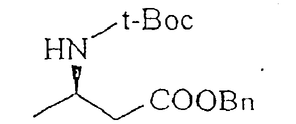 |
| 28 | C₃₃H₄₀N₂O₈ [592,74] | (-)-N-t-Boc-D-Asparaginsäure-β-benzylester-epigalanthaminester | 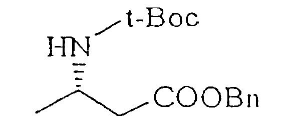 |
| 29 | C₂₇H₃₃N₂O₆S [518,65] | (-)-N-t-Boc-L-Methionin-epigalanthaminester | 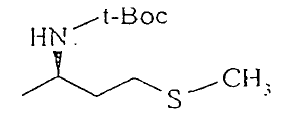 |
| 31 | C₂₇H₃₈N₂O₆S [518,65] | (-)-N-t-Boc-D-Methionin-epigalanthaminester | 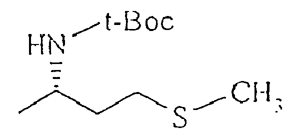 |
| 32 | C₃₁H₃₈N₂O₆ [534,65] | (-)-N-t-Boc-L-Phenylalanin-epigalanthaminester | 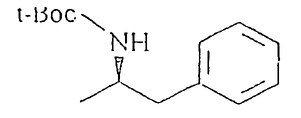 |

### Allgemeine Arbeitsvorschrift:

800 mg (2,78 mmol) (-)Galanthamin, 1,2 Äquivalent t-Boc-Aminosäure und 876,0 mg (3,34 mmol) Triphenylphosphin wurden in 50 ml absolutem Tetrahydrofuran vorgelegt. Nach der Zugabe von 581,7 mg (3,34 mmol) Azodicarbonsäurediethylester (DEAD) wurde das Reaktionsgemisch 3 Stunden bei Raumtemperatur gerührt. Nach der Umsetzung wurde die Lösung eingedampft und der ölige Rückstand durch Säulenchromatographie zuerst in Ethylacetat, um die vielen hochlaufenden Nebenprodukte zu trennen, und dann in Aceton gereinigt. Das ölige Produkt dehnte sich beim Trocknen im Vakuum zum Schaum aus, der sich dann in der Luft aushärtete.

DC: Aceton: MeOH = 9: 1

| Produkt | Ausbeute [% d. Th.] | α_{D}(25°C, c=1) | Schmelzpunkt [°C] |
|---|---|---|---|
| 25 | 93 | -187,3° | 65-66 |
| 26 | 50 | -146,6° | 53-56 |
| 28 | 53 | -140,0° | 63-67 |
| 29 | 78 | -181,7°; | 117-119 |
| 31 | 62 | -140,6° | 126-130 |
| 32 | 44 | -159,1° | 67-69 |

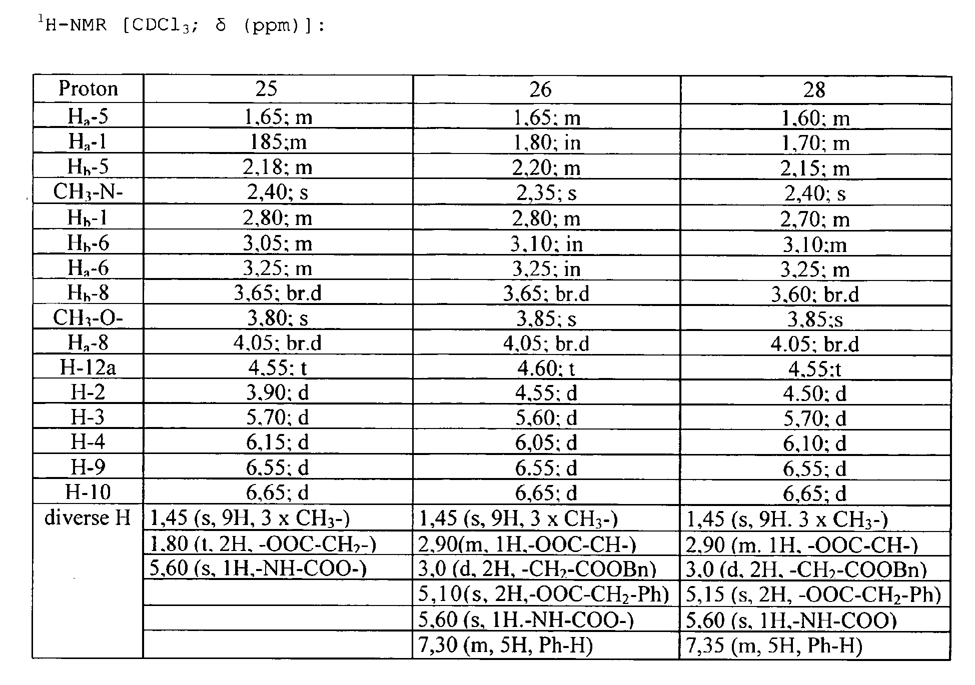

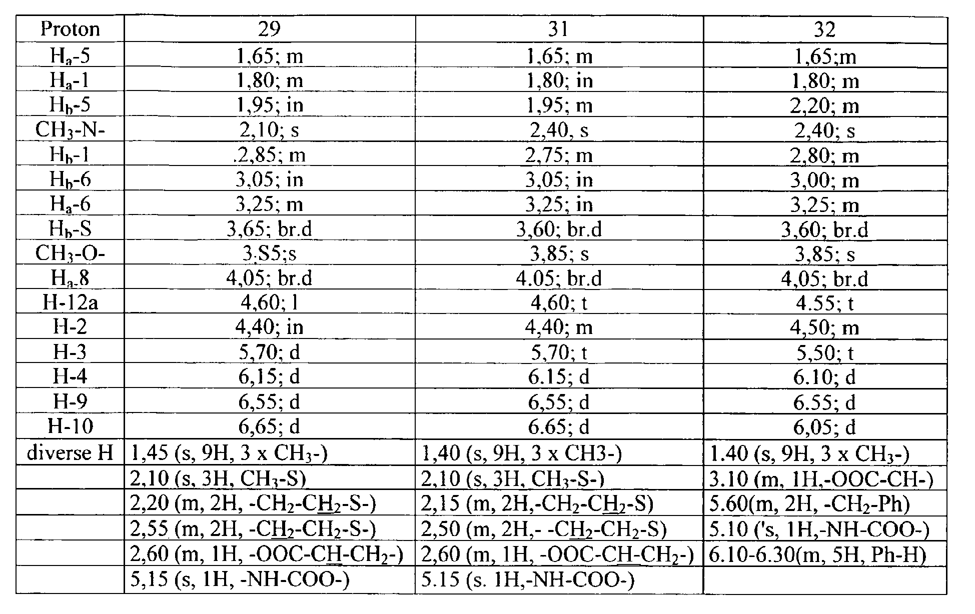

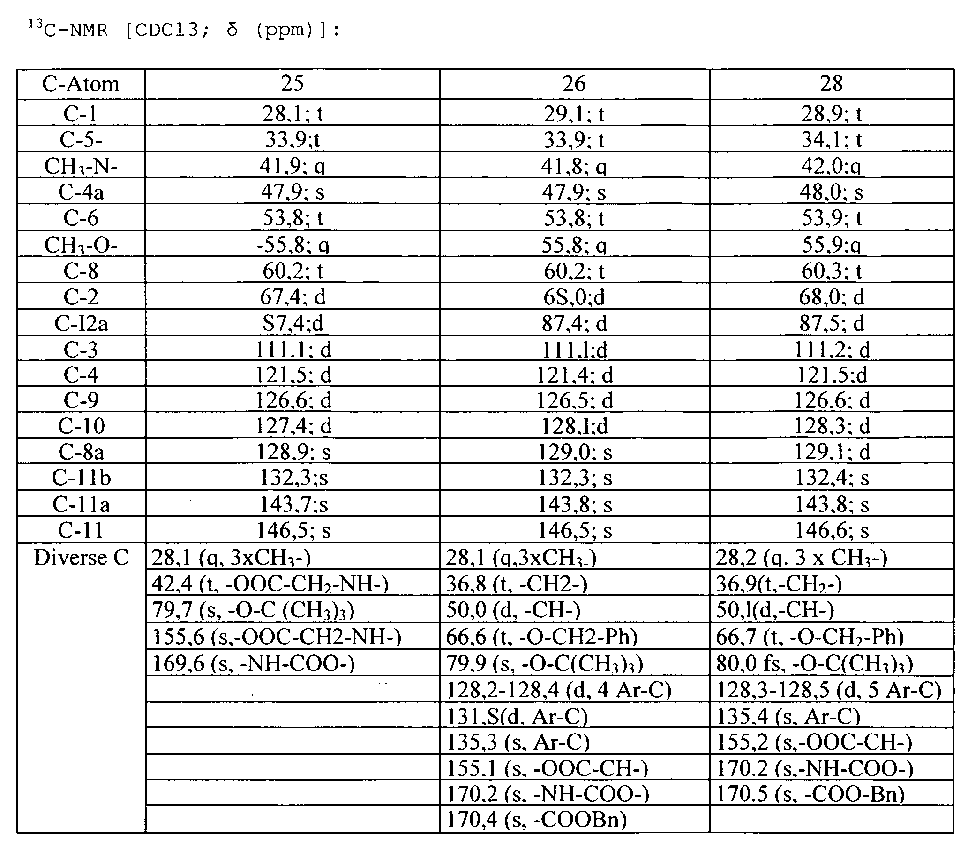

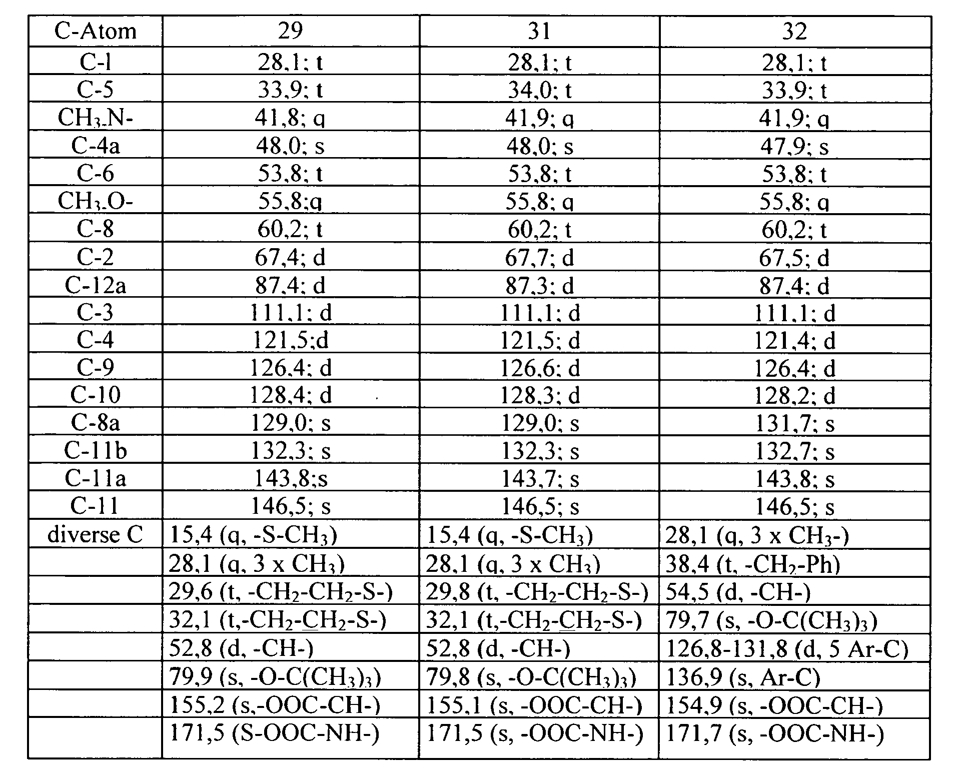

**(+)-N-tert.-Boc-Aminosäurc-epigalanthaminester:**

| Produkt | Summenformel | Bezeichnung | R |
|---|---|---|---|
| 27 | C₃₃H₄₀N₂O₈ [592,74] | (+)-N-t-Boc-L-Asparaginsäure-β-benzylester-epigalanthaminester | 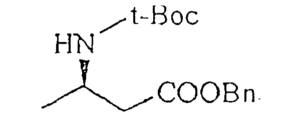 |
| 30 | C₂₇H₃ₛN₂O₆S [518,65] | (+)-N-t-Boc-L-Methionin-epigalanthaminester | 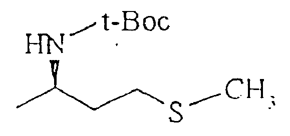 |

### Allgemeine Arbeitsvorschrift:

800 mg (2,78 mmol) (+)-Galanthamin, 1,2 Äquivalent t-Boc-Aminosäure und 876,0 mg (3,34 mmol) Triphenylphosphin wurden in 50 ml absolutem Tetrahydrofuran vorgelegt. Nach der Zugabe von 581,7 mg (3,34 mmol) Azodicarbonsäurediethylester (DEAD) wurde das Reaktionsgemisch 3 Stunden bei Raumtemperatur gerührt. Nach der Umsetzung wurde die Lösung eingedampft und der ölige Rückstand durch Säulenchromatographie zuerst in Essigsäureethylester, um die vielen hochlaufenden Nebenprodukte zu trennen, und dann in Aceton gereinigt. Das ölige Produkt dehnte sich beim Trocknen im Vakuum zum Schaum aus, der sich dann in der Luft aushärtete.

| Produkt | Ausbeute [% d. Th.] | α_{D}(25°C, c=1) | Schmelzpunkt [°C] |
|---|---|---|---|
| 27 | 75 | + 121° | 130-134 |
| 30 | 41 | +117° | 112-115 |

### (±)-Bromgalanthamin-phenylcarbamat (33):

400 mg (1,09 mmol) rohes Bromgalanthamin wurden in 15 ml absolutem TKF gelöst, in Argonatmosphäre mit 390 mg (3,28 mmol) Phenylisocyanat versetzt und 24 Stunden unter Rückfluss gerührt. Das Reaktionsgemisch wurde eingedampft und der Rückstand durch Säulenchromatographie (EE : MeOH = 3:2) gereinigt: 450 mg (85 % d. Th.) farblose Kristalle.

DC: EE: MeOH = 3:2

¹H-NMR[CDCl₃; δ (ppm) ] :
1,60 (m, 1H, Hₐ-5) ; 2, 10 (m, 1H, H_{b}-5); 2, 35 (m, 1H, Hₐ-1); 2,40(s, 3H, N-CH3); 2,70 (br. d, 1H, H_{b}-1) ; 3,0(m, 1R H_{b}-6) ; 3,20(m, 1H, Hₐ-6); 3,80(s, 3H, CH30-); 3,95(dd, 1H, H-3); 4,30(br. d, 1H, Hₐ-8); 4,55(t, 1H, H-12a); 5,35(t, 1H, H-2); 5,95(dd, 1H, H-3); 6,30(d, 1H, H-4); 6,90(s, 1H, H-10); 7,0(s, 1H, -OOC-NH-); 7,0-7,30(m, 5H, Ar-H).

¹³C-NMR [CDCI3; δ (ppm)]:
27,7 (t, C-1); 34,2 (t, C-5); 42,0 (s, N-CH3); 48,5 (s, C-4a); 53,4(t, C-6); 56,0 (q,CH₃O-); 58,6(t, C-8); 63,6(d, C-2): 86,6(d, C-12a); 113,9(s, C-9); 115,7(d, C-3); 118,7(4 C-4); 123,2, 123,5 (d, 2 Ar-C); 127,9 (s, C-8a); 128,9 (d, C-10); 130,3 (s, 3 Ar-C); 133,3 (s, C-11b); 138,0 (s, Ar-C);144,0(s, C-11a); 146,1(s, C-11); 153,3(s, -OOC-NH-).

(±)-Bromgalanthamin-R-a-methylbenzyIcarbamat (34):
510 mg (1,39 mmol) rohes Bromgalanthamin wurden in 20 ml absolutem THF gelöst, in Argonatmosphäre mit 615 mg (4,18 g mmol) R-(+)-a-Methylbenzylisocyanant versetzt und 2 Tage unter Rückfluss gerührt. Das Reaktionsgemisch wurde eingedampft und der Rückstand durch Säulenchromatographie (EE : MeOH =4:1) gereinigt: 600 mg (84 % d. Th.) farblose Kristalle.

DC: EE:MeOH = 4:1

¹H-NMR [(CDCI3); δ (ppm):
1.40 (s, 3H, CH₃-) ; 1,55 (m, 1H, Hₐ-5) ; 2,0(m, 1H, a-1) ; 2,05(m, 1H, H_{b}-5) ; 2,35(s, 3H, N-CH₃) ; 2, 65 (m, 1H, H_{b}-1) ; 2,95(m, 1H, H_{b}-6); 3,25(m, 1H, Hₐ-6); 3, 75 (s, 3H, CH₃O-) ; 3,95(d, 1H, H_{b}-8) ; 4, 25 (d, 1H, Hₐ-8) ; 4.50(t, 1H, H-12a); 4,80(m, -NH-CH-); 5,20(s, 1H, -NH-CH-); 5,22(t, 1H, H-2); 5,88(dd, 1H, H-3); 6,20(d, 1H, H-4); 6,90(s, 1H, H-10); 7,30(m, 5H, Ar-H).

¹³C-NMR [(CDCl₃); δ (ppm)]:
22,1(q, -CH-CH₃); 22,1(s, -CH-CH₃); 27,5(t, C-1); 33,7(t, C-5); 41,4(q, N-CH₃); 48,1(s, C-4a); 52,8(t, C-6); 55,6(q, CH₃O-); 58,0(t, C-8); 62,7(d, C-2); 86,2(d, C-12a); 113,4(s, C-9); 115,3(d, C-4); 123,6; 125,6; 126,8(d, 3 Ar-C); 127,3(s, Ar-C); 128,1; 129,3(d, 2 Ar-C); 132,9(s, C-8a); 143,0(s, C-11b); 143,7(s, C-11a); 145,7(s, C-11); 155,0(s, -OOC-NH-).

### (±)-N-Pentyl-demethyIbromgalanthamin (35) :

In einer Argonatmosphäre bei Raumtemperatur wurden 430 mg (2,84 mmol) n-Pentylbromid zu einer Lösung von 100 mg (2,84 mmol) rohem Demetylbromgalanthamin in 30 ml absolutem THF zugetropft. Anschließend wurde das Reaktionsgemisch unter Rückfluss 2 Tage gerührt. Das Gemisch wurde eingedampft, der ölige Rückstand in 10 ml Wasser aufgenommen und mit konz. Ammoniumhydroxyd auf pH =10 gestellt, wobei ein gelber Niederschlag entstand. Der Niederschlag wird abgesaugt, mit wenig Wasser gewaschen und nach Trocknung (wurde in der Luft viskos) durch Säulenchromatographie (Chloroform : Aceton = 85 : 15) gereinigt: 510 mg (43 % d. Th.) braunes Öl.

DC: CHCI₃: Aceton = 85: 15

¹H-NMR [CDCl₃; δ (ppm)] :
0, 90 (t, 3H, -CH₃) ; 1, 30 (m, 4H,-CH₂-CH₂-CH₃); 1, 50 (t, 2H, -N-CH₂-); 1,55 (m, 1H, Hₐ-5); 1,98 (m, 1H, Hₐ-1); 2,15(m, H_{b}-5); 2, 30 (s, OH); 2, 50 (sext. , 2H, -CH₂-CH₂-CH₃) ; 2, 65 (dd, 1H, H_{b}-1); 3,05(m, 1H, H_{b}-6); 3, 28 (m. 1H, Hg-6); 3,80(s, 3H, CH3O-); 3, 95 (br. d, 1H, H_{b}-8) ; 4, 10 (t, 1H, H-2); 4, 35 (br. d, 1H, Hₐ-8); 4,55(t, 1H, H-12a); 6,0(dd, 1H, H-3); 6,10(d, 1H, H-4); 6,85(s, 1H, H-10).

¹³C-NMR [CDCl₃; δ (ppm)] :
13,9 (q, -CH₃ ) ; 22 , 4 (t, -CH₂-CH₂-CH₃) ; 27,1 (t, -CH₂-CH₂-CH₃) ; 29,4 (t,N-CH₂-CH₂-) ; 29,7(t, C-1) ; 33, 1 (t, N-CH₂-CH₂-) ; 48,8(s, C-4a); 52,5(t, C-5); 52, 3 (t, C-6); 56,0(q, CH₃O-) ; 56,0(t, C-8); 61,7(d, C-2); 88,7(d, C-12a); 114,3(s, C-9); 115,7(d, C-3); 126,7(d; C-4); 127,8(d, C-10); 128,1(s, C-8a); 134,1(s, C-11b); 144,0(s, C-11a); 145,3(s, C-11).

### O-TBDMS-N-Demethylbromgalanthamin (36):

Eine Lösung von 200 mg (0.57 mmol) 4, 63 mg (0.63 mmol) Triethylamin, 38 mg (0.57 mmol) Imidazol, 157 mg (1.14 mmol) Kaliumcarbonat und 171 mg (1.14 mmol) t-Butyldimethylchlorsilan in 15 ml absolutem Tetrahydrofuran wird für 12 Stdn. auf Rückfluss erhitzt. Anschließend wird das Tetrahydrofuran abrotiert und der Rückstand durch Säulenchromatographie (15 g Kieselgel, Laufmittel: CHCl₃ : MeOH = 95:5) gereinigt, wodurch 30 mg (12 % d. Th.) ölige Substanz an 36 erhalten werden.

DC: CHCl₃:MeOH = 9:1

¹H-NMR (CDCl₃; δ (ppm)):
0.09 (s, 9H, C(CH₃)₃); 0.85 (s, 6H, Si(CH₃)₂); 1.82 (dd, 1H, H-9); 1.96 -2.14 (m, 2H, H-9'/5); 2.34 (ddd, 1H, H-5'); 3.31 (ddd, 1H, H-10); 3.51 (ddd, 1H, H-10'); 3.80 (s, 3H, OCH₃); 3.86 (d, 1H, H-12); 4.46 (b, 1H, H-6); 4.60 (b, 1H, H-4a); 4.22 (d, 1H, H-12'); 5.98 (dd, 1H, H-8); 6.01 (d, 1H, H-7); 6.88 (s, 1H, H-2)

### O-TMS-Bromgalanthamin (37):

Eine Lösung von 800 mg (2.19 mmol) rac. Bromgalanthamin (1), 260 mg (2.40 mmol) Trimethylsilylchlorid und 243 mg (2.40 mmol) Triethylamin in 30 ml absolutem Tetrahydrofuran wird auf Rückfluss erhitzt. Nach 2 Stdn. werden weitere 130 mg (1.2 mmol) Trimethylsilylchlorid zugetropft und 1 Std. auf Rückfluss erhitzt. Anschließend wird das Reaktionsgemisch eingedampft, in etwas Dichlormethan aufgenommen und über eine Nutschensäule gereinigt, wodurch quantitativ hellgelbe Kristalle vom Schmp. 228 - 230°C an 37 erhalten werden.

DC: CHCl₃:MeOH = 9:1

¹H-NMR (CDCl₃; δ (ppm)) :
0.10 (s, 9H, Si(CH₃)₃); 1.75 (breites d, 1H, H-9); 2.00 - 2.20 (m, 2H, H-975); 2.35 - 2.50 (breites d, 1H, H-5'); 2.50 (s, 3H, NCH₃) ; 3.0 - 3.15 (m, 1H, H-10); 3.50 (ddd, 1H, H-10'); 3.85 (s, 3H, OCH₃); 4.20 (d, 1H, H-12, J_{(12,12')} = 16.0 Hz) ; 4.25 (b, 1H, H-6); 4.50 (d, 1H, H-12', J_{(12,12')} = 16.0 Hz) ; 4.60 (dd, 1H, H-4a); 5.90 (dd, 1H, H-8, J_{(7,8)} = 9.8 Hz); 6.00 (dd, 1H, H-7, J_{(7,8)} = 9.8 Hz); 6.90 (s, 1H, H-2)

### (-)-O-TBDMS-BromgaIanthamin (38):

Eine Lösung von 2.0 g (5.46 mmol) (-)-Bromgalanthamin (3), 1.23 g (8.20 mmol) t-Butyldimethylchlorsilan und 0.61 g (6.00 mmol) Triethylamin in 50 ml Tetrahydrofuran wird für 4 Stdn. auf 50 °C erhitzt. Anschließend wird das Tetrahydrofuran abrotiert, der Rückstand in etwas Dichlormethan aufgenommen und über eine 1 cm Kieselgelsäule gereinigt, wodurch 1.8 g (69 % d. Th.) amorphe, zähe Substanz mit einem Dreh wert von α_{D}²⁰ [CHCl₃] = -66° an 38 erhalten werden.

DC: CHCl₃:MeOH = 9:1

¹H-NMR (CDCl₃; δ (ppm)) :
0.05 (s, 6H, Si(CH₃)₂) ; 0.90 (s, 9H, SiC(CH₃)₃); 1.75 - 1.90 (m, 1H, H-9); 1.95 - 2.10 (m, 2H, H-5/9' , J_{(5,5},₎ = 16.9 Hz); 2.55 (s, 3H, NCH₃); 2.65 (dd, 1H,H-5', J_{(5,5')}, = 16.9 Hz); 3.00 - 3.15 (m, 1H, H-10, J_{(10,10'}) = 12.5 Hz); 3.45 (ddd, 1H, H-10', J_{(10,10')} = 12.5 Hz); 3.85 (s, 3H, OCH₃) ; 4.15 (dd, 1H, H-6); 4.20 (d, 1H, H-12, J_{(12,12'}) = 16.0 Hz); 4.45 (d, 1H, H-12', J(_{12,12'}) = 16.0 Hz); 4.60 (b, 1H, H-4a); 5.59, 6.05 (AB, 2H, H-7/8, J_{(7,8)} = 10.7 Hz) ; 6.95 (s, 1H, H-2)

### O-TBDMS-Galanthamin (39):

Eine Lösung von 500 mg (1.36 mmol) Galanthamin Hydrobromid, 137 mg (1.36 mmol) Triethylamin, 224 mg (1.36 mmol) Kaliumcarbonat und 244 mg (1.63 mmol) t-Butyldimethylchlorsilan in 20 ml absolutem Tetrahydrofuran und 5 ml absolutem N,N-Dimethylformamid wird 4 Stdn. bei 60°C gerührt. Anschließend wird das Reaktionsgemisch eingedampft und über eine 1 cm Kieselgelsäule gereinigt, wodurch 320 mg (59 % d. Th.) gelbe ölige Substanz an 39 erhalten werden.

DC: CHCl₃: MeOH = 9:1

¹H-NMR (CDCl₃, δ (ppm) ) :
0.05, 0.10 (2* s, 6H, Si(CH₃)2; 0.85,.0.90 (2* s, 9H, SiC(CH₃)₃); 1.55(ddd, 1H, H-9, J(9,9'), = 14.2 Hz); 2.00 - 2.20 (m, 2H, H-5/9', J_{(9,9')} = 14.2 Hz) ; 2.25 - 2.45 (m, 1H, H-5'); 2.35 (s, 3H, NCH₃); 3.00 (ddd, 1H, H-10, J_{(10,10')} = 11.6 Hz) ; 3.30 (ddd, 1H, H-10', J_{(10,10')} = 11.6 Hz); 3.60 (d, 1H, H-12, J_{(12,12')} = 14.2 Hz); 3.85 (s, 3H, OCH₃) ; 4.15 (d, 1H, H-12', J(12,12') = 14.2 Hz); 4.25 (dd, 1H, H-6); 4.55 (dd, 1H, H-4a); 5.85 (dd, 1H, H-8, J_{(7,8)} = 9.8 Hz); 6.10 (d, 1H, H-7, J_{(7,8)}, = 9.8 Hz); 6.50, 6.60 (AB, 2H, H-1/2, J_{(1,2)} = 8.0Hz)

### N-Allyl-N-demethyl-narwedin (41):

Eine Lösung von 100 mg (0.29 mmol) Demethylbromnarwedin (15), 38 mg (0.31 mmol) Allylbromid, 46 mg (0.31 mmol) Natriurmodid und 85 mg (0.62 mmol) Kaliumcarbonat in 10 ml absolutem Aceton wird 12 Stdn. auf Rückfluss erhitzt. Anschließend wird die Lösung eingedampft, in 2 N Salzsäure aufgenommen, mit konz. Ammoniaklösung basisch gemacht und mit Trichlormethan extrahiert. Die vereinigten organischen Phasen werden einmal mit gesättigter wässriger Natriumchloridlösung gewaschen, getrocknet (Na^OJ, filtriert und eingedampft, wodurch 50 mg Rohprodukt erhalten werden, das durch Säulenchromatographie (15g Kieselgel, Laufmittel: CHCl₃ : MeOH = 9:1) gereinigt wird, wodurch 28 mg (25 % d. Th.) farblose Kristalle an 41 erhalten werden.

DC: CHCl₃: MeOH = 9:1

¹H-NMR (CDCl₃; δ (ppm) ) :
1.80 - 2.25 (m, 3H, H-5/9/9'); 2.75 (ddd, 1H, H-5'); 3.05 - 3.25 (m, 2H, H-10/10'); 3.78 (s, 2H, NCH₂) ; 3. 84 (s, 3H, OCH₃) ; 4.00 (d, 1H, H-12); 4 . 55 (d, 1H, H-12') ; 4.73 (b, 1H, H-4a); 5.18 (dd, 2H, =CH₂); 5.90 (dd, 1H, =CH); 6.04 (d, 1H, H-8); 6.90 (s, 1H, H-2); 7.03 (d, 1H, H-7)

### (6R)-4a,5,9,10,11,12-Hexahydro-1-brom-3-methoxy-11-(phenylmethyl)-6H-benzofuro[3a,3,2-ef] [2]-benzazepin-6-on (44):

Eine Lösung von 500 mg (1.43 mmol) Demethylbromnarwedin (15 , 244 mg (1.43 mmol) Benzylbromid, 14 mg (1.43 mmol) Natriumiodid und 400 mg (2.90 mmol) Kaliumcarbonat in 40 ml absolutem Aceton wird 4 Stdn. auf Rückfluss erhitzt. Anschließend wird die Lösung eingedampft, in 2 N Salzsäure aufgenommen, mit konz. Ammoniaklösung basisch gemacht und mit Trichlorrnethan extrahiert. Die vereinigten organischen Phasen werden einmal mit gesättigter wässriger Natriumchloridlösung gewaschen, getrocknet (Na₂SO₄), filtriert und eingedampft, wodurch 350 mg Rohprodukt erhalten werden, das durch Säulenchromatographie (15 g Kieselgel, Laufmittel: EtOAc : PE = 1:1) gereinigt wird, wodurch 280 mg (45° o d. Th.) farblose Kristalle vom Schmp. 135 - 138°C an 44 erhalten werden.

DC: CHCl₃ : MeOH = 9:1

¹H-NMR (CDCl₃; δ(ppm)):
1.88 (dd, 1H, H-9); 2.15 (ddd, 1H, H-9'); 2.55 - 2.80 (m, 2H, H-5/5¹); 2.98 - 3.38 (m, 2H, H-10/10«); 3.77 (s, 2H, NCH₂); 3.86 (s, 3H, OCH₃) ; 4.03 (d, 1H, H-12); 4.31 (d, 1H, H-12'); 4.74 (b. 1H, H-4a); 6.04 (d, 1H, H-8); 6.93 (s, 1H, H-2); 7.08 (d, 1H, H-7); 7.21 - 7.46 (m, 5H, Ph)

13_{C-NMR} (CDCl₃; δ (ppm)) :
31.6 (t, C-5); 37.0 (t, C-9); 49.4 (d, C-8a); 51.1 (t, C-10); 54.8 (t, NCH₂) ; 56.1 (q, OCH₃) ; 56.8 (t, C-12); 88.1 (d, C-4a); 114.1 (d; C-1); 116.4 (d, C-8); 127.1, 127.3 (2 d, C-7, Ph-4); 128.3 (d, Ph-1/2/6); 128.7 (2 d, Ph-3/5); 131.7 (s, C-12a); 138.1 (s, C-12b); 143.9 (s, C-3a); 144.6 (d, C-2); 146.6 (s, C-3); 193.3 (s, C-6)

### (6R)-4a, 5, 9, 10, 11, 12-hexahydro-11-acetyl-1-brom-3-methoxy-6H-benzofuro[3a, 3, 2-ef]-[2] benzazepin-6-ol acetat (48):

Eine Lösung von 300 mg (0,85 mmol) 4, 258 mg (2,55 mmol) Triethylamin in 15 ml absolutem Aceton wird langsam bei 0°C mit 200 mg (2,55 mmol) Acetylchlorid versetzt und anschließend wird 24 Stdn. auf Rückfluss erhitzt. Die Lösung wird zur Trockene eingedampft, in 2 N Salzsäure aufgenommen und dreimal mit 30 ml Essigsäureethylester ausgeschüttelt. Die vereinigten organischen Phasen werden einmal mit gesättigter wässriger Natriumchloridlösung gewaschen, getrocknet (Na₂SO₄), filtriert und eingedampft. Das Rohprodukt, das mit 59 verunreinigt ist, wird durch MPLC (60 g Kieselgel, Laufmittel: CHCl₃: MeOH = 1:1) gereinigt, wodurch 190 mg (51 % d. Th.) ölige Substanz an 48 erhalten werden.

DC: Chloroform : MeOH = 9:1

¹H-NMR (CDCl₃; δ (ppm) ) :
1.70 (ddd, 1H, H-9); 1.80 (dd, 1H, H-9'); 1.95 (ddd, 1H, H-5); 2.03, 2.12 (2s, 6H, 2 COCH₃); 2.02 - 2.18 (m, 1H, H-5'); 2.68 (ddd, 1H, H-10, J_{(10/10')} = 14.3 Hz) ; 3.20 (ddd, 1H, H-10', J_{(10/10')} = 14.3 Hz) ; 3. 85 (s, 3H, OCH₃) ; 4.33 (d, 1H, H-12, J_{(12/12)} = 16.9 Hz) ; 4.55 (b, 1H, H-6, J_{(6,8)} = 4.8 Hz); 5.14 (d, 1H, H-12', J_{(12/12)}= 16.9 Hz) ; 5.32 (dd, 1H, H-4a, J_{(4a,5)} = J_{(4a,5)} = 5.2 Hz) ; 5.93 (dd, 1H, H-8, J_{(7,8)} = 10.3 Hz, J_{(6,8)} = 4.8 Hz) ; 6.15 (d, 1H, H-7, J_{(7,8)} =10.3 Hz); 6.92 (s,1H,H-2)

**Alkylierung von N-Demethylbromgalanthamin (4): (R₇, = /, Z = N)**

| Substanznr. | Rest R6 | Name | Summenformel, MG |
|---|---|---|---|
| 49 | 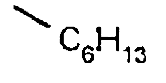 | (6R)-4a,5,9,10,11,12-Hexahydro-1-brom-3-methoxy-11-hexyl-6H-benzofuro[3a,3,2-ef] [2]benzazepin-6- ol | C₂₂H₃₀BrN0₃ [436.40] |
| 52 | 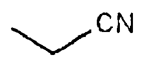 | (6R)-4a,5,9,10,11,12-Hexahydro-1-brom-3-methoxy-1 1-(cyanomethyl)-6H-benzofuro[3a,3,2- ef] [2]benzazepin-6-ol | C₁₈H₁₉BrN₂O₃ [391.27] |
| 51 | 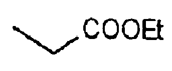 | (6R)-4a,5,9,10,1 1,12-Hexahydro-1-brom-6-hydroxy-3-methoxy-6H-benzofuro[3a,3,2-ef][2]benzazepin-11-essigsäureethylester | C₂₀H₂₄BrNO₅ [438.33] |
| 53 | 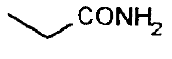 | (6R)-4a,5,9,10,11,12-Hexahydro-1-brom-6-hydroxy-3 -methoxy-6H-benzofuro[3a,3,2-ef] [2]-benzazepin-11-essigsäureamid | C₁₈H₂₁BrN₂O₄ [409.29] |
| 55 | 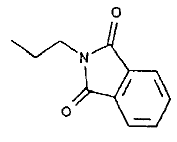 | (6R)-4a,5,9,10,11,12-Hexahydro-1-brom-3-methoxy-11-[2-(1H-isoindol-1,3(2H)-dion-2-yl)-ethyl]-6H-benzofuro[3a,3,2-ef][2]benzazepin-6-ol | C₂₆H₂₅BrN₂O₅ [525.41] |
| 50 | 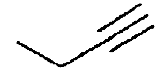 | (6R)-4a,5,9,10,11,12-Hexahydro-1-brom-3-methoxy-11 -(2-propinyl)-6H-benzofuro[3a,3,2-ef] [2]benzazepin-6-ol | C₁₉H₂₀BrNO₃ [390.28] |
| 54 | 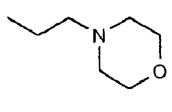 | (6R)-4a,5,9,10,11,12-Hexahydro 1-brom-3-methoxy-11-(2-morpholinoethyl)-6H-benzofuro[3a,3,2-ef] [2]benzazepin-6-ol | C₂₂H₂₉BrNO₃ [465.39] |
| 56 | 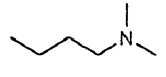 | (6R)-4a,5,9,10,11,12-Hexahydro-1-brom-3-methoxy-i 1-(3-dimethylaminopropyl)-6H-benzofuro[3a,3,2-ef][2]benzazepin-6-ol | C₂₁H₂₉BrN₂O₃ [437.39] |
| 58 | 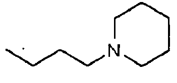 | (6R)-4a,5,9,10,11,12-Hexahydro-1-brom-3-methoxy-11-(3-piperidinopropyl) -6H-benzofuro[3 a,3,2-ef] [2]benzazepin-6-ol | C₂,H₃₃BrN₂0; [477.45] |
| 57 | 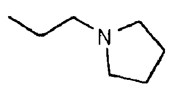 | (6R)-4a,5,9,10,11,12-Hexahydro-1-brom-3-methoxy-11-(2-pyrrolidinoethyl)-6H-benzofuro[3a,3,2-ef][2]benzazepin-6-ol | C₂₂H₂₉BrN₂0₃ [449.40] |
| 42 | 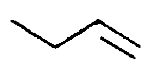 | (6R)-4a,5,9,10,11,12-Hexahydro-1-brom-3-methoxy-11-(2-propenyl)-6H-benzofuro[3a,3,2-ef] [2]-benzazepin-6-ol | C₁₉H₂₂BrNO₃ [392.30] |
| 45 | 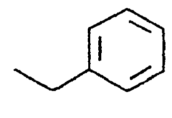 | (6R)-4a,5,9,10,11,12-Hexahydro-1-brom-3-methoxy-11-(phenylmethyl)-6H-benzofuro[3 a,3,2-ef] [2]-benzazepin-6-ol | C₂₃H₂₄BrNO₃ [442.36] |

Methode: Eine Mischung von 500 mg (1.42 mmol) N-Demethylbromgalanthamin (4), 391 mg (2.84 mmol) Kaliumcarbonat und 272 mg (1.70 mmol) Kaliumjodid wird in einer Reibschale gut zermahlen und verrieben. Anschließend wird die Mischung in 20 ml absolutem Aceton mit 1.2 Äquivalenten Halogenidreagens versetzt und auf Rückfluss erhitzt. Nach vollständigem Umsatz (DC) wird das Reaktionsgemisch eingedampft, der Rückstand in 100 ml 2 N Salzsäure aufgenommen, mit Essigsäureethylester gewaschen, mit konzentriertem wässrigen Ammoniak basisch gemacht und entweder der Niederschlag abgesaugt oder dreimal mit je 30 ml Essigsäureethylester extrahiert. Der Niederschlag wird bei 50°C / 50 mbar getrocknet, die vereinigten organischen Phasen werden einmal mit gesättigter wässriger Natriumchloridlösung gewaschen, getrocknet (Na₂SO₄, Aktivkohle), filtriert und eingedampft. Die weitere Reinigung erfolgt über Säulenchromatographie (15 g Kieselgel; Laufmittel: CHCl₃ → CHCl₃ :MeOH = 9:1).

DC: CHCl₃: MeOH = 9:1

| Substanznr. | Reagens | Reaktionszeit | Ausbeute | Smp. |
|---|---|---|---|---|
| 49 | 1-Bromhexan | 24 h | 67 % ölige Substanz | - |
| 52 | Chloressigsäurenitril | 2 h | 89 % farblose Kristalle | 150- 153°C |
| 51 | Chloressigsäure-ethylester | 1h | quant. ölige Substanz | - |
| 53 | Chloressigsäureamid | 1h | 90 % farblose Kristalle | 164- 165°C |
| 55 | N-(2-Bromethyl)-phtalimid | 48 h | quant. gelbe Kristalle | 88 -89°C |
| 50 | Propargylbromid | 4 h | 57 % ölige Substanz | - |
| 54 | N-(2-Chlorethyl)-morpholin * HCl | 24 h | 98 % ölige Substanz | - |
| 56 | (3-Chlorpropyl)-dimethylamin * HCl | 72 h | 46 % ölige Substanz | - |
| 58 | N-(3-Chlorpropyl)-piperidin * HCl | 30 h | 85 % ölige Substanz | - |
| 57 | N-(2-Chlorethyl)-pyrrolidin * HCl | 24 h | 25 % ölige Substanz | |
| 42 | Allylbromid | | 80% | - |
| 45 | Benzylbromid | | 92% | - |

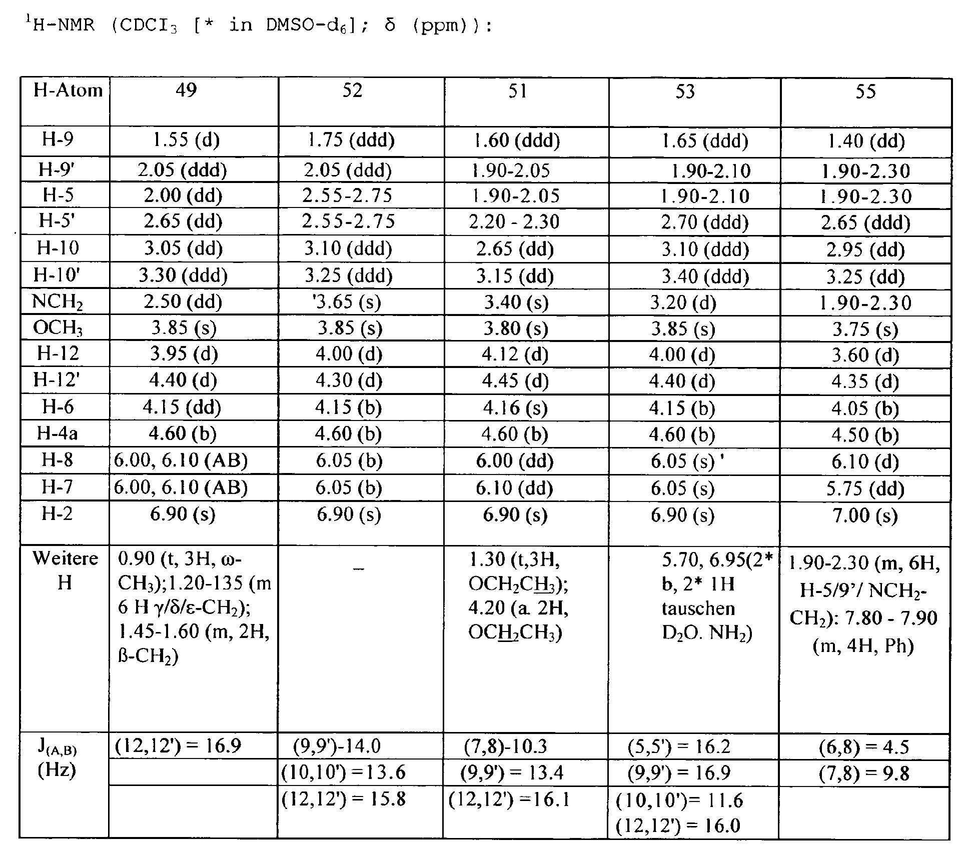

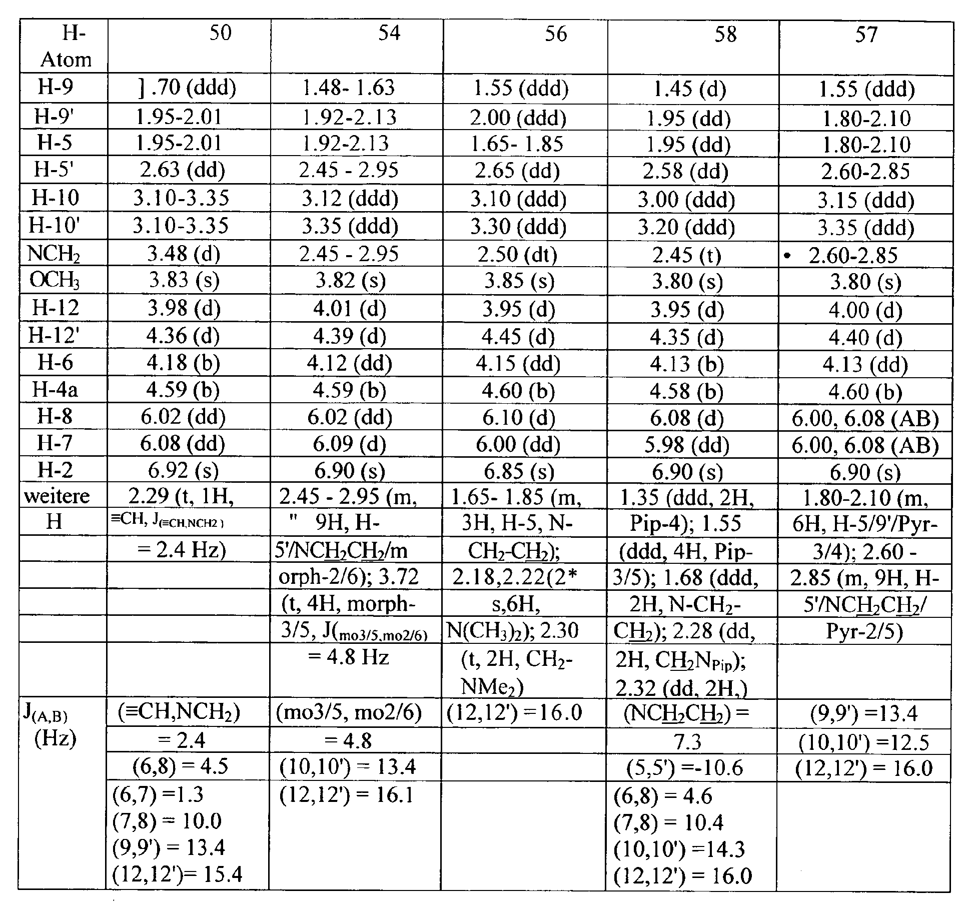

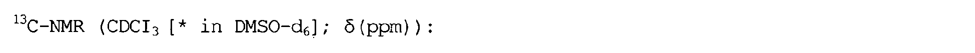

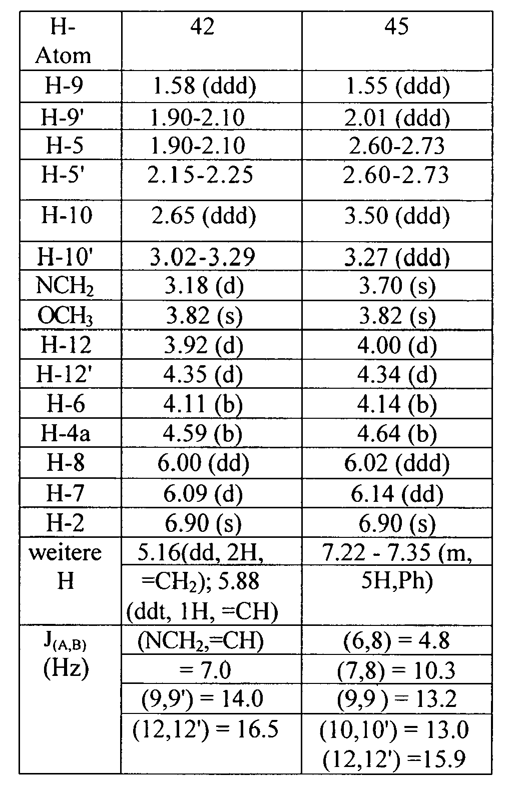

¹³C-NMR (CDCI₃ [* in DMSO-d₆] ; δ (ppm)) :

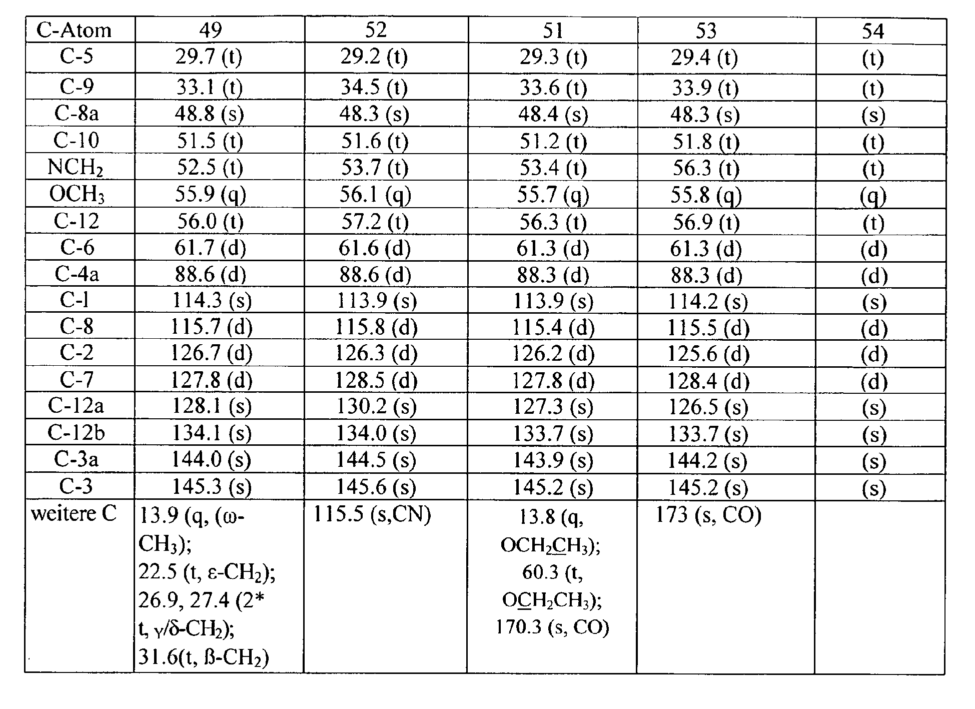

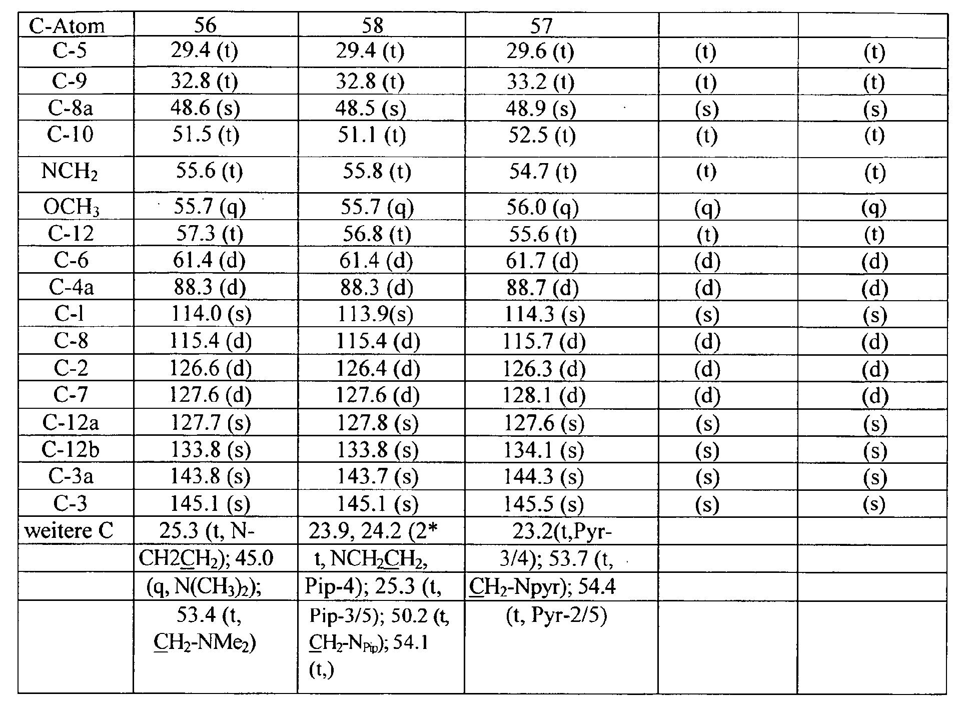

**Acylierung von N-Demethylbromgalanthamin (4): (R₇ = /, Z - N)**

| Substanznr. | Rest R₆ | Name | Summenformel, MG |
|---|---|---|---|
| 59 | 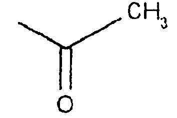 | (6R)-4a,5,9,10,11,12-Hexahydro-1-brom-3-methoxy-1 1-acetyl-6H-benzoforo[3a,3,2- ef] [2]benzazepin-6-ol | C₁₈H₂₀BrNO₄ [394.27] |
| 60 | 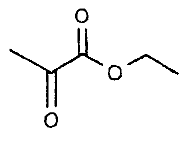 | (6R)-4a,5,9,10,1 1,12-Hexahydro-1-brom- 6-hydroxy-3-methoxy-6H-benzofuro [3a,3,2-ef] [2]benzazepin-11-α-oxo-essigsäureethylester | C₂₀H₂₂BrNO₆ [452.31] |
| 62 | 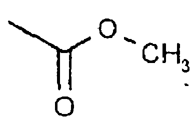 | (6R)-4a,5,9,10,11,12-Hexahydro-1-brom-6-hydroxy-3-methoxy-6H-benzofuro[3a,3,2-ef] [2]benzazepin-11-carbonsäuremethylester | C₁₈H₂₀BrNO₅ [410.27] |
| 61 | 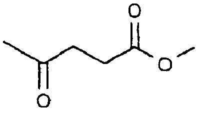 | (6R)-4a,5,9,10,11,12-Hexahydro-1-brom-6-hydroxy-3-methoxy-6H-benzofuro[3 a,3,2-ef][2]benzazepin-11-γ-oxo-buttersäuremethylester | C₂₁H₂₄BrNO₆ [466.34] |
| 64 | 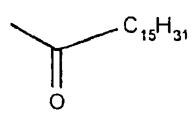 | (6R)-4a,5,9,10,11,12-Hexahydro-1-brom-3-methoxy-11-{1-oxohexadecyl)-6H-benzofuro-[3a,3,2-ef] [2]benzazepin-6-ol | C₃₂H₄₈BrNO₄ [590.65] |

Eine Lösung von 500 mg (1.42 mmol) N-Demethylbromgalanthamin (4) und 156 mg (1.56 mmol) Triethylamin in 20 ml absolutem Aceton wird mit 0.9 Äquivalenten Säurehalogenid versetzt und auf Rückfluss erhitzt. Nach vollständigem Umsatz (DC) wird das Reaktionsgemisch eingedampft, der Rückstand in 100 ml 2 N Salzsäure aufgenommen, mit etwas Essigsäureethylester gewaschen, mit konzentriertem wässrigen Ammoniak basisch gemacht und entweder der Niederschlag abgesaugt oder dreimal mit je 30 ml Essigsäureethylester extrahiert. Der Niederschlag wird bei 50°C / 50 mbar getrocknet, die vereinigten organischen Phasen werden einmal mit gesättigter wässriger Natriumchloridlösung gewaschen, getrocknet (Na₂SO₄, Aktivkohle), filtriert und eingedampft. Die weitere Reinigung erfolgt über Säulenchromatographie (7 g Kieselgel; Laufmittel: CHCl₃: MeOH = 9:1).

DC: CHCl₃ : MeOH = 9:1

| Substanznr. | Reagens | Reaktios-zeit | Ausbeute | Srap. |
|---|---|---|---|---|
| 59 | Essigsäurechlorid | 3h | 84 % gelbe Kristalle | 76-78°C |
| 60 | Oxalsäureethylesterchlorid | 1.5 h | 54 % gelbe Kristalle | 66-69°C |
| 62 | Chlorameisensäuremethylester | 1h | 93 % farblose Kristalle | 158-159°C |
| 61 | Bernsteinsäuremethylesterchlorid | 1.5 h | 35 % farblose Kristalle | 53-57°C |
| 64 | Palmitinsäurechlorid | | 99 % | |

**¹H-NMR(CDCl₃) [* in DMSO-d₆]; δ (ppm)):**

| H-Atom | 59 | 60 | 62* | 61 | 64 |
|---|---|---|---|---|---|
| H-9 | 1.79 (ddd) | 1.92 (ddd) | 1.60-1.90 | 1.75 (ddd) | 1.74 (ddd) |
| H-9' | 1.90 (ddd) | 2.03 (ddd) | 1.60-1.90 | 1.94 (ddd) | 2.24 (ddd) |
| H-5 | 1.97 (dd) | 2.25 (ddd) | 2.05 (dd) | 2.06 (dd) | 1.95 (ddd) |
| H-5' | 2.05 (dd) | 2.68 (dd) | 2.40 (dd) | 2.45 - 2.70 | 2.45 (ddd) |
| H-10 | 2.67 (ddd) | 3.38 (ddd) | 3.40 (dd) | 2.98 (ddd) | 2.68 (ddd) |
| H-10' | 3.20 (ddd) | 3.68 (ddd) | 3.90 (dd) | 3.22 (ddd) | 3.20 (ddd) |
| OCH₃ | 3.83 (s) | 3.85 (s) | 3.75 (s) | 3.80 (s) | 3.84 (s) |
| H-12 | 4.33 (d) | 4.25-4.45 | 4.20 (d) | 4.33 (d) | 4.31 (d) |
| H-12' | 5.13 (d) | 5.20 (d.Konf_{A}), 5,75(d,Konf_{B}) | 5.20 (d) | 5.22 (d) | 5.18 (d) |
| H-6 | 4.13 (b) | 4.10 (b) | 4.10 (b) | 4.12 (dd) | 4.13 (dd) |
| H-4a | 4.60 (b) | 4.45 (b, Konf_{A}), 4.60 (b, Konf_{B}) | 4.50 (b) | 4.60 (dd) | 4.60 (b) |
| H-8 | 6.03 (dd) | 5.90-6.15 | 5.85 (dd) | 6.02 (dd) | 6.05 (dd) |
| H-7 | 5.90 (d) | 5.90-6.15 | 6.00 (dd) | 5.96 (d) | 5.91 (d) |
| H-2 | 6.94 (s) | 6.90 (s) | 6.85 (s) | 6.90 (s) | 6.90 (s) |
| weitere H | 2.11 (s,3 H, OCH₃); 2.30 (b, 1H tauscht D₂0,OH) | 4.25 -4.45 (m, 3H, H-12_{Konf.A/B}^{/} COOCH₂);1.10 (t, 3H, OCH₂CH₃) | 3.55(s, 3H, | 2.45-2.70(m, | 0.89(t, ω- |
| | | | COOCH₃) | 5H, H-5/ | CH₃);1.18- |
| | | | | COCH₂CH₂); 3.65 | 1.40 (m, 22H, |
| | | | | (s,3H,COOCH₃) | CH₂⁽⁴⁻¹⁴⁾);1.45 - 1.67 (m, 4H, |
| | | | | | CH₂⁽²⁻³⁾);2.18 (t, 2H, COCH₂) |

¹³C-NMR (DMSO-d₆; δ (ppm)):
59: 29.6 (q, COCH₃) ; 30.3, 36.1 (t, C-5_{konformeresA/B)} ; 37.9, 43.4 (t, C-9_{KonformeresA/B}) 46.5, 48.8 (t, C-10_{KonformeresA/B})48.4 (s, C-8a); 51.4, 55.8 (t, C-12_{KonformeresA/B}) ; 55. 9 (q, OCH₃) ; 86.3, 86.5 (d, C-4a_{KonformeresA/B}) 115.4 (d, C-8) ; 126.3. 126.4 (d, C-2_{KonformeresA/B}) ; 127.7 (S, C-1) ; 128.5 (s, C-12a) ; 128.7 (d, C-7) ; 133.2, 133.4 (s, C-12b_{KonformeresA/B}) 144.0, 144.3 (s, C-_{KonformeresA/B}) ; 146.6, 147.0 (s, C-3_{KonformeresA/B}) ; 168.9, 169. 2 ( S, CO_{KonformeresA/B})
62: 30.2, 30.5 (t, C-5_{YaonformeresA/B}); 36.5, 37.3 (t, C-9_{KonformeresA/B}) ; 44.7, 45.0 (t, C-10_{KonformeresA/B}); 48.4 (s, C-8a); 49.7, 50.4 (t, C-12_{KonformeresA/B}) ; 52.2 (q, COOCH₃) ; 55.7 (q, OCH₃) ; 59.7 (d, C-6); 86.8 (d, C-4a) ; 111.8, 112.1 (S,C-1-_{KonformeresA/B}) ; 115.2 (d, C-8) ; 125.8, 126.0 (d, C-2_{KonformeressA/B)}; 128.1, 128.3 (S, C-12a_{KonformeresA/B}); 128.5, 128.6 (d, C-7_{KonformeresA/B}); 133.1 (s, C-12b); 143.9 (s, C-3a); 146.4 (s, C-3); 155.2 (s, CO)

### rac. N-Boc-Bromgalanthamin (63)

Zu einer Lösung von 1.0 g (2.84 mmol) rac. N-Demethylbromgalantliamin (4) und 620 mg (2.84 mmol) Pyrokohlensäuredi-t-butylester in 50 ml absolutem Tetrahydrofuran werden 286 mg (2.84 mmol) Triethylamin zugetropft und auf Rückfluss erhitzt. Nach 15 min. wird das Tetrahydrofuran abrotiert und der Rückstand in 50 ml Essigsäureethylester aufgenommen. Die organische Phase wird je einmal mit 2 N Salzsäure, gesättigter wässriger Natriumhydrogencarbonatlösung und gesättigter wässriger Natriumchloridlösung gewaschen, getrocknet (Na₂SO₄ und eingedampft, wodurch quantitativ farblose Kristalle an 63 erhalten werden.

DC: EtOAc:MeOH = 4:1

¹H-NMR (CDCl₃; δ (ppm)):
1.45 (s, 9H, t-Bu); 1.80 (dd, 1H, H-9); 2.05 (dd, 1H, H-9') ; 2.30 (ddd, 1H, H- 5); 2.65 (ddd, 1H, H-5'); 3.30 (ddd, 1H, H-10) ; 3.85 (s, 3H, OCH₃); 4.05 -4.30 (m,2H,H-6/10'); 4.10 (d, 1H, H-12, J_{(12,12')} = 15.1 Hz); 4.60 (dd, 1H, H-4a); 5.25 (d, 1H, H-12', J_{(12,12')} = 15.1 Hz); 5.90 (d, 1H, H-8, J_{(7,8)} = 8.9 Hz; 6.00(dd, 1H, H-7, J₍₇₈₎ = 8.9 Hz); 6.90 (s, 1H, H-2)

**Modifikationen an N-substituierten Galanthaminderivaten:**

| Substanznr. | Eduktnr. | R6 Edukt | R6 Produkt | R1 | Methode |
|---|---|---|---|---|---|
| 66 | 61 | 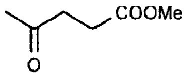 | 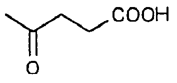 | Br | A |
| 67 | 60 | 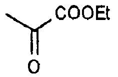 | 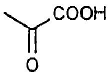 | Br | A |
| 71 | 51 | 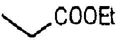 | 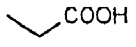 | Br | A |
| 68 | 51 | 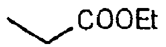 | 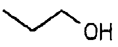 | Br | B |
| 69 | 51 | 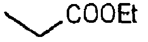 | 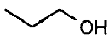 | H | C |
| 68 | 60 | 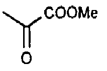 | 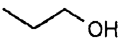 | Br | D |
| 70 | 55 | 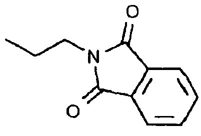 | 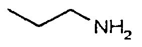 | Br | E |
| 65 | 59 | 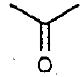 | 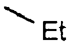 | H | F |

| Substanznr. | Name | SF,MG | Ausbeute | Smp. |
|---|---|---|---|---|
| 66 | (6R)-4a,5,9,10,11,12-Hexahydro-1-brom-6-hydroxy- 3 -methoxy-6H-benzofuro[3 a,3,2-ef] [2]-benzazepin-11-y-oxo-buttersäure | C₂₀H₂₂BrNO₆ [452.31] | 89 % gelbe Kristalle | 107-109°C |
| 67 | (6R)-4a,5,9,10,11,12-Hexahydro-1-brom-6-hydroxy-3-methoxy-6H-benzofuro[3a,3,2-ef] [2]-benzazepin-11-α-oxo-essigsäure | C₁₈H₁₈BrNO₆ [424.26] | 22 % rote Kristalle | Zersetzung > 120°C |
| 71 | (6R)-4a,5,9,10,11,12-Hexahydro 1-brom-6-hydroxy-3-methoxy-6H-benzofuro[3a,3,2-ef] [2]-benzazepin-11-essigsaure | C₁₈H₂₀BrNO₅ [410.27] | quant. farblose Kristalle | Zersetzung >200°C |

### Methode B:

Eine ca. 5 %ige Lösung des Eduktes in absolutem Tetrahydrofuran wird bei 0°C mit zwei Äquivalenten 10% iger Lithiunialuminiumhydridlösung in Tetrahydrofuran versetzt. Nach 1.5 Stdn. wird mit Wasser : Tetrahydrofuran 1:1 hydrolysiert, das Tetrahydrofuran abrotiert und der Rückstand in 2 N Salzsäure gelöst. Nach der Zugabe von 2.5 Äquivalenten Weinsäure wird mit konzentriertem wässrigen Ammoniak basisch gemacht und mit Essigsäureethylester extrahiert. Die vereinigten organischen Phasen werden einmal mit gesättigter wässriger Natriumchloridlösung gewaschen, getrocknet (Na₂SO₄), filtriert und eingedampft. Das Rohprodukt wird über Säulenchromatographie (15 g Kieselgel G60, Laufmittel: CHCl₃ : MeOH = 9:1) gereinigt.

DC: CHCl₃: MeOH = 9:1

| Substanznr. | Name | SF, MG | Ausbeute | Smp. |
|---|---|---|---|---|
| 68 | (6R)-4a,5,9,10,11,12-Hexahydro 1 -brom-3-methoxy-11-(2-hydroxyethyl)-6H- benzofuro[3 a,3,2-ef][2]-benzazepin-6-ol | C₁₈H₂₂BrN0₄ [396.29] | quantitativ ölige ubstanz | - |

### Methode C:

Eine ca. 5 %ige Lösung des Eduktes in absolutem Tetrahydrofuran wird bei 0°C mit vier Äquivalenten 10 %iger Lithiumaluminiumhydridlösung in Tetrahydrofuran versetzt. Nach 15 min. wird auf Rückfluss erhitzt. Nach 24 Stdn. wird mit Wasser: Tetrahydrofuran 1:1 hydrolysiert, das Tetrahydrofuran abrotiert und der Rückstand in 2 N Salzsäure gelöst. Nach der Zugabe von fünf Äquivalenten Weinsäure wird mit konzentriertem wässrigen Ammoniak basisch gemacht und mit Essigsäureethylester extrahiert. Die vereinigten organischen Phasen werden einmal mit gesättigter wässriger Natriumchloridlösung gewaschen, getrocknet (Na₂SO₄), filtriert und eingedampft. Das Rohprodukt wird über Säulenchromatographie (15 g Kieselgel G60, Laufmittel: CHCl₃: MeOH = 9:1) gereinigt.

DC: CHCl₃: MeOH = 9:1

| Substanznr. | Name | SF,MG | Ausbeute | Smp. |
|---|---|---|---|---|
| 69 | (6R)-4a,5,9,10,11,12-Hexahydro-3-methoxy-11-(2-hydroxyethyl)-6H-benzofuro[3 a,3,2-ef][2]-benzazepin-6-ol | C₁₈H₂₃NO₄ [317.39] | 81 % ölige Substanz | - |

### Methode D:

0.84 ml 10 %ige Lithiumaluminiumhydridlösung (2.20 mmol) in 10 ml absolutem Tetrahydrofuran werden auf Rückfluss erhitzt. Dann werden 100 mg (0.22 mmol) mt7 in 5 ml absolutem Tetrahydrofuran gelöst und der siedenden Lösung zugetropft. Nach 15 Min. wird die Reaktionsmischung auf 0°C abgekühlt und mit Wasser: Tetrahydrofuran 1:1 hydrolisiert. Anschließend wird das Tetrahydrofuran abrotiert, der Rückstand in 50 ml 2 N Salzsäure aufgenommen, mit 0.80 g Weinsäure versetzt, mit konzentriertem wässrigen Ammoniak basisch gemacht und dreimal mit je 30 ml Essigsäureethylester extrahiert. Die vereinigten organischen Phasen werden einmal mit gesättigter wässriger Natriumchloridlösung gewaschen, getrocknet (Na₂SO₄), filtriert und eingedampft, wodurch 100 mg Rohprodukt erhalten werden, das durch Säulenchromatographie (15 g Kieselgel, Laufmittel: CHCl₃: MeOH = 9:1) gereinigt wird.

DC: CHCl₃:MeOH = 9:1

| Substanznr. | Name | SF, MG | Ausbeute | Smp. |
|---|---|---|---|---|
| 68 | (6R)-4a,5,9,10,11,12-Hexahydro-1-brom-3 -methoxy-1 1-(2-hydroxyethyl)-6H-benzofuro[3 a,3,2-ef] [2]-benzazepin-6-ol | C₁₈H₂₂BrNO₄ [396.29] | 42% ölige Substanz | - |

### Methode E:

170 mg (0.32 mmol) st80 und 80 mg (1.60 mmol) werden in 10 ml absolutem Ethanol auf Rückfluss erhitzt. Nach 30 Min. wird die Reaktionsmischung abgekühlt und nach 1 Std. der entstandene Niederschlag abgesaugt. Der Niederschlag wird einmal mit Ethanol nachgewaschen und die ethanolische Phase anschließend einrotiert. Das Rohprodukt wird durch Säulenchromatographie gereinigt (15 g Kieselgel, Laufmittel: CHCl₃: MeOH = 9:1).

DC: CHCl₃: MeOH = 9:1

| Substanznr. | Name | SF, MG | Ausbeute | Smp. |
|---|---|---|---|---|
| 70 | (6R)-4a,5,9,10,11,12-Hexahydro-1 -brom-3-methoxy-11 -(2-aminoethyl)-6H-benzofuro[3 a,3,2-ef] [2]-benzazepin-6-ol | C₁₈H₂₃BrN₂O₃ [395.30] | 70 % farblose Kristalle | 116-117°C |

### Methode F:

Zu 2 ml 10 %ige Lithiumaluminiumhydridlösung in Tetrahydrofuran (5,26 mmol) werden unter Rückfluss 50 mg (0,381 mmol) st62 in 1,5 ml absolutem Tetrahydrofuran zugetropft. Anschließend wird weitere 90 Minuten auf Rückfluss erhitzt. Danach wird bei 0 °C mit Wasser : Tetrahydrofuran = 1:1 hydrolysiert und das Gemisch zur Trockene einrotiert. Anschließend wird der Rückstand in 2 N Salzsäure aufgenommen, mit 1,2 g Weinsäure versetzt und mit konzentriertem wässrigem Ammoniak basisch gemacht. Danach wird dreimal mit je 40 ml Essigsäureethylester extrahiert, die vereinigten organischen Phasen werden einmal mit gesättigter wässriger Natriumchloridlösung gewaschen, getrocknet (Na₂SO₄), filtriert und eingedampft. Das Rohprodukt wird durch Säulenchromatographie gereinigt (15 g Kieselgel, Laufmittel: CHCl₃: MeOH = 9:1).

DC: CHCl₃: MeOH = 9:1

| Substanznr. | Name | SF,MG | Ausbeute | Smp. |
|---|---|---|---|---|
| 65 | (6R)-4a,5,9,10,11,12-Hexahydro-1-brom-3-methoxy-1 1-ethyl-6H-benzofuro[3 a,3,2-ef] [2]-benzazepin-6-ol | C₁₈H₂₂NO₃ [300.38] | 76 % ölige Substanz | - |

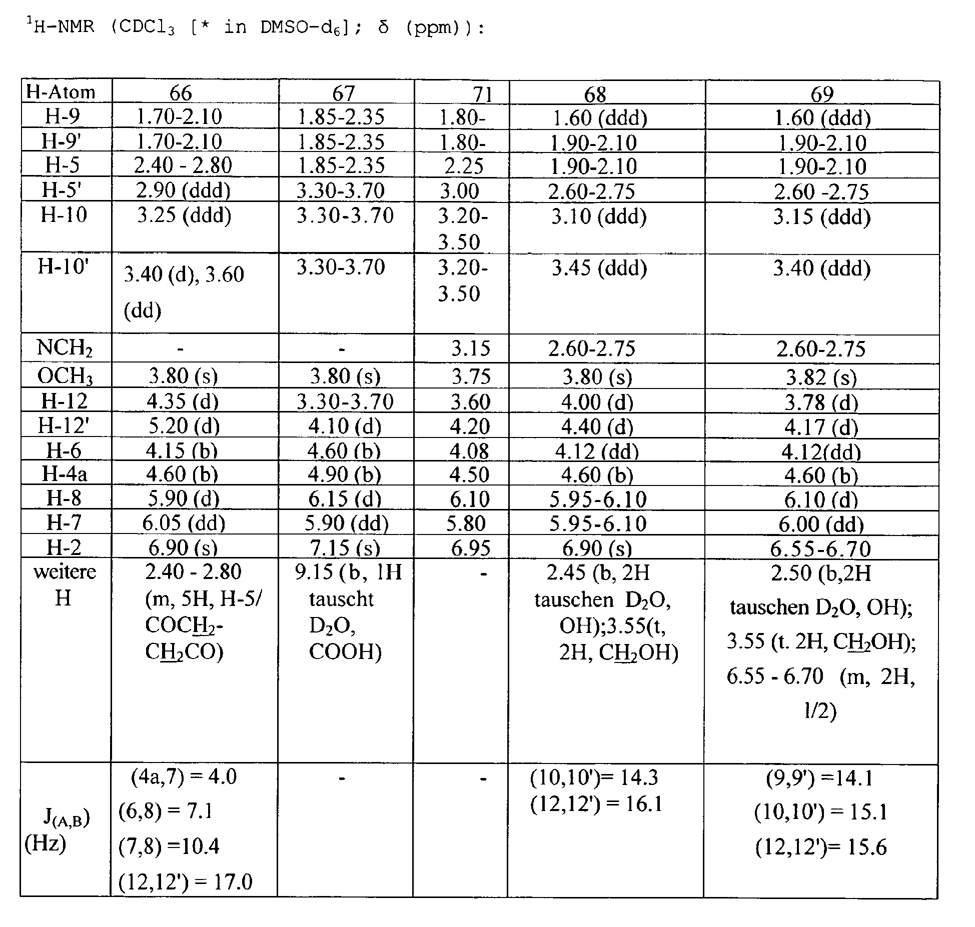

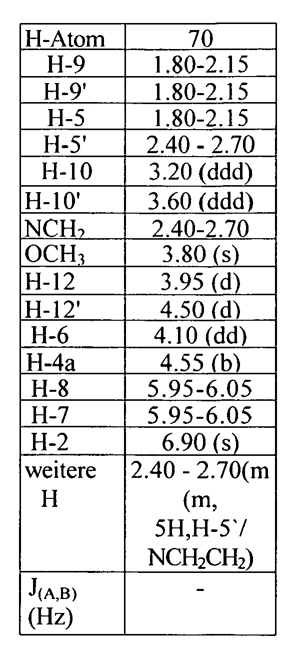

**¹³C-NMR (CDCI₃ [* in DMSO-d₆] ; δ (ppm)) :**

| C-Atom | 66* | 68 | 69 | 70 | 65 |
|---|---|---|---|---|---|
| C-5 | 28.8, 30.2 (t) | 29.4 (t) | 29.7 (t) | (t) | (t) |
| C-9 | 36.0, 37.8 (t) | 33.2 (t) | 33.2 (t) | (t) | (t) |
| C-8a | 48.4 (s) | 48.6 (s) | 48.2 (s) | (s) | (s) |
| C-10 | 43.6,45.4(t) | 51.7 (t) | 51.7 (t) | (t) | (t) |
| NCH₂ | - | 54.9 (t) | 52.0 (t) | (t) | (t) |
| OCH3 | 55.8 (q) | 55.7 (q) | 55.6 (q) | (q) | (q) |
| C-12 | 48.8. 50.4 (t) | 57.6 (t) | 57.6 (t) | (t) | (t) |
| C-6 | 59.3 (d) | 61.4 (d) | 61.7 (d) | (d) | (d) |
| C-4a | 86.4, 86.6 (d) | 88.3 (d) | 88.4 (d) | (d) | (d) |
| C-1 | 111.0. 112.1 (s) | 1143 (s) | 121.8 (d) | | |
| C-8 | 115.3 (d) | 115.4 (d) | 110.9 (d) | (d) | (d) |
| C-2 | 128.4, 128.6 (d) | 121.7 (d) | 126.4 (d) | (d) | (d) |
| C-7 | 126.3 (d) | 127.9 (d) | 127.5 (d) | (d) | (d) |
| C-12a | 127.4 (s) | 127.3 (s) | 128.8 (s) | (s) | (s) |
| C-12b | 133.2, 133.4 (s) | 133.7 (s) | 132.8 (s) | (s) | (s) |
| C-3a | 143.8, 144.2 (s) | 144.0 (s) | 144.0 (s) | (s) | (s) |
| C-3 | 146.5, 146.9 (s) | 145.2 (s) | 145.7 (s) | (s) | (s) |

| | | | |
|---|---|---|---|
| weitere C | 27.4 (t, NCO- | 56.6 (t, | 56.7 (t, |
| | CH₂); 27.9 (t, CH₂COOH); 170.0, 170.4 (s, CON); 173.6, 173.8 (s, COO) | CH₂OH) | CH₂OH) |

Allgemeine Arbeitsvorschrift zur Bromabspaltung mit Zink und Calziumchlorid:

| Substanznr | Eduktnr. | R₄ | R5 | R6 | SF, MG |
|---|---|---|---|---|---|
| 112 | 4 | OH | H | 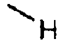 | C₁₆H₁₉N0₃ [273.22] |
| 73 | 52 | OH | H | 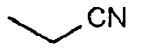 | C₁₈H₂₀N₂O₃ [312.37] |
| 74 | 54 | OH | H | 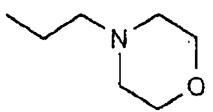 | C₂₂H₃₀N₂O₄ [386.50] |
| 43 | 42 | OH | H | 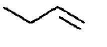 | C₁₉H₂₃NO₃ [313.40] |
| 46 | 45 | OH | H | 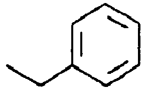 | C₂₃H₂₅NO₃ [363.46] |
| 72 | 64 | OH | H | 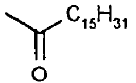 | C₃₂H₄₉NO₄ [511.75] |
| 47 | 44 | =0 | | 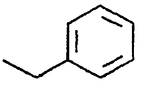 | C₂₃H₂₃NO₃ [361.44] |

Eine Lösung von 500 mg Edukt und 1.0 g Calziumchlorid in 50 ml 50 %igem Ethanol wird mit 2.0 g frisch aktiviertem Zinkpulver versetzt und auf Rückfluss erhitzt. Anschließend wird das überschüssige Zink abfiltriert, mit Methanol nachgewaschen und die Restlösung einrotiert. Der Rückstand wird in 100 ml 1N Salzsäure aufgenommen, mit konzentriertem wässrigem Ammoniak basisch gemacht und mit dreimal 50 ml Essigsäureethylester extrahiert. Die vereinigten organischen Phasen werden einmal mit gesättigter wässriger Natriumchloridlösung gewaschen, getrocknet (Na₂SO₄), filtriert und eingedampft. Das Rohprodukt wird durch Säulenchromatographie (15 g Kieselgel, Laufmittel: CHCl₃: MeOH = 9:1) gereinigt.

| Substanznr. | Name | Reaktionszeit | Ausbeute | Smp. |
|---|---|---|---|---|
| 112 | (6R)-4a,5,9,10,11,12-Hexahydro- 1-brom-3-methoxy-6H-benzofuro[3a,3,2-ef][2]-benzazepin-6-ol | 1.5 h | 93 % farblose Kristalle | 236-240°C |
| 73 | (6R)-4a,5,9,10,11,12-Hexahydro-3-methoxy-11-(cyanomethyl)- 6H-benzofuro [3 a, 3,2-ef] [2]-benzazepin-6-ol | 3 h | 55 % farblose Kristalle | 68-70°C |
| 74 | (6R)-4a,5,9,10,11,12-Hexahydro-3-methoxy-11-(2-morpholino ethyl)-6H-benzofuro[3a,3,2-ef][2]benzazepin-6-ol | 3h | 80% | |
| 72 | (6R)-4a,5,9,10,11,12-Hexahydro-3-methoxy-11-(1-oxohexadecyl)-6H-benzofuro[3a,3,2-ef] [2]benzazepin-6-ol | 3h | 84 % farblose Kristalle | |
| 43 | (6R)-4a,5,9,10,11,12-Hexahydro-3-methoxy-11-(2-propenyl)-6H-benzofuro[3a,3,2-ef] [2]-benzazepin-6-ol | 3h | 96% | |
| 46 | (6R)-4a,5,9,10,11,12-Hexahydro-3-methoxy-11-(phenylmethyl)- 6H-benzofuro[3a,3,2-ef][2]-benzazepin-6-ol | 3h | 52% | |
| 47 | (6R)-4a,5,9,10,11,12-Hexahydro-3-methoxy-11-(phenylmethyl)-6H-benzofuro[3a,3,2-ef][2]-benzazepin-6-on | 3.5 h | quantitativ orange Kristalle | 159-162°C |

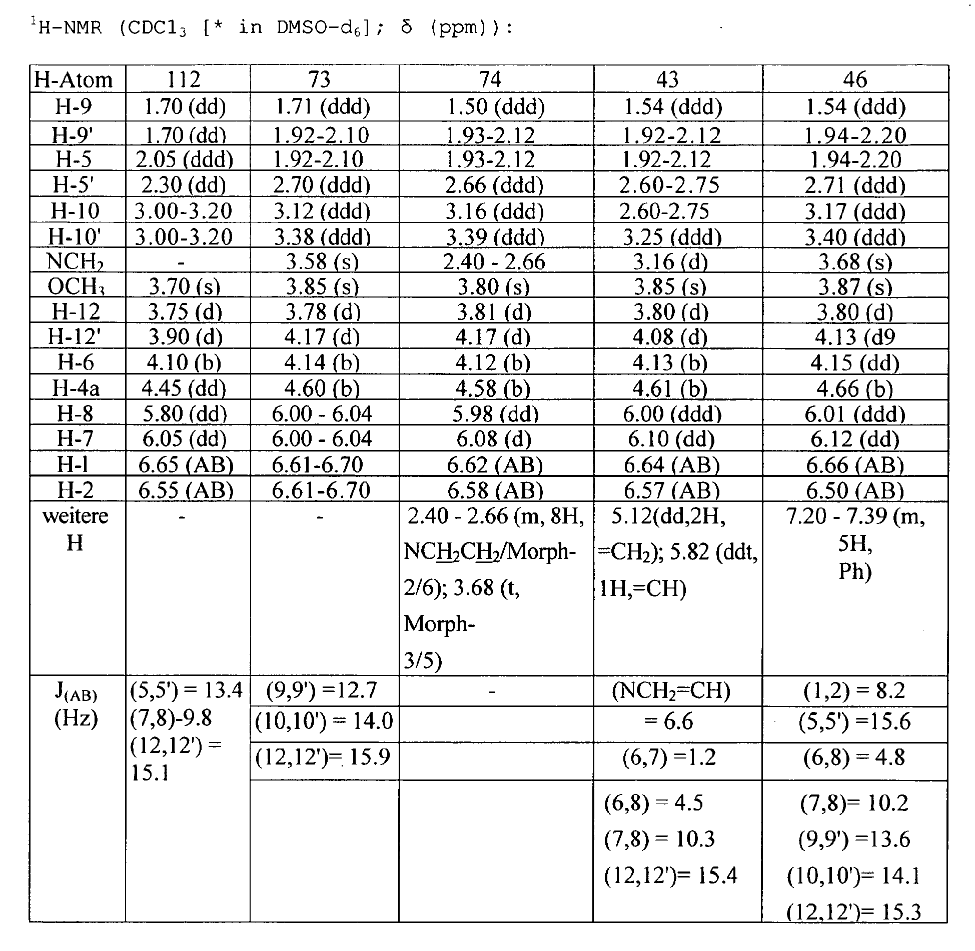

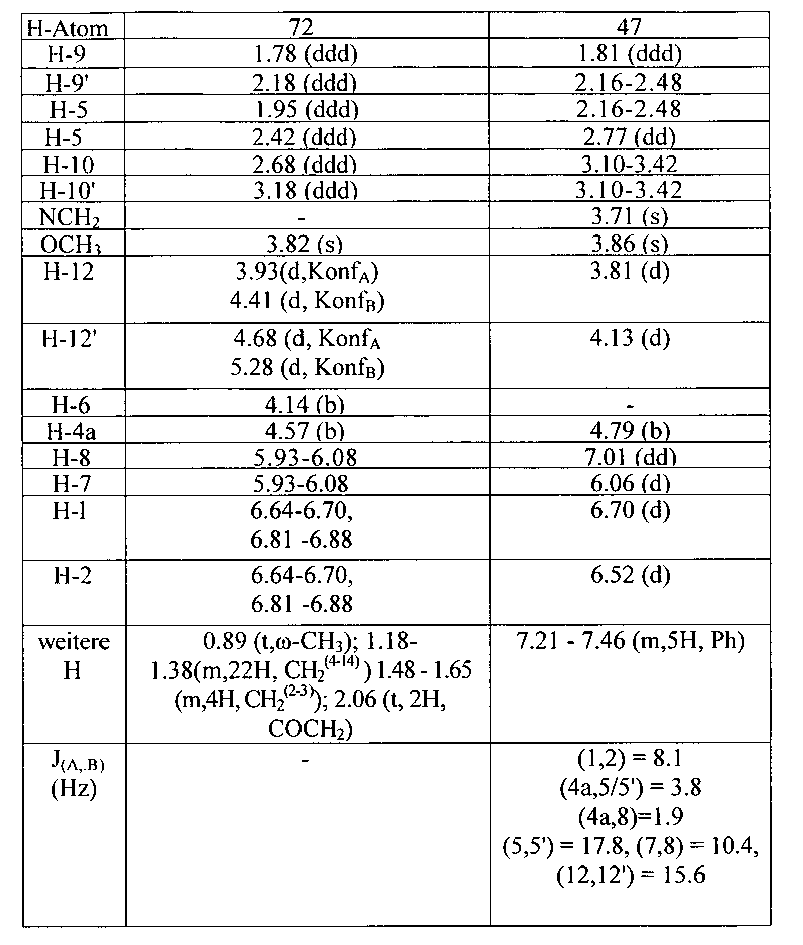

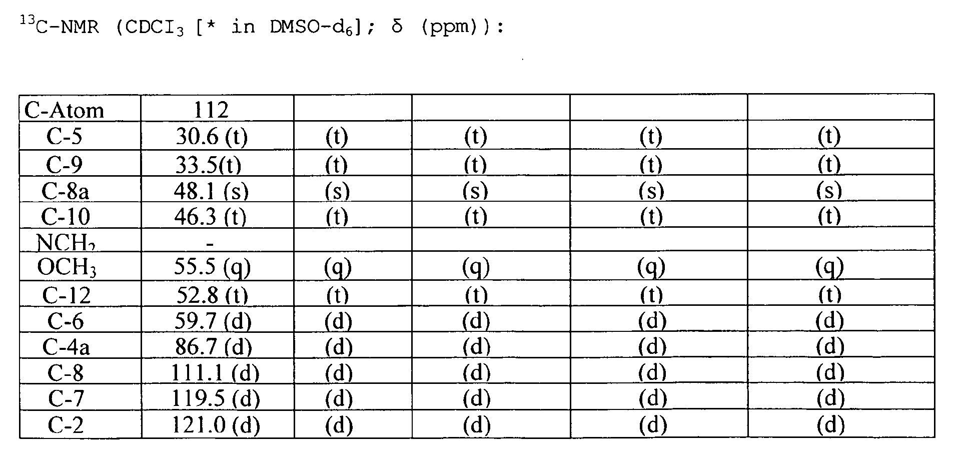

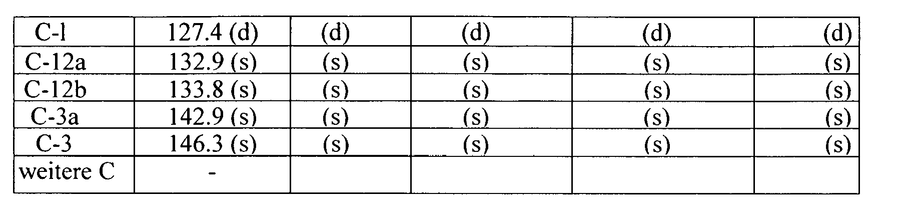

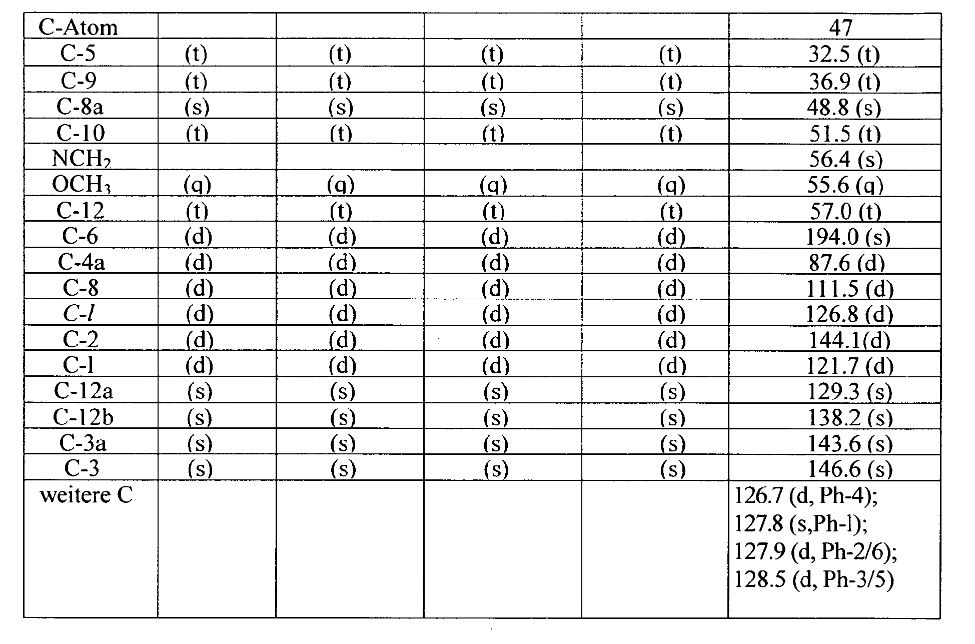

### O-TOS-Narwedinoxim (75):

Eine Suspension von 1.05 g (3.51 mmol) Narwedinoxim (76,77) in 20 ml absolutem Pyridin wird mit 1.33 g (7.02 mmol) p-Toluolsulfonsäurechlorid versetzt und 20 Stdn. bei Raumtemp. gerührt. Anschließend wird das Reaktionsgemisch auf 100 ml Wasser gegossen und je dreimal mit 50 ml Essigsäureethylester extrahiert. Die vereinigten organischen Phasen werden einmal mit gesättigter wässriger Natriumchloridlösung gewaschen, getrocknet (N₂SO₄, Aktivkohle), filtriert und eingedampft. Die Reinigung erfolgt über Säulenchromatographie (50 g Kieselgel, Laufmittel: CHCl₃→ CHCl₃: MeOH = 9:1), wodurch 1.27 g (80 % d. Th.) gelbe Kristalle vom Schmp. 78 - 79°C an 75 erhalten werden.

DC: CHCl₃: MeOH = 9:1

¹H-NMR (CDCl₃; δ (ppm)) :
1.55-1.65, 1.80 - 1.95 (2* m,2H, H-9/9' _{KonformereaA/B)}; 2.05 - 2.25 (m, 1H, H-5_{KonformeresA/B}); 2.40,2.43 (2* s, 6H, NCH₃, Ph-CH)₃; 2.50 - 2.70 (m, 1H, H-5_{KonformeresA/B}); 2.95 - 3.25 (m, 1H, H-10_{KonformeresA/B}); 3. 60 3.85(m,2H, H-10' /12 _{KonformeresA/B}); 4.00-4, 25 (m,1H, H12_{Konformeres(A/B}); 4.55 (b, 1H, H-4a_{KonformeresA/B}); 6.15, 7.10 (2*d, 1H, H-8_{KonformeresA/B}); 6.40, 7.65 (2*d, 1H, H-7 _{KonformeresA/B}); 6.50-6.70 (m, 2H, H-1/2 _{Konformeres A/B}); 7.20-7.35 (m, 2H, Ph-3/5 _{KonformeresA/B}); 7.75_7.90 (m, 2H, Ph_2/6 _{KonformeresA/B})

13_{C-NMR} (DMSO-d₆; δ (ppm)) :
21.1 (q,Ph-CH₃); 23.9 (t, C-5); 31.6 (t, C-9); 40.6 (q,NCH₃); 48.7 (s, C-8a); 52.9 (t, C-10); 55.5 (q, OCH₃); 59.2 (t, C-12); 84. 3 (d, C-4a) ; 111.9 (d, C-2); 118. 6, 121 . 6 (d, C-8_{KonformeresA/B}); 125. 5, 128.0 (d, C-7_{KonformeresA/B})(d, Ph-2/6); 130.0 (d, Ph-3/5) ; 131.8 (s, C-12a); 136.1 (s, Ph-1); (s, C-12b); 138.7 (d, C-1); 143.1 (s, C-3a); 145.4 (s, C-3); 145.8 (s, )Ph-4); 159.8 (s, C-6)

### rac, (-)- und (+)-0-Methylnarwedinoxim (78,79):

Eine Lösung von 300 mg (1.05 mmol) Narwedin in 10 ml Ethanol wird mit 88 mg (1.05 mmol) O-Methylhydroxylamin und 53 mg (0.53 mmol) Kaliumhydrogencarbonat versetzt und für 4 Stdn. auf Rückfluss erhitzt. Anschließend wird das Reaktionsgemisch eingedampft, der Rückstand in 50 ml 1 N Salzsäure aufgenommen, mit konzentriertem wässrigen Ammoniak basisch gemacht und dreimal mit je 30 ml Essigsäureethylester extrahiert. Die vereinigten organischen Phasen werden einmal mit gesättigter wässriger Natnumchloridlösung gewaschen, getrocknet (Na₂SO₄), filtriert und eingedampft, wodurch quantitativ zähe amorphe Substanz (mit den Drehwerten α_{D}²⁰[CHCl₃] =-152° für 78 bzw. α_{D}²⁰[CHCl₃] = +108° für 79) an 78/79 erhalten werden.

DC: CHCl₃:MeOH = 9:1

¹H-NMR (CDCl₃; δ (ppm)) : 1.70 (ddd, 1H, H-9) ; 2.20 (ddd, 1H, H-9'); 2.30 - 2.45 (m, 1H, H-5) ; 2.40 (s, 3H, NCH₃) ; 2.70 (ddd, 1H, H-5'); 3.00 - 3.35 (m, 2H, H-10/10'); 3.65, 3.70, 4.00, 4. 10 (4* d, 2H, H-12_{Konformeres A/B} /12 _{Konformeres A/B}) : 3. 80 (s, 3H, OCH₃) ; 3.85, 3. 90 (2* S, 3H, N-OCH _{KonformeresA/B}) ; 4.60 (b, 1H, H-4a) ; 6.15, 6.20, 6.75 (s, d, d, 2H, H-7/8_{Konformeres A/B}); 6.55 - 6.70 (m, 2H, H-1/2)

### Narwedinimin (80):

Eine Lösung von 100 mg (0.35 mmol) Narwedin in 10 ml 7 N methanolischem Ammoniak wird im Glasautoklaven für 10 Stdn. auf 100°C erhitzt. Anschließend wird der überschüssige Methanol abrotiert, wodurch quantitativ farblose Kristalle vom Schmp. 105 - 110°C an 80 erhalten werden.

DC: CHCl₃:MeOH = 9:1

¹H-NMR (CDCl₃ [Bildung von Narwedin und Zersetzungsprodukten während der Messung];
δ (ppm)): 1.80 (ddd, 1H, H-9); 2.00 - 2.35 (m, 2H, H-5/9'); 2.45 (s, 3H, NCH₃); 2.80 (ddd, 1H, H-5'); 3.00 - 3.35 (m, 2H, H-10/10'); 3.70 (d, 1H, H-12); 3.80 (s, 2H, OCH₃); 4.05 (d, 1H, H-12'); 4.65 (b, 1H, H-4a); 6.15 (d, 1H, H-8); 6.45 (d, 1H, H-7); 6.55 - 6.70 (m, 2H, H-1/2)

### rac, (+)- oder (-)-Narwedinoxim (76,77):

1.0 g (3.51 mmol) Narwedin, 266 mg (3.86 mmol) Hydroxylaminhydrochlorid und 193 mg (1.93 mmol) Kaliumhydrogencarbonat werden in 30 ml 96 %igem Ethanol auf Rückfluss erhitzt. Nach 3 Stdn. wird das Reaktionsgemisch einrotiert, der Rückstand in 50 ml 2 N Salzsäure aufgenommen und mit konzentriertem wässrigen Ammoniak das Produkt gefällt. Nach kristallisieren über Nacht wird eine erste Fraktion von 0.81 g (81 % d. Th.) erhalten. Durch Exrahieren der Mutterlauge mit dreimal 30 ml Essigsäureethylester wird eine zweite Fraktion erhalten, wodurch quantitativ farblose Kristalle vom Schmp. 170 - 171 °C an 76,77 erhalten werden.

| | a_{D}²⁰[CHCl₃] | ee nach CE (CE=Kapillar -Elektrophorese |
|---|---|---|
| (-)-Narwedinoxim (77) | -79° | 20% |
| (+)-Narwedinoxim (76) | +126° | 12% |

DC: CHCl₃:MeOH = 9:1

¹H-NMR (CDCl₃; δ (ppm)) :
1.70 (dd, 1H, H-9, J_{(9,9')} = 13.4 Hz) ; 2.20 (ddd, 1H, H-9' J_{(9,9')} = 13.4 Hz); 2.40 (s, 3H, NCH₃); 2.45 (dd, 1H, H-5, J_{(5,5)} = 16.9 Hz) ; 3.10 (m, 2H, H-5', J_{(5,5)} = 16.9 Hz) ; 3.30 (ddd, 1H, H-10, J₍₁₀₁₀₎ = 14.2 Hz); 3.75 (d, 1H, H-12, J_{(12,12')} = 16.0 Hz) ; 3.80 (s, 3H, OCH₃) ; 3.85 (dd, 1H, H-10', J_{(10,10'}) = 14.2 Hz); 4.10 (d, 1H, H-12', J_{(12,12')} = 16.0 Hz); 4.65 (b, 1H, H-4a); 6.20 (b, 2H, H-7/8); 6.55 - 6.65 (m, 2H, H-1/2)

¹³C-NMR (DMSO-d₆; δ (ppm)):
22.3 (t, C-5); 32.8 (t, C-9); 41.2 (q, NCH₃) ; 48.7 (s, C-8a); 53.1 (t, C-10); 55.5 (q, OCH₃) ; 59.5 (t, C-12); 85.9 (d, C-4a); 111.6 (d, C-8); 121.1 (d, C-2); 122.5 (d, C-7); 129.5 (s, C-12a); 130.7 (d, C-1); 132.5 (s, C-12b); 143.1 (s, C-3a); 145.8 (s, C-3); 150.1 (s, C-6)

**Umsetzung von Narwedin mit Hydrazinen und Hydraziden:**

| Substanznr. | Rest R₄, R₅ | Name | Summenformel, MG |
|---|---|---|---|
| 81 | 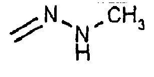 | 4a,5,9,10,11,12-Hexahydro-3-methoxy- 11-methyl-6H-benzofuro[3a,3,2- ef][2]benzazepin-6-on 2- Methylhydrazon | C₁₈H₂₃N₃O₂ [313.40] |
| 84 | 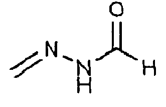 | Ameisensäure-2-{4a,5,9,10,11,12-hexahydro-3-methoxy-11-methyl-6H-benzofuro[3a,3,2-ef] [2]benzazepin-6-yliden} hydrazid | C₁₈H₂₁N₃O₃ [327.39] |
| 83 | 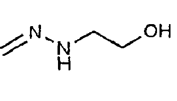 | 4a,5,9,10,11,12-Hexahydro-3-methoxy-1 1-methyl-6H-benzofuro[3a,3,2-ef] [2]benzazepin-6-on 2-(2- Hydroxyethyl)hydrazon | C₁₉H₂₅N₃O₃ [343.43] |
| 86 | 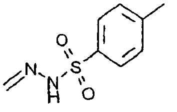 | 4-Methylbenzolsulfonsäure-2-{4a,5,9,10,11,12-hexahydro-3-methoxy-11-methyl-6H-benzofuro[3a,3,2-ef][2]- benzazepin-6-yliden} hydrazid | C₂₄H₂₇N₃O₄S [453.56] |
| 85 | 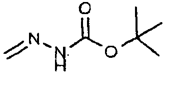 | Pyrokohlensäure-t-butylester-2-{4a,5,9,10,11, 12-hexahydro-3-methoxy-11 -methyl-6H-benzofuro[3 a,3,2-ef] [2]- benzazepin-6-yliden} hydrazid | C₂₂H₂₉N₃O₄ [399.49] |
| 89 | 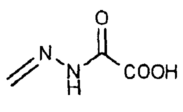 | Pyrokohlensäure-2-{4a,5,9,1 0,11,12-hexahydro-3-methoxy-11-methyl-6H-benzofuro[3a,3,2-ef][2]benzazepin-6-yliden} hydrazid | C₁₉H₂₁N₃O₅ [371.40] |
| 82 | 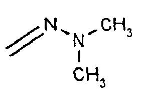 | 4a,5,9,10,11,12-Hexahydro-3-methoxy-11-methyl-6H-benzofuro[3 a,3,2-ef] [2]- benzazepin-6-on2,2-Dimethylhydrazon | C₁₉H₂₅N₃O₂ [327.43] |
| 88 | 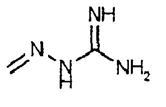 | 2-{4a,5,9,10,11,12-Hexahydro-3-methoxy-1 1-methyl-6H-benzofuro[3a,3,2-ef] [2]benzazepin-6- yliden} -hydrazincarboximidamid | C₁₈H₂₃N₅O₂ [341.42] |
| 90 | 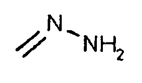 | 4a,5,9,10,11,12-Hexahydro- 3-methoxy-11-methyl-6H-benzofuro[3a,3,2-ef] [2]benzazepin-6-on Hydrazon | C₁₇H₂₁N₃O₂ [299.38] |
| 87 | 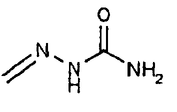 | Carbaminsäure-2-{4a,5,9,10,11,12-hexahydro-3-methoxy-1 1-methyl-6H-benzofuro[3a,3,2-ef][2]benzazepin-6-yliden} hydrazid | C₁₈H₂₂N₄O₃ [342.40] |

Methode: Eine Lösung von 500 mg (1.75 mmol) Narwedin und 1.1 bis 1.2 Äquivalenten N-Alkylhydrazon bzw. Säurehydrazid in 10 ml Ethanol wird mit 0.25 Äquivalenten (43 mg, 0.44 mmol) konzentrierter Schwefelsäure versetzt und auf Rückfluss erhitzt. Anschließend wird das Reaktionsgemisch eingedampft, der Rückstand in 50 ml 1 N Salzsäure aufgenommen, mit konzentriertem wässrigen Ammoniak basisch gemacht und der entstandene Niederschlag abgesaugt oder die wässrige Phase dreimal mit je 30 ml Essigsäureethylester extrahiert. Der Niederschlag wird bei 50 °C / 50 mbar getrocknet, die vereinigten organischen Phasen werden einmal mit gesättigter wässriger Natriumchloridlösung gewaschen, getrocknet (Na₂SO₄), filtriert und eingedampft.

DC: CHCl₃:MeOH = 9:1

| Substanznr. | Reagens; äqu. H₂SO₄ | Reaktionszeit | Ausbeute | Smp. |
|---|---|---|---|---|
| 81 | Methylhydrazin; 0.25 | 4h | 76 % gelbe Kristalle | 97-99°C |
| 84 | Ameisensäurehydrazid; 0.0 | 48 h | 63 % gelbe Kristalle | 145-148°C |
| 83 | 2-Hydrazinoethanol; 0.0 | 30 h | 61 % gelbe Kristalle | 100-105°C |
| 86 | p-Toluolsulfonsäurehydrazid; 0.25 | 6h | 97 % farblose Kristalle | 210-212°C |
| 85 | t-Butylcarbazat; 0.25 | 4h | quantitativ farblose Kristalle | Umwandlung bei 155-160°C, Zersetzung >200°C |
| 89 | Oxalsäureethylester- | 30 h | 64 % gelbe Kristalle | 189- 191 °C |
| 82 | hydrazid; 0.25 N,N-Dimethylhydr azin; | 12 h | 78 % ölige Substanz | _ |
| 88 | 0.25 Aminoguanidin | 20 h | quantitativ gelbe | 112 - 113°C |
| 90 | Hydrogencarbonat; 0.0 10 Äqu. Hydrazinhydrat; | 2h | Kristalle 94 % ölige Substanz | - |
| 87 | 2.5 Semicarbazid Hydrochlorid; 0.5 Äqu. KHCO₃ | 4h | 88 % farblose Kristalle (Lit. [] % d. Th.) | Zersetzung ab 225°C (Lit. []Zers. ab °C) |

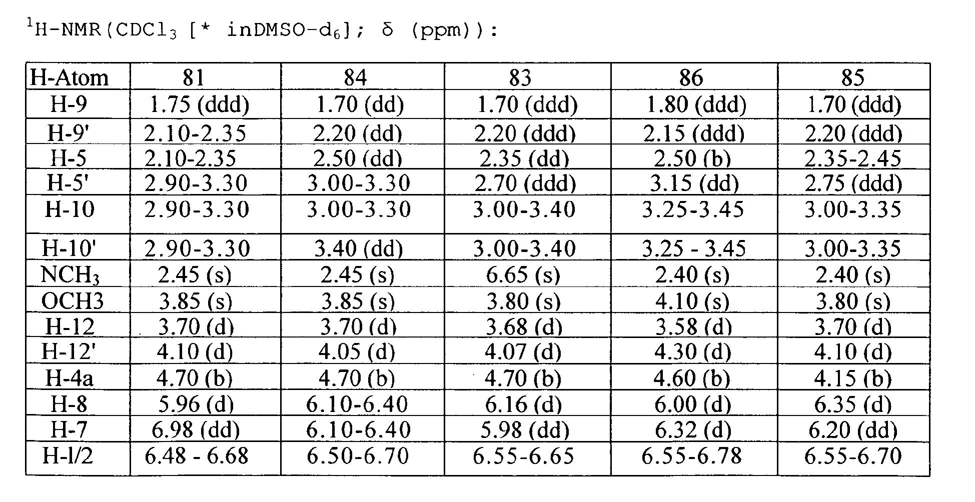

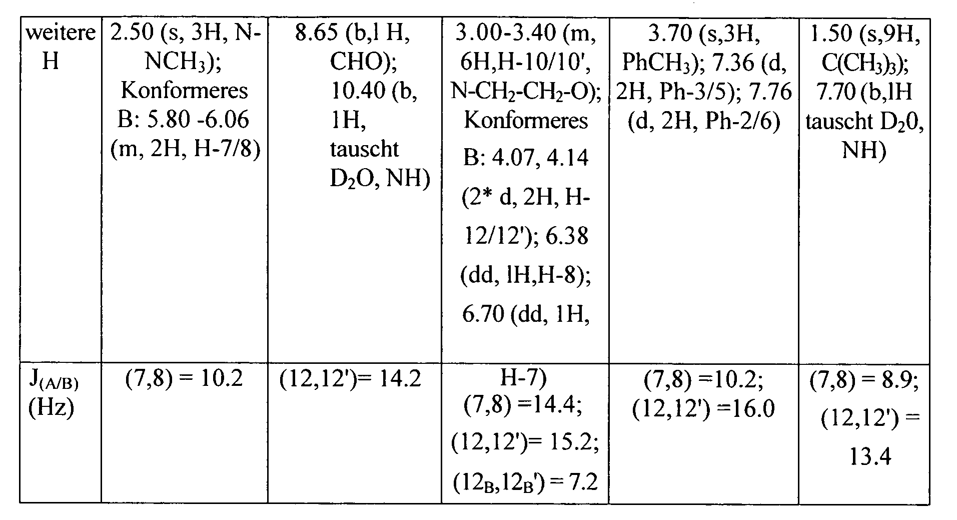

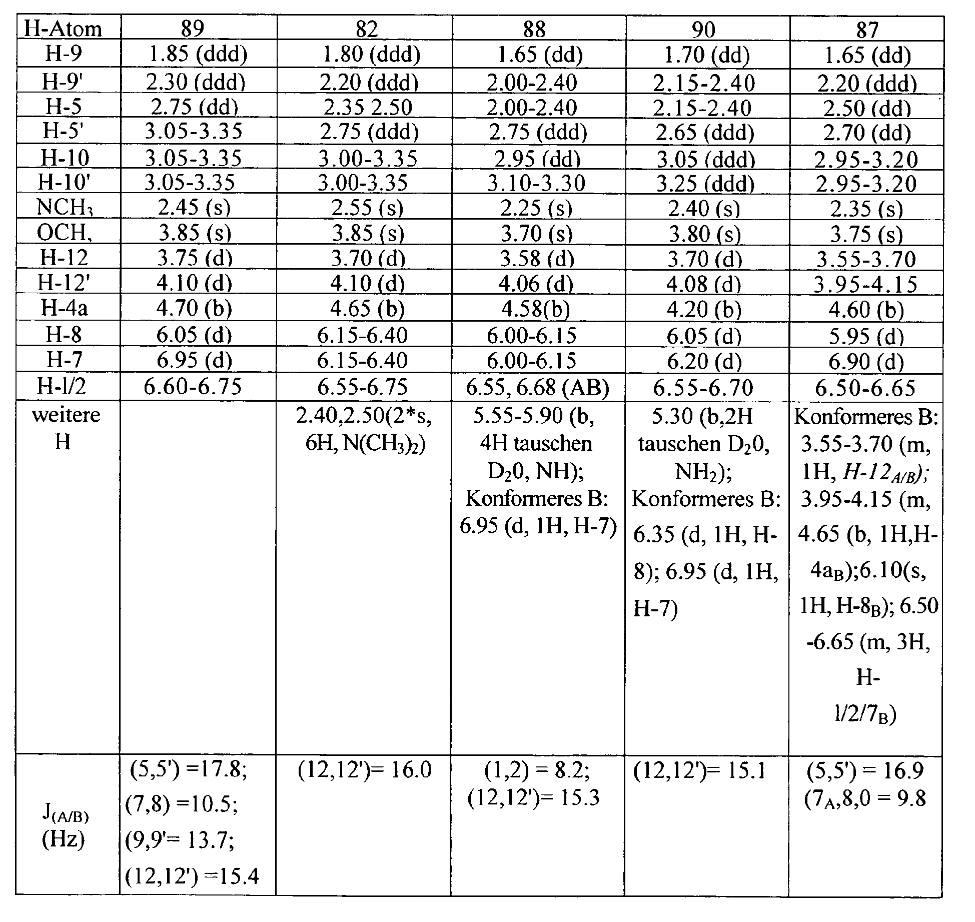

13_{C-NMR} (CDCI₃ [* in DMSO-d₆] ; δ (ppm)):
86: 24.8 (t, C-5); 31.7 (t, C-9); 41.2 (q, NCH₃); 53.0 (t, C-10); 47.8 (s, C-8a); 55.5 (q, OCH₃) ; 58.8 (t, C-12); 85.5 (d, C-4a); 111.9 (d, C-8); 122.3 (d, C-2); 125.0 (d, C-7); 125.2 (s, Ph-1); 127.5 (d, Ph-2/6); 129.5 (d, Ph-3/5); 132.2 (d, C-1); 132.3 (s, C-12a); 136.2 (s, C-12b); 143.3 (s, C-3a); 143.8 (s, Ph-4); 145.8 (s, C-3); 149.8 (s, C-6)
85 *: 24.5 (t, C-5) ; 28.1 (q, C(CH₃)₃); 32.4 (t, C-9) ; 41.2 (q, NCH₃) ; 48.2 (s, C-8a); 53.1 (t, C-10); 55.5 (q, OCH₃) ; 59.3 (t, C-12); 79.4 (s, C(CH₃)₃) ; 86.0 (d, C-4a); 111.7 (d, C-8); 121.5 (d, C-2); 125.5 (d, C-7); 131.2 (d, C-1); 128.5 (s, C-12a); 132.5 (s, C-12b); 143.3 (s, C-3a); 145.6 (s, C-3); 145.8 (s, C-6); 153.0 (s, CO)

**(-)-Alkylgalanthaminium-halogenid**

| Produkt | Summenformel | Bezeichnung | R |
|---|---|---|---|
| | C₂₂H₃₁BrNO₃ [401,95] | (-)-Pentylgalanthaminium-bromid | 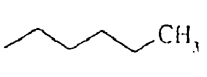 |
| 91 | C₂₁H₃₀ClN₂O₃ [358,49] | (-)-2-Dimethylaminoethy]galanthaminium-chlorid | 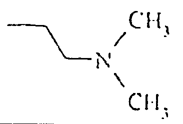 |
| 92 | C₂₃H₃₂CIN₂O₃ [419,97] | (-)-2-Pyrrolidin-N-ethylgalanthaminium-chlorid | 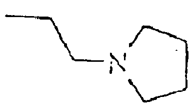 |
| 93 | C₂₃H₃₂ClN₂O₄ [435,97] | (-)-2-Morpholin-N-elhylga!anthaminium-chlorid | 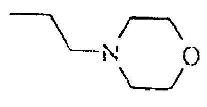 |
| 94 | C₂₄H₃₄ClN₂O₃ [434,0] | (-)-2-Piperidin-N-elhylgalanthaminium-chlorid | 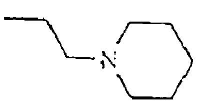 |
| 95 | C₂₅H₃₆ClN₂O₃ [448,03] | (-)-3-Piperidin-N-propylgalanthaminium-chlorid | 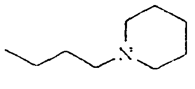 |
| | C₂₀H₂₅BrNO₃ [407,33] | (-)-Allylgalanthaminium-bromid | 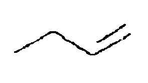 |

### Allgemeine Arbeitsvorschrift:

800 mg (2,78 mmol) (-)-Galanthamin und 3,84 g (27,8 mmol) Kaliumcarbonat wurden in 100 ml Aceton vorgelegt. Nach der Zugabe von 1,5 Äquivalenten Halogenid und einer Spatelspitze Kaliumiodid wurde das Reaktionsgemisch 24-36 Stunden unter Rückfluss gerührt. Dann wurde Kaliumcarbonat abgesaugt und das Filtrat eingedampft. Der ölige Rückstand wurde schließlich durch Säulenchromatographie in Gemisch von Trichlormethan und ammoniakalischem Methanol (9:1) gereinigt.

DC: CHCl₃:MeOH (10% NH₃)= 9 : 1

| | Produkt | Ausbeute [% d. Th.] | *α_{D} (25°C, c=1) | Schmelzpunkt [°C] |
|---|---|---|---|---|
| | | 71 | -83,7° | 130-132 |
| | 91 | 72 | -46,6° | 148-150 |
| | 92 | 43 | -62,5° | 120-125 |
| | 93 | 94 | -52,3° | 225-229 |
| | 94 | 48 | -70,8° | 136-140 |
| | 95 | 70 | -71,5° | 126-131 |
| | | 41 | -74,3° | 188-192 |
| | | | | |

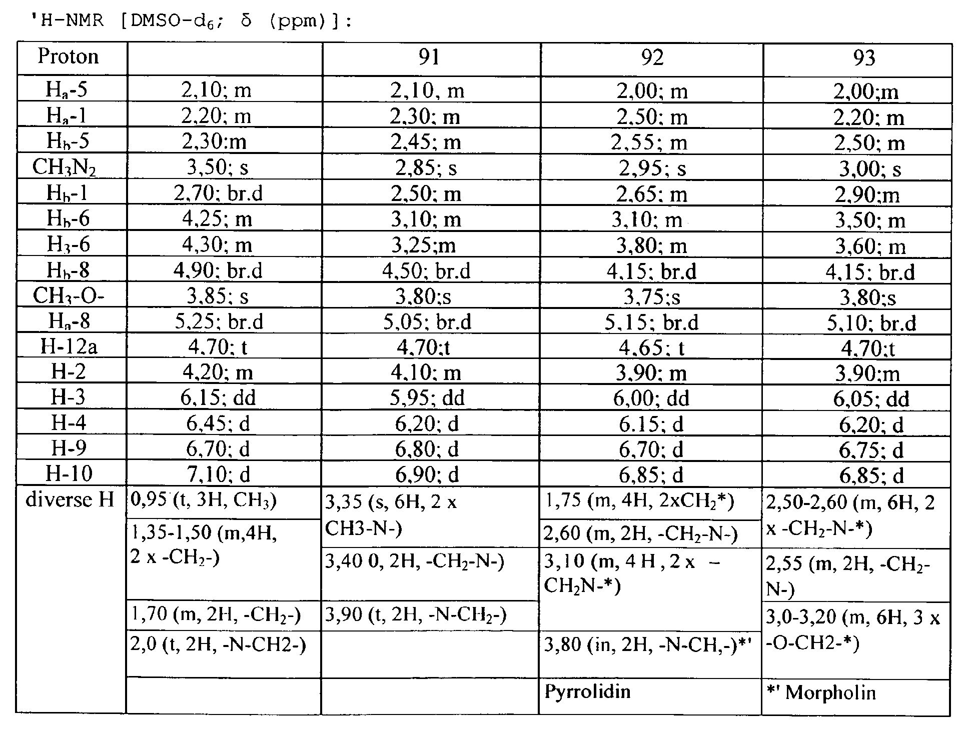

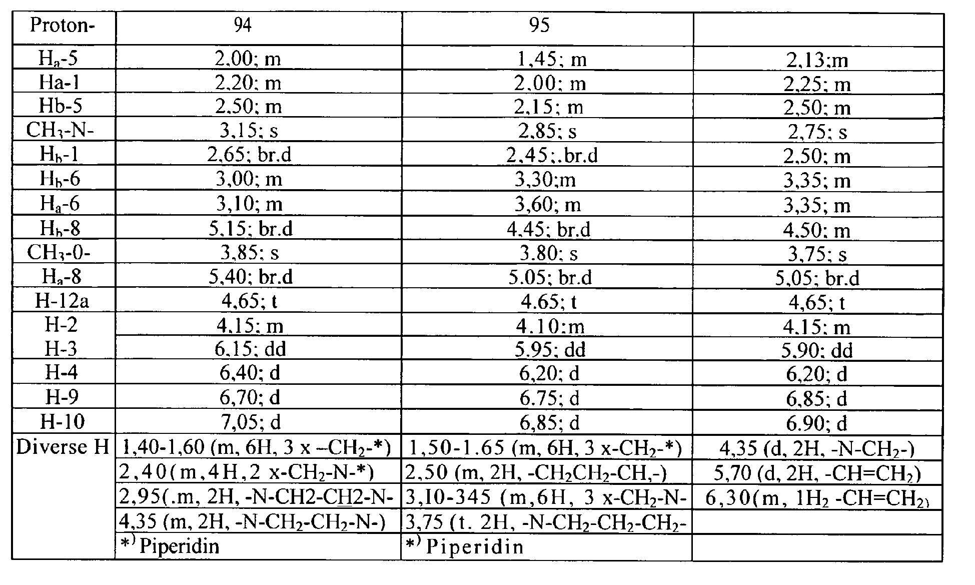

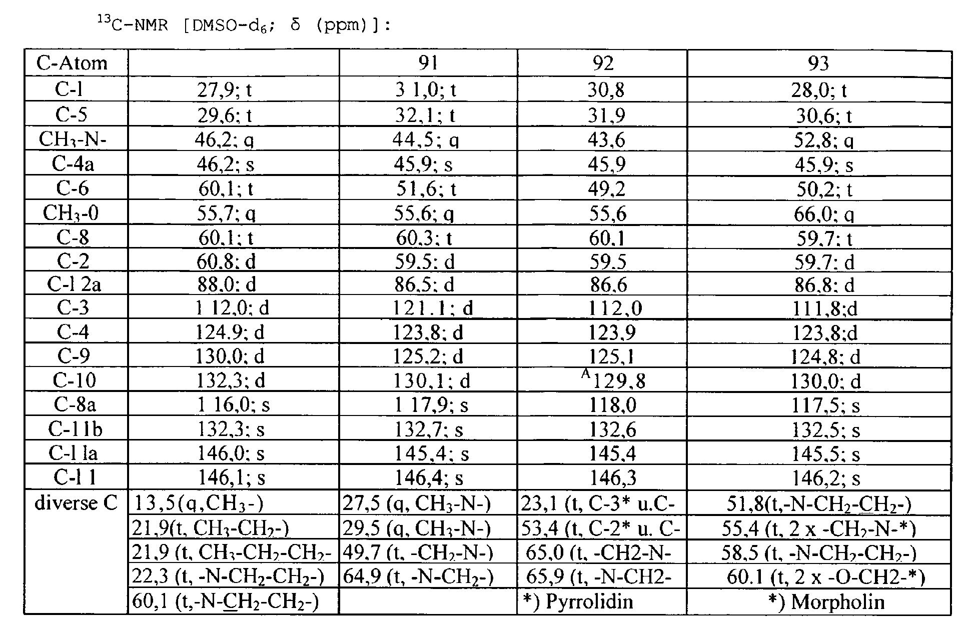

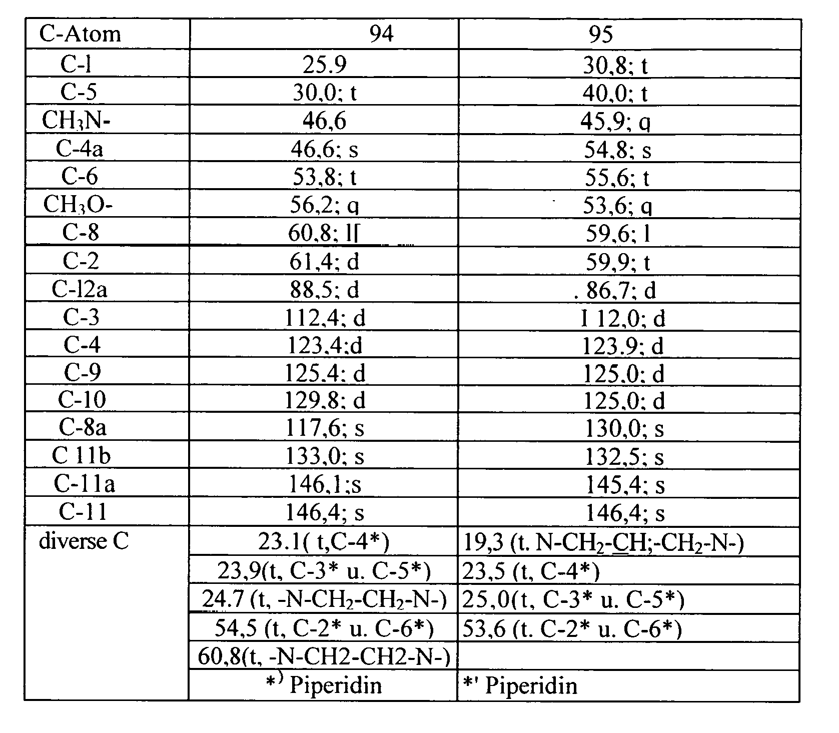

**(+)-Alkvlgalanthamininium-haIogenid**

| Produkt | Summenformel | Bezeichnung | R |
|---|---|---|---|
| 96 | C₂₃H₃₂ClN₂O₄ [435,97J | (-)-2-Morpholin-N-ethylgalanthaminium-chlorid | 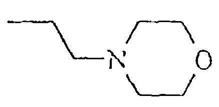 |
| 97 | C₂₅H₃₆ClN₂O₃ [448,03] | (-)-3-Piperidin-N-propylgalanthaminium-chlorid | 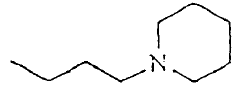 |

### Allgemeine Arbeitsvorschrift:

800 mg (2,78 mmol) (-)-Galanthamin und 3,84 g (27,8 mmol) Kaliumcarbonat wurden in 100 ml Aceton vorgelegt. Nach der Zugabe von 1,5 Äquivalenten Halogenid und einer Spatelspitze Kaliumjodid wurde das Reaktionsgemisch 24-36 Stunden unter Rückfluss gerührt. Dann wurde Kaliumcarbonat abgesaugt und das Filtrat eingedampft. Der ölige Rückstand wurde schließlich durch Säulenchromatographie in Gemisch von Trichlormethan und ammoniakalischem Methanol (9:1) gereinigt.

DC: CHCl₃:MeOH (10% NH₃)= 9 : 1

| Produkt | Ausbeute [% d. Th.] | α_{D}(25°C, c =1) | Schmelzpunkt [°C] |
|---|---|---|---|
| 96 | 44 | +48,6° | 185-190 |
| 97 | 65 | +64,0° | 118-124 |

### N-Propargyl-galanthaminimumbromid (99):

IR (KBr): 3489 s br; 3218 s; 3014 w; 2915 s br; 2133 w; 1619 s; 1507 m; 1440 s; 1274 s; 1203 m; 1070 s; 1012 m; 951 m; 865 w; 791 s cm⁻¹

¹H-NMR (D₂0) δ 6,95 (m, 2H); 6,12 (m, 2H); 5,08 (d, 1H) ; 4,70 (m, 2H); 4,46 (m, 2H); 4,29 (m, 2H); 4,11 (m, 1H); 3,80 (s, 3H) ; 3,69 (m, 1H); 3,00 (s, 3H) ; 2,41 (m; 2H), 2,20 (m, 2H).

¹³C-NMR(D₂0)δ: 148,1 (s), 147,9 (s); 134,5 (s); 130,2 (d); 127,7 (d); 127,0 (d); 119,4 (q); 114,7 (d); 89,6 (d); 85,0 (d); 72,6 (s); 67,5 (t); 63,3 (t); 62,4 (d); 61,0 (t); 58,1 (q); 48,1 (s); 46,3 (q); 33,5 (t); 31,3 (t).

### N-Acetamido-galanthaminiumbromid (100):

¹H-NMR (D₂0) δ: 6,95 (m,.2H); 6,13 (m, 2H); 5,18 (d, 1H) ; 4,70-4,28 (m, 7H), 3,83 (s, 3H); 3,08 (s, 3H); 2,50 (d, 1H); 2,39 (d, 1H); 2,18 (m, 2H).

¹³C-NMR(D₂0) δ: 168,7 (s); 148,2 (s); 148,0 (s); 134,7 (s); 130,2 (d); 128,2 (d); 127,2 (d); 119,5 (s); 114,8 (d); 89,7 (d); 68,3 (t); 64,0 (t); 62,5 (d); 59,6 (t); 58,2 (q); 48,2 (q); 33,5 (t); 31,3 (t); 18,9 (q).

### (-)-Galanthamin-N-oxid (98):

1.5 g (4.08 mmol) (-)-Galanthaminhydrobromid werden in 50 ml Wasser gelöst, mit konzentriertem wässrigem Ammoniak ausgefällt und mit dreimal 25 ml Trichlormethan extrahiert. Die organische Phase wird auf 30 bis 50 ml eingeengt und mit 1.4 g (4.08 mmol) 50 %iger Metachlorperbenzoesäure versetzt. Nach 30 min. wird das Reaktionsgemisch eingedampft und auf eine Nutschensäule aufgegeben. Mit Trichlormethan wird nun der Großteil der Metachlorperbenzoesäure abgetrennt, dann wird mit Trichlormethan : Methanol = 1:1 das N-Oxid herausgewaschen. Die weitere Reinigung des N-Oxides erfolgt über MPLC (60 g SiO₂, LM: CHCl₃ : MeOH = 2:1), wodurch quantitativ farblose Kristalle mit einem Schmp. von 80 - 85°C und einem Drehwert von a_{D}²⁶[MeOH] = -102.9° an 98 erhalten werden.

DC: CHCI3 : MeOH = 8:2

¹H-NMR (DMSO-d₆; δ (ppm)):
1.75 - 1.95 (m, 1H, H-9); 2.00 - 2.40 (m, 3H, H-5/579'); 2.95 (s, 3H,NCH₃); 3.30 - 3.75 (m, 2'H, H-10/10'); 3.75 (s, 3H, OCH₃); 4.10 (b, 1H, H-12); 4.35 (d, 1H, H-12'); 4.60 (b, 1H, H-6); 4.95 (breites d, 1H, H-4a); 5.90 (dd, 1H, H-8); 6.15 (b, 1H, H-7); 6.75 - 6.90 (m, 2H, H-1/2)

¹³C-NMR (DMSO-d₆; δ (ppm)) :
31.2 (t, C-5); 34.3 (t, C-9); 45.6 (s, C-8a); 52.5 (q, NCH₃); 55.5 (q,OCH₃); 59.5 (d, C-6); 69.0 (t, C-10); 73.9 (t, C-12); 86.6 (d, C-4a); 112.0 (d, C-8); 120.0 (s, C-12a); 122.9 (d, C-7); 125.1 (d, C-2); 130.3 (d, C-1), 132.0 (s, C-12b); 144.9 (s, C-3a); 146.5 (s, C-3)

### (6R)-4a, 5, 9, 10, 11 , 12-Hexahydro-1-brom-3-methoxy-11-metliyl-12-oxo-6H-benzofuro[3a, 3,2-ef] [2]benzazepin-6-ol (102):

Eine Suspension von 450 mg (1.19 mmol) 4a,5,9,10,1 1,12-Hexahydro-1-brom-3-methoxy-1 1-methyl-12-oxo-6H-benzofuro [3a, 3,2-ef] [2] benzazepin-6-on (101) in 10 ml absolutem Tetrahydrofuran wird bei 0°C mit 3.6 ml (3.6 mmol) 1 N L-Selectrid Lösung in Tetrahydrofuran versetzt. Nach 30 Min. wird mit 5 ml Wasser: Tetrahydrofuran 1:1 hydrolysiert. Anschließend wird das Reaktionsgemisch eingedampft, der Rückstand in 80 ml 2 N Salzsäure aufgenommen und 1 Std. bei Raumtemp. gerührt. Anschließend wird dreimal mit je 40 ml Essigsäureethylester extrahiert. Die vereinigten organischen Phasen werden einmal mit gesättigter wässriger Natriumchloridlösung gewaschen, getrocknet (Na₂SO₄), filtriert und eingedampft, wodurch quantitativ Rohprodukt erhalten wird, das durch Säulenchromatographie (15 g Kieselgel, Laufmittel: CHCl₃: MeOH = 9:1) gereinigt wird, wodurch quantitativ farblose Kristalle vom Schmp. 188 -189°C an 102 erhalten werden. DC: CHCl₃: MeOH = 9:1

¹H-NMR(CDCl₃; δ (PPM)) :
1.73 (ddd, 1H, H-9, J_{(9,9')}, = 15.1 Hz); 2.03 (ddd, 1H, H-9, J_{(9,9')} = 15.1Hz); 2.27 (ddd, 1H, H-5, J_{(5,5)}, = 14.3 Hz); 2.64 (ddd, 1H, H-5, J_{(5,5')} = 14.3 Hz); 3.18 (s, 3H, NCH₃); 3.19 (ddd, 1H, H-10, J_{(10,10)}= 14.8 Hz); 3.75 (ddd, 1H, H-10', J_{(10,10)} = 14,8Hz) ; 3.86 (s, 3H, OCH₃); 4.10 (b, 1H, H-6); 4.69 (b, 1H, H-4a); 5.48 (d, 1H, H-8, J_{(7, 8)} = 10.0 Hz); 5.88 (dd, 1H, H-7, J_{(7,8)} = 10.0 Hz) ; 7.10 (s, 1H, H-2)

¹³C-NMR (CDCl₃; δ (ppm)) :
29.8 (t, C-5); 34.1 (q, NCH₃) ; 38.2 (t, C-9); 48. 3 (s, C-8a); 48.8 (t, C-10); 56.3 (q, OCH₃); 60.9 (d, C-6); 89.9 (d, C-4a); 113.8 (s, C-1); 118.0 (d, C-8); 123.3 (s, C-12a); 126.3 (d, C-7); 130.8 (d, C-2); 132.1 (s, C-12b); 144.8 (s, C-3); 146.2 (s, C-3a); 165.1 (s, C-12)

¹³_{C-NM}R (CDCl₃; δ (ppm)) :
29.8 (t, C-5); 34.1 (q, NCH₃); 38.2 (t, C-9); 48.3 (s, C-8a); 48.8 (t, C-10); 56.3 (q, OCH₃); 60.9 (d, C-4a); 113.8 (s, C-1); 118.0 (d, C-8); 123.3 (s, C-12a); 126.3 (d, C-7); 130.8 (d, C-2); 132.1 (s, C-12b); 144.8 (s, C-3); 146.2 (s, C-3a); 165.1 (s, C-12)

### Herstellung der Produkte 105, 107:

Methode: Eine Mischung von 500 mg (1.42 mmol) N-Demethylbromgalanthamin (4), 391 mg (2.84 mmol) Kaliumcarbonat und 272 mg (1.70 mmol) Kaliumjodid wird in einer Reibschale gut zermahlen und verrieben. Anschließend wird die Mischung in 20 ml absolutem Aceton mit 1.2 Äquivalenten Halogenidreagens versetzt und auf Rückfluss erhitzt'. Nach vollständigem Umsatz (DC) wird das Reaktionsgemisch eingedampft, der Rückstand in 100 ml 2 N Salzsäure aufgenommen, mit Essigsäureethylester gewaschen, mit konzentriertem wässrigen Ammoniak basisch gemacht und entweder der Niederschlag abgesaugt oder dreimal mit je 30 ml Essigsäureethylester extrahiert. Der Niederschlag wird bei 50°C/50 mbar getrocknet, die vereinigten organischen Phasen werden einmal mit gesättigter wässriger Natriumchloridlösung gewaschen, getrocknet (Na₂SO₄, Aktivkohle), filtriert und eingedampft. Die weitere Reinigung erfolgt über Säulenchromatographie (15 g Kieselgel; Laufmittel: CHCl₃ → CHCl₃ : MeOH =9:1). DC:CHCl₃:MeOH = 9:1

### 105:

Edukt: (4) und (136). Ausbeute: 62.3 % d.Th. farbloser Schaum.

¹H-NMR (CDCl₃; δ (ppm)) :
2.36-1.36 (m, 12 H); 2.62 (ddd, 1H); 2.89 - 3.35 (m, 7H); 3.60 (2H, m), 3.80 (s, 3H); 3.85 (d, 1H); 4.10 (dd, 1H); 4.29 (H, b), 4.48 (d, 1H); 4.56 (b, 1H), 5.90 - 6.05 (m, 2H); 6.85-6.69 (4H, m), 7.23 (2H, m)

### 107:

Edukt: (4) und (137). Ausbeute: 44.9% d.Th. farbloser Schaum.

¹H-NMR (CDCl₃; δ (ppm)) :
1.65-1.85 (4H, m), 2.20-1.90 (m, 6H); 2.60-2.28 (2H, m) 2.62 (ddd, 1H); 2.89 - 3.35 (m, 5H); 3.60 (2H, m), 3.80 (s, 3H); 3.85 (d, 1H); 4.10 (dd, 1H); 4.20 (H, b), 4.48 (d, 1H); 4.56 (b, 1H), 5.90 - 6.05 (m, 2H); 6.65-6.30 (4H, m), 7.05-6.83 (2H, m)

### Arbeitsvorschrift für Produkt 109:

1.25 g (1 39) werden in 10 ml Thionylchlorid 30 min. auf Rückflusstemperatur erwärmt, überschüssiges Thionylchlorid abdestilliert, der Rückstand in 40 ml wasserfreiem THF aufgenommen und zu einer Lösung aus 2.0 g (4) in 20 ml THF zugetropft und 1 Stunde bei Rückflusstemperatur gerührt. Die Reaktionslösung wurde einedampft und das Rohprodukt mittels Säulenchromatographie (CHCl₃/MeOH 2-5%) gereinigt: 1.75 g (57% d.Th.) farbloser Schaum (109).

¹H-NMR (CDCl₃; δ (ppm)):
1.65 - 1.85 (m, 4H), 1.98 (ddd, 1H); 2.25 (b, 2H); 2.67-2.58 (m, 3H); 2.75-2.71 (2H, m), 2.87 (H, dd), 3.05 - 3.35 (m, 5H); 3.55 (2H, m), 3.67-3.74 (2H, d), 3.80 (s, 3H); 3.85 (d, 1H); 4.10 (dd, 1H); 4.40 (d, 1H); 4.56 (b, 1H); 5.90 - 6.05 (m, 2H); 6.85 (s, 1H), 7.30 (5H, m)

### Arbeits Vorschrift für Produkt 108:

1.0 g (144) werden iinlOml Thionylchlorid 2 Stunden auf Rückflusstemperatur erwärmt, überschüssiges Thionylchlorid abdestilliert, der Rückstand in 20 ml wasserfreiem THF aufgenommen und zu einer Lösung aus 1.33 g (4) in 20 ml THF zugetropft und 1 Stunde bei Raumtemperatur gerührt. Die Reaktionslösung wurde eingedampft, mit ges. NaHCO₃ Lösung aufgenommen und mit Ether (3 x 40 ml) extrahiert. Die Etherphase wurde eingedampft und das Rohprodukt mittels Säulenchromatographie (CHCl₃/MeOH 5%) gereinigt: 1.22 g (56% d.Th.) farbloser Schaum (108).

¹H-NMRCCDCl,; δ (ppm)) :
1.63 - 1.80 (m, 4H), 1.98 (ddd, 1H); 2.20 (b, 2H); 2.61-2.48 (m,3H); 2.69-2.74 (2H, m), 2.90 (H, dd), 3.02 - 3.45 (m, 3H); 3.59 (2H, m), 3.60-3.72 (2H, d), 3.87 (s, 3H); 3.95 (d, 1H); 4.22 (dd, 1H); 4.45 (d, 1H); 4.76 (b, 1H); 5.68 - 6.00 (m, 2H); 6.95 (s, 1H), 7.10-7.42 (5H,m) "Maritidinon-Typ" 4,4a-Dihydro-7-brom-9-methoxy-3-oxo (3H,6H) (5,10b) ethanophenanthridin-10-ol (113):
Eine Lösung von 4.70 g (13.4 mmol) N-Demethylbromnarwedin (15) und 2.35 g Calziumchlorid in 200 ml 70 %igem Ethanol wird für 3.5 Stdn. auf Rückfluss erhitzt. Anschließend wird das Reaktionsgemisch einrotiert, der Rückstand in 80 ml 1 N Salzsäure aufgenommen und mit konzentriertem wässrigem Ammoniak das Produkt ausgefällt. Nach Kühlen (+4°C) über Nacht wird der Niederschlag abgesaugt und bei 50°C / 50 mbar getrocknet. Die wässrige Phase wird dreimal mit Essigsäureethylester extrahiert, die vereinigten organischen Phasen werden einmal mit gesättigter wässriger Natriumchloridlösung gewaschen, getrocknet (Na₂SO₄), filtriert und eingedampft, wodurch insgesamt 4.37 g (93 % d. Th.) farblose Kristalle vom Schmp. 185-190° C an 113 erhalten werden.

DC: EtOAc:MeOH = 8:2

¹H-NMR (DMSO-d₆; δ (ppm)):
1.95 (ddd, 1H, H-11); 2.15 (ddd, 1H, H-11'); 2.30 (dd, 1H, H-4, J_{(4,4')} =16.0 Hz); 2.65 (dd, 1H, H-4', J_{(4,4')}= 16.0 Hz); 2.80 (ddd, 1H, H-12, J(_{12,12'}) =15.1 Hz); 3.05 (ddd, 1H, H-12', J_{(12,12')}.= 15.1 Hz); 3.30 (dd, 1H, H-4a); 3.55 (d, 1H, H-6, J_{(6,6')} = 16.9 Hz) ; 3.75 (s, 3H, 0-CH₃) ; 3.90 (d, 1H, H-6', J_{(w')} = 16.9 Hz); 5.80 (d, 1H, H-2, J_{(1,2)} = 9.3 Hz) ; 7.00 (s, 1H, H-8) ; 7.90 (d, 1H, H-1, J_{(1,2)} = 9.3 Hz)

¹³C-NMR(DMSO-d₆; δ (ppm)):
38.0 (t, C-11); 39.8 (t, C-4); 42.8 (s, C-10b); 53.1 (t, C-12); 55.9 (t, C-6); 56.0 (q, OCH₃); 64.1 (d, C-4a); 109.6 (s, C-7); 113.6 (d, C-2); 123.2 (s, C-6a); 126.6 (d, C-8); 129.1 (s, C-10a); 142.9 (s, C-10); 147.5 (s, C-9); 155.3 (d,C-1); 197.4 (s, C-3)

### 3S-4, 4a-Dihydro-7-brom-9-methoxy-10-hydroxy- (3H, 6H) (5,10b) ethanophenanthridin-3-ol (114):

Zu einer Suspension von 1.0 g (2.86 mmol) Maritidinon-Typ (113) in 5 ml absolutem Tetrahydrofuran werden bei 0°C 10 ml einer 1 N L-Selectrid-Lösung in Tetrahydrofuran zugetropft, anschließend wird rasch auf Rückfluss erhitzt. Nach 1.5 Stdn. wird bei 0°C mit 10 ml Tetrahydrofuran : Wasser 1:1 hydrolysiert und das Tetrahydrofuran abrotiert. Der Rückstand wird in 80 ml 1 N Salzsäure aufgenommen, mit konzentriertem wässrigem Ammoniak basisch gemacht und mit Essigsäureethylester extrahiert. Die vereinigten organischen Phasen werden mit gesättigter wässriger Natriumchloridlösung gewaschen, getrocknet (Na₂SO₄), filtriert und eingedampft, wodurch quantitativ gelbe Kristalle vom Schmp. 165 - 167°C an 114 erhalten werden.

DC: CHCl₃ : MeOH =9:1

### 114 und 3R-2,3,4,4a-Tetrahydro-7-brom-9-methoxy-10-hydroxy (1H, 6H) (5,10b) ethanophenanthridin-3-ol (116) :

Zu einer Suspension von 100 mg (0.29 mmol) Maritidinon-Typ (113) in 1 ml absolutem Tetrahydrofuran wird bei 0°C 1 ml einer 1 N L-Selectride-Lösung in Tetrahydrofuran zugetropft und bei 0^{c}C gerührt. Nach 1 Std. wird noch 1 ml einer 1 N L-Selectride-Lösung in Tetrahydrofuran zugetropft, 2.5 Stdn. bei 0°C und 3.5 Stdn. bei Raumtemp. gerührt. Anschließend wird mit 2 ml einer 1:1 Mischung aus Tetrahydrofuran und Wasser hydrolysiert, in 50 ml 2 N Salzsäure aufgenommen, nach kurzem Rühren mit konzentriertem wässrigen Ammoniak basisch gemacht und mit Essigsäureethylester extrahiert. Die vereinigten organischen Phasen werden mit gesättigter wässriger Natriumchloridlösung gewaschen, getrocknet (Na₂SO₄), filtriert und eingedampft. Die zwei Produkte werden durch Säulenchromatographie (7 g Kieselgel, Laufrnittel: CHCl₃ : MeOH = 8:2) getrennt, wodurch 30 mg (30 % d. Th.) farblose Krsitalle an 114 und 20 mg (20 % d. Th.) farbloser Kristalle an 116 erhalten werden.

DC: CHCl₃: MeOH = 9:1

### 114:

¹H-NMR [CDCl, ; δ (ppm)) :
1.50 (ddd, 1H, H-4); 1.80 (ddd, 1H, H-11); 2.20 (ddd, 1H, H-11'); 2.45 (ddd, 1H, H-4'), 2.60 - 2.80 (m, 2H, H-4a/12); 3. 30 (ddd, 1H, H-12'); 3.60 (d, 1H, H-6, J_{(6,6')} = 17.8 Hz) ; 3.75 (s, 3H, OCH₃) ; 4.00 (d, 1H, H-6', J_{(6,6')} = 17,8Hz); 4.30 (dd, 1H, H-3); 5.55 (dd, 1H, H-2, J_{(2,3)} = 9.8 Hz); 6.75 (dd, 1H, H-3, J_{(2,3)} = 9.8 Hz); 6.80 (s, 1H, H-8)

¹³C-NMR (CDCl₃; δ (ppm)) :
26.9 (t, C-11); 35.7 (t, C-4); 37.7 (s, C-10b); 47.7 (t, C-12); 50.7 (t, C-6); 51.0 (q, OCH₃) ; 58.8 (d, C-4a); 62.8 (d, C-3); 105.3 (s, C-7); 107.4 (d,C-2); 118.3 (s, C-6a); 124.8 (d, C-8); 125.5 (s, C-10a); 127.4 (d,C-1); 137.9 (s, C-10); 141.3 (s, C-9)

### 116:

¹H-NMR [CDCl3; δ (ppm)):
1.55 - 1.95 (m, 4H, H-1/1 /4/11); 2.15 (m, 1H, H-1 1'); 2.35 (m, 1H, H-2); 2.60 (dd, 1H, H-4'); 2.75 - 2.95 (m, 2H, H-4a/12); 3.15 (dd, 1H, H-2'); 3.40 (ddd, 1H, H-12'); 3.70 (d, 1H, H-6, J_{(6,6')} = 6.2 Hz); 3. 85 (d, 3H, OCH₃) ; 4.00 (d, 1H, H-6' , *J_{(6,6')}* = 6.2 Hz) ; 4.15 (ddd, 1H, H-3); 6.90 (s, 1H, H-8)

### 3S-4,4a-Dihydro-9-methoxy-10-hydroxy-(3H, 6H) (5,10b) ethynophenanthridin-3-ol (115):

Eine Lösung von 1.0 g (2.84 mmol) Marititin-Typ (114) und 2.0 g Calziumchlorid in 50 ml 50 %igem Ethanol wird mit 4.0 g frisch aktiviertem Zinkpulver versetzt und für 2 Stdn. auf Rückfluss erhitzt. Anschließend wird das überschüssige Zink abfiltriert, mit Methanol nachgewaschen und die Restlösung einrotiert. Der Rückstand wird in 80 ml 1 N Salzsäure aufgenommen, mit konzentriertem wässrigem Ammoniak basisch gemacht und mit dreimal 50 ml Essigsäureethylester extrahiert. Die vereinigten organischen Phasen werden einmal mit gesättigter wässriger Natriumchloridlösung gewaschen, getrocknet (Na₂SO₄, Aktivkohle), filtriert und eingedampft, wodurch 450 mg Rohprodukt erhalten werden, das durch Säulenchromatographie (7 g Kieselgel, Laufmittel zunächst CHCl₃: MeOH = 8:2, dann CHCl₃ : MeOH : NH₄OH = 49.9 : 49.9 : 0.2) gereinigt wird, wodurch 270 mg (35 % d. Th.) rote Kristalle vom Schmp. 59 -60°C an 115 erhalten werden.

DC: CHC1₃: MeOH = 9:1

¹H-NMR (DMSO-d₆; δ (ppm)):
1.40 (ddd, 1H, H-4); 1.65 (ddd, 1H, H-1 1); 2.00 (ddd, 1H, H-1 1'); 2.20 (ddd, 1H, H-4'); 2.65 (dd, 1H, H-4a); 3.10 (ddd, 1H, H-12); 3.30 - 3.50 (m, 1H, H-12'); 3.45 (d, 1H, H-6, J(_{6,6'}) = 15.1 Hz); 3.75 (s, 3H, OCH₃) ; 4.05 (d, 1H, H-6', J_{(6,6')}= 15,1 Hz) ; 4.20 (dd, 1H, H-3) ; 5.45 (d, 1H, H-2, J_{(1,2)} = 8.9 Hz); 6.40 (d, 1H, H-1, J_{(1,2)} = 8.9 Hz); 6.65 - 6.75 (m, 2H, H-7/8); 8.40 (b, 1H tauscht D₂O, Ph-OH)

¹C-NMR (DMSO-d₆; δ (ppm)):
32.2 (t, C-1 1) ; 41.1 (t, C-4) ; 42.7 (s, C-10b) ; 52.3 (t, C-12) ; 54.6 (t, C-6); 55.8 (q, OCH₃) ; 64,1 (d, C-4a); 67.1 (d, C-3); 109.4 (d, C-7); 115.8 (d, C-2); 124.9 (s, C-6a); 129.9 (s, C-10a); 130.2 (d, C-8); 132.5 (d, C-1); 143.7 (s,C-10); 146.0 (s, C-9)

### [4aS-(4aα, 6β,8aR*)]-4a,5,9,10,11,12-Hexahydro-3-methoxy-11-methyl-1 -nitro-6H-benzofuro[3a,3,2-ef][2]benzazepin-6-ol (117):

Zu einer Lösung von 250 mg (0,87 mmol) Galanthamin 10 ml Eisessig wird bei 15-20°C eine Mischung aus 0,5 ml rauchender Salpetersäure und 2 ml Eisessig zugetropft. Nach einer Stunde Rühren bei Raumtemp. werden weitere 0,25 ml rauchende Salpetersäure in 1 ml Eisessig zugetropft und eine weitere Stunde gerührt. Anschließend wird auf 80 ml Wasser gegossen und mit 40%iger Natronlauge basisch gemacht. Die wässrige Phase wird dreimal mit je 30 ml Essigsäureethylester extrahiert. Die vereinigten organischen Phasen werden einmal mit gesättigter wässriger Natriumchloridlösung gewaschen, getrocknet (Na₂SO₄), filtriert und eingedampft, wodurch 252 mg (87% d.Th.) gelbe Kristalle vom Schmp. 48 -50°C an 117 erhalten werden.

DC:CHCl₃:MeOH = 9:1

¹H-NMR (CDCl₃; δ (ppm)) :
1,67 (ddd, 1H, H-9); 1.95-2.30 (m, 2H, H-5/9'); 2.20 (ddd, 1H, H-5'); 2.44 (s, 3H. NCH₃); 2.91 (ddd, 1H, H-10); 3.18 (ddd, 1H, H-10'); 3.87 (s, 3H, OCH₃); 4,01 (d, 1H, H-12); 4.16 (dd, 1H, H-6); 4.32 (d, 1H, H-12'); 4.68 (b, 1H, H-4a); 6.04 (dd, 1H, H-8); 6.16 (d, 1H, H-7); 7.35 (s, 1H, H-2)

¹³C-NMR (CDCl₃; δ (ppm)):
29.6 (t, C-5); 33.3 (t, C-9); 93.6 (q, NCH₃); 48.5 (s, C-8a); 53.4 (t, C-10); 54.4 (t, C-12); 56. 1 (q, OCH₃) ; 61.4 (d, C-6); 89.6 (s, C-4a); 108.9 (d, C-8); 126.5 (,); 126.9 (,); 128.3 (d, C-7); 134.8 (,); 143.0 (,); 143.4 (,); 149.8 (,)

### [9aS-(4a,a,6β,8aR*)]-4a,5,9,10,11,12-Hexahydro-1-amincH3-methoxy-11-methyl-6H-benzofuro[3a,3,2-ef] [2]benzazepm-6-ol (118):

Zu einer Lösung von 200 mg (0.60 mmol) 117 in 10 ml Methanol wird bei Raumtemp. eine Lösung von 420 mg (2.41 mmol) Natriumdithionit in 10 ml Wasser zugetropft und eine Stunde gerührt. Anschließend wird der Methanol abrotiert, der Rückstand in 50 ml Wasser aufgenommen, mit konzentriertem wässrigem Ammoniak basich gemacht und fünfmal mit je 30 ml Trichlormethan extrahiert. Die vereinigten organischen Phasen werden einmal mit gesättigter wässriger Natriumchloridlösung gewaschen, getrocknet (Na₂SO₄) filtriert und eingedampft, wodurch 148 mg (82% d.Th.) gelbe Kristalle vom Schmp. 151 - 153 °C an 118 erhalten werden.

DC: CHCl₃:MeOH = 9:1

¹H-NMR (CDCl₃; δ (PPM)):
1.59 (ddd, 1H, H-9); 1.90 - 2.10 (m, 2H, H-5/9¹) ; 2.43 (s, 3H, NCH₃); 2.62 (ddd, 1H, H-5'); 2.96 (ddd, 1H, H-10) ; 3.20 (ddd, 1H, H-10'); 3.70 (d, 1H, H-12); 3.79 (s, 3H, OCH₃); 4.10 (d, 1H, H-12'); 4.52 (b, 1H, H-4a); 5.98 (dd, 1H, H-8) ; 6.08 (d, 1H, H-7); 6.16 (s, 1H, H-2)

### Neue, substituierte, überbrückte Basen:

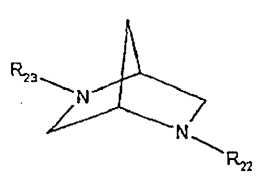

| Subst.Nr. | J-Nr. | R₂₃ | R₂₃ |
|---|---|---|---|
| 120 | | Benzyl | p-Nitro-phenyl- |
| 121 | | Benzyl | p-Amino-phenyl |
| 122 | | Benzyl | p-Chlorphenyl |
| 123 | | Benzyl | p-Hydroxyphenyl |
| 124 | | Benzyl | o-Nitrophenyl |
| 125 | | Benzyl | o-Aminophenyl |
| 126 | | Benzyl | o-Chlorphenyl |
| 127 | | Benzyl | o-Dimethylaminophenyl |
| 128 | | p-Ts | Phenyl |
| 129 | | H | Phenyl |
| 130 | | p-Ts | p-Methylphenyl |
| 131 | | H | p-Methylphenyl |
| 132 | | p-Ts | p-Chlorphenyl |
| 133 | | H | p-Chlorphenyl |
| 134 | | p-Ts | p-Fluorphenyl |
| 135 | | H | p-Fluorphenyl |
| 136 | | -CH₂-CH₂-CH₂-Cl | Phenyl |
| 137 | | -CH₂-CH₂-Cl | p-Fluorphenyl |
| 138 | | -CH₂-CH₂-OH | t-BOC |
| 139 | | -CH₂-CH₂-OH | Benzyl |
| 140 | | -CH₂-CN | Benzyl |
| 141 | | -CH₂-CH₂-NH₂ | Benzyl |
| 142 | | -CH₂-CH₂-CN | Benzyl |
| 143 | | -(CH₂)₃-NH₂ | Benzyl |
| 144 | | -CH₂-COOEt | Benzyl |
| 145 | | t-BOC | -CH(Ph)₂ |

### 5-Benzyl-2-(4-nitrophenyl)-2,5-diazabicyclo[2.2.1]heptan (120):

Zu einer Lösung von 5.30 g 2-Benzyl-2,5-diazabicyclo[2.2.1]heptan x 2 HBr in 20 ml wasserfreiem DMSO wurden 3.97 g getrocknetes, fein verriebenes K₂CO₃ und 2.03 g 4-Fluornitrobenzol zugegeben. Nun wurde 3 Stunden lang bei 80 °C magnetisch gerührt, auf 100 ml Wasser gegossen, ausgefallene Kristalle abgesaugt, mit Diisopropylether gewaschen und im Vakuum getrocknet: 4.10 g (120) als farblose Kristalle (92% d.Th.), Schmp.: 170-173 °C. DC: Toluol/Aceton (1:1) oder CHCl₃.

¹H-NMR (CDCl₃):
8.10 (2H, d), 7.35-7.2 (5H, m), 6.45 (2H, d), 4.40 (1H, m), 3.75 (2H, s), 3.65 (1H, b), 3.45 (2H, dd), 2.95, 2.30 (2H, dd), 2.10, 1.85 (2H, dd)

¹³C-NMR (CDCl₃) :
151.14, 139.01, 136.55,128.26, 126,97, 126.35, 110.42, 60.42, 58.28, 58.191, 53.17, 35.78.

### 5-Benzyl-2-(4-aminophenyl)-2,5-diazabicyclo[2.2.1]heptan (121):

4.1 g (120) wurden in 360 ml Ethanol und 20 ml Wasser mit 5 g NH₄Cl und 7 Eisen Pulver unter mech. Rühren 4 Stunden auf Rückflusstemperatur erwärmt. Die Reaktionslösung wird über Celite und Aktivkohle filtriert, eingedampft, mit 100 ml Wasser aufgenommen, mittels K₂CO₃ auf pH 10 gebracht und mit Ether (4 x 50 ml) extrahiert. Die vereinigten organischen Phasen wurden über Na₂SO₄ getrocknet, eingedampft und kugelrohrdestilliert (Kp:5 mBar: 160-170°C): 3.0 g (81% d.Th)(121) als farbloses Öl. DC: CHCl₃/Methanol (9:1).

¹H-NMR (CDCL₃) :
7.35-7.15 (5H, m), 6.65 (2H, d), 6.45 (2H, d), 4.15 (1H, m), 3.70 (2H, s), 3.50 (H, m) 3.40, 3.30 (2H, dd), 3.20 (2H, b), 2.90,2.70 (2H, dd), 2.05-1.85 (2H, dd)

### 5-Benzyl-2-(4-chlorphenyl)-2,5diazabicyclo[2.2.1]heptan (122):

1.5 g (121) wurden in 20 ml conc. HCl gelöst und bei 0-5 °C eine Lösung von 0.38 g NaN0₂ in 3 ml Wasser zugetropft, sodass die Temp. unter 5°C blieb. Nun wurde die Lösung auf eine aus 1.61 g CuSO₄ x 5 H₂O, 41 g NaCl, 0.39 g NaHSO₃ und 0.23 g NaOH hergestellten Lösung von CuCl in 10 ml HCl conz. getropft und 4 Stunden auf 50°C erwärmt. Nun wurde auf 100 ml Wasser gegossen, mit K₂CO₃ alkalisch gemacht und mit Ether (5 x 100 ml) extrahiert. Eindampfen und Kugelrohrdestillation (Kp 5 mbar, 135°C) ergaben: 0.6 g (37% d.Th:) (122) als farbloses Öl. DC: CHCl₃/Methanol (9:1).

¹H-NMR (CDCI₃) :
7.40 - 7.15 (7H, m), 6.90 - 6.50 (2H, m), 4.25 (1H, m), 3.70 (2H, s), 3.60-3.45 (H, m), 3.45-3.30 (2H, m), 2.95, 2.70 (2H, dd), 2.10-1.80 (2H, m).

### 5-Benzyl-2-(4-hydroxyphenyl)-2,5-diazabicyclo[2.2.1]heptan (123):

Zu einer Lösung von 1.17 g (122) in 17 ml conz. HCL: wurden 0.35 g NaNO₂ in 5 ml Wasser langsam zugetropft, sodass die Temperatur unter 5°C blieb. Nun wurde 2 Stunden bei 60°C gerührt, die Lösung mit NaHCO₃ neutralisiert und mit Ether (4 x 50 ml) extrahiert. Die vereinigten organischen Phasen wurden über Na;SO₄ getrocknet und eingedampft und kugelrohrdestilliert (Kp: 0.05 mbar, 140°C): 0.1 g (123) als farbloses Öl (7.5% d.Th.). DC: CHCl₃/Methanol (9:1).

¹H-NMR (CDCl₃) : 7.50-7.00 (8 H, m), 685-6. 40 (2H, m), 4,25 (1H, m), 3.80-3.30 (5H, m), 3.05-2.65 (2H, m), 2.05, 1.90 (2H,dd).

### 5-Benzyl-2-(2-nitrophenyl)-2,5-diazabicyclo[2.2.1]heptan (124):

Zu einer Lösung von 22,3 g 2-Benzyl-2,5-diazabicyclo[2.2.1]heptan x 2 HBr in 110 ml wasserfreiem DMSO wurden 17.6 getrocknetes, fein verriebenes K₂CO₃ und 9.0 g 2-Fluornitrobenzol zugegeben. Nun wurde 3 Stunden lang bei 80°C magnetisch gerührt, auf 300 ml Wasser gegossen, ausgefallene Kristalle abgesaugt, mit Diisopropylether gewaschen und im Vakuum getrocknet: 19.1 g (124) als farblose Kristalle (96.9% d.Th.), Schmp.: 107-108°C. DC: Toluol/Aceton (1:1) oder CHCl₃.

¹H-NMR (CDCl₃) :
7,75 (h, d) ,7.35 (h, d), 7.30-7.15 (5H, m), 6.85-6.70 (2H, m), 4.30 (H, m), 3.65 (2H, s), 3.55 (2H, m), 2.90 (2H, dd), 2.85 (H, m), 2.00 (2H, dd).

### 5-Benzyl-2-(2-aminophenyl)-2,5-diazabicyclo[2.2.1]heptan (125):

5.0 g (124) wurden in 360 ml Ethanol und 20 ml Wasser mit 4 g NH₄Cl und 6.7 Eisen Pulver unter mech. Rühren 4 Stunden auf Rückflusstemperatur erwärmt. Die Reaktionslösung wird über Celite und Aktivkohle filtriert, eingedampft, mit 100 ml Wasser aufgenommen, mittels K₂CO₃ auf pH 10 gebracht und mit Ether (4 x 50 ml) extrahiert. Die vereinigten organischen Phasen wurden über Na₂SO₄ getrocknet, eingedampft und kugelrohrdestilliert (Kp: 5 mBar: 160-170°C): 2.20 g (48,8% d.Th) (125) als farbloses Öl. DC: CHCl₃/Methanol (9:1).

¹H-NMR (CDCl₃) :
7.45-7.20 (5H, m), 7.05-6.65 (4H, m), 3.95-3.65 (5H, m), 3.60-3.40 (2H, m), 3.20-3.00 (H. m), 2.95-2.75 (2H, m), 2.00-1.85 (2H, m).

### 5-Benzyl-2-(2-chlorphenyl)-2,5-diazabicyclo[2.2.1]heptan(126):

### Arbeitsvorschrift analog (122):

Ausbeute nach Kugelrohrdestillation (Kp: 5mbar, 135°C): 0.60 g (37.5% d.Th.) (126) als farbloses Öl. DC: CHCl₃/Methanol(9:1).

¹H-NMR (CDCl₃) :
7.50-7.20 (6H, m), 6.85-6.55 (3H, m), 4.25 (H, m), 3.85-3.70 (2H, s), 3.65-3.50 (H, b), 3.45-3.30 (2H, m), 3.00, 2.75 (2H, dd), 2.15-1.80 (2H, m)

### 5-Benzyl-2-(2-dimethylaminophenyl-2,5-diazabicyclo[2.2.1]heptan (127):

### 5-Benzyl-2-(2-methylaminophenyl)-2 ,5-diazabicyclo[2.2.1]heptan (127-a):

0.95 g (125) wurden mit 0.5 g PO(OMe)₃ für 2 Stunden auf 160-180 °C erwärmt, abgekühlt, mit 5 ml 30% NaOH hydrolisiert, 10 ml Wasser zugegeben und mit Ether (3x10 ml) extrahiert. Eindampfen und Säulenchromatographie (CHCl₃/Methanol 3%) ergaben 0.15 g farbloses Öl (127-a) (15.6% d.Th.) und 0.09 g farbloses Öl (107) (8.5% d.Th.).

¹H-NMR (CDCl₃) (127-b) :
7.45-7.20 (5H, m), 7.10-6.95 (2H, t), 6.80-6.00 (2H, dd), 3.90-3.65 (4H, m), 3.65-3.40 (2H, dd), 3.50-2.60 (6H, m), 2.0-1.80 (2H, m)

¹H-NMR (CDCI₃) (127):
7.40-7.20 (5H, m), 6.70-6.55 (3H, m), 6.40 (H, m), 3.75 (2H, s), 3.80-3.65 (H, m), 3.60-3.55 (2H, dd), 3.45-3.20 (3H, m), 2.90-2.75 (6H, s,s), 2.30-2.15 (2H, dd).

**Herstellung phenylsubstituierter 2,5-Diazabicyclo[2.2.1]heptane:**

| Nr. | Ausbeute | Schmp/Kp | DC | Methode |
|---|---|---|---|---|
| 128 | 62.5 % | 139-143°C | Petrolether/EtOAc (7:3) | A |
| 129 | 71% | 0.05 mbar/120-130° | DC:CHCl₃/Methanol(9:1) | B |
| 130 | 46 % | 149-15TC | DC: Petrolether/EtOAc (7:3) | A |
| 131 | 65% | 0.05 mbar/130-140°C | DC:CHCl₃/Methanol(9:1) | B |
| 132 | 69% | 214-217°C | DC: Petrolether/EtOAc (7:3) | A |
| 133 | 56% | 0.05 mbar/120-130°C | DC:CHCl₃/Methanol(9:1) | B |
| 134 | 55% | Schmp: 180-184°C | DC: Petrolether/EtOAc (7:3) | A |
| 135 | 74% | 0.05mbar/120-130°C | DC:CHCl₃/Methanol(9:1) | B |

### Methode (A) zur Cyclisierung des Tritosyl-4-hydroxiprolinol:

20 g (35 mmol) Tritosyl-4-hydroxiprolinol werden mit 75 ml Toluol, 9.8 g (100 mmol) Triethylamin, und 35 mmol des entsprechend substituierten Anilins (frisch destilliert oder umkristallisiert) im Stahlautoklaven 3 Stunden lang auf 160-170° C erwärmt. Nach Abkühlen und Öffnen des Autoklaven wird das Produkt mit 100 ml Toluol aus dem Autoklaven ausgespült, einmal mit 100 ml ges. NaCl Lösung und einmal mit 100 ml ges. NaHCO₃ Lösung geschüttelt und die organische Phase über Na₂SO₄ gertrocknet und eingedampft. Das kristalline Produkt wird mit Isopropanol digeriert, filtriert und getrocknet.

### Methode (B) zur Abspaltung der p-Ts Schutzgruppe:

2.5 g Edukt werden in 40 ml Eisessig und 20 ml conz. Schwefelsäure 2 Stunden lang bei 80 °C gerührt. Anschließend wird auf 200 ml Eis/Wasser gegossen, mit EtOAc (2 mal 100 ml) extrahiert (EtOAc Phase wird verworfen), die wässrige Phase mit 30% NaOH bis pH 12 versetzt und mit EtOAc (6 x 50 ml) extrahiert. Diese Ethylacetatphase wird eingedampft und kugelrohrdestilliert: farbloses Öl.

NMR-Spektren:
5-Phenyl-2-p-tosyl-2,5-diazabicyclo[2.2.1]heptan (128):

¹H-NMR (CDCl₃) :
7.68 (2H, d), 7.29 (2H, d), 7.18 (2H, m), 6.72 (H, t), 6.4 (2H, dd), 4.51 (H, b), 4.32 (H, b), 3.52 (2H, dd), 3.24 (2H, dd), 2.42 (3H, s), 1.86 (H, d), 1.40 (H, d).

¹³C-NMR(CDCl₃) :
146.18, 143.49, 135.27, 129.66, 129.10,127.18, 116.84, 112.39, 59.98, 56.91, 56.52, 52.25.36.50.21.37.

### 2-Phenyl-2,5-diazabicyclo[2.2.1]heptan(129):

¹H-NMR (CDCl₃) :
7.23 (2H, m), 6.71 (3H, m), 4.30 (H, b), 3.78 (H, b), 3.66 (H, dd), 3.18-2.89 (3H, m), 2.06-1.78 (3H, m).

¹³C-NMR(CDCl₃) :
146.92, 129.09, 116.08,112.41,59.78,56.62,56.22,49.65,37.18

### 5-(4-Methylphenyl)-2-p-tosyl-2,5-diazabicyclo[2.2.1]heptan (130):

¹H-NMR (CDCl₃) :
7.68 (2H, d), 7.27 (2H, d), 7.00 (2H, d), 6.36 (2H, d), 4.49 (H, s), 4.25 (H, s), 3.53 (H, d), 3.46 (H, dd), 3.26 (H, dd), 3.17 (H, d), 2.41 (3H, s), 2.24 (3H, s), 1.83 (H, d), 1.38 (H, d).

¹³C-NMR(CDCl₃):
144.09, 143.44, 135.35, 129.63, 127.30, 125.96, 112.55, 60.02, 57.06, 56.73, 51.99, 36 46, 21.36,20.16.

### 2-(4-Methylphenyl)-2,5-diazabicyclo[2.2.1]heptan (131):

¹H-NMR (CDCl₃) : 7.05 (2H, d), 6.48 (2H, d), 4.25 (H, s), 3.77 (H, s), 3.68 (H, dd), 3. 16 (H, dd) 3.02 (H, dd), 2.92 (H, dd), 2.24 (3H, s), 1.95 (H, d), 1.82 (H, b), 1.80 (H, d).

### 5-(4-Chlorphenyl)-2-p-tosyl-2,5-diazabicyclo[2.2.1]heptan (132):

¹H-NMR (CDCI₃) :
7.52 (2H, d), 7.13 (2H, d), 6.96 (2H, d), 6.22 (2H, d), 4.38 (H, s), 4.12 (H, s), 3.40-3.29 (2H, m), 3.12 (H, dd), 3.03 (H, dd), 2.30 (3H, s), 1.73 (H, d), 1.28 (H, d).

13_{C-NMR} (CDCL₃/DMSO) :
144.70,143.30,134.58,129.44, 128.40, 126.77, 120.58, 113.34, 59.60,56.73, 56.44,51.81,36.09,21.00.

### 2-(4-Chlorphenyl)-2,5-diazabicyclo[2.2.1]heptan (133):

¹H-NMR (CDCI₃) :
7.14 (2H, d), 6.45 (2H, d), 4.23 (H, s), 3.76 (H, s), 3.62 (H, d), 3.08 (H, d), 3.00 (H, d), 2.89 (H, d), 1.92 (H, d), 1.81 (H, d), 1.56 (H, b).

¹³C-NMR (CDCI₃) :
145.53, 128.78, 120.56, 113.44, 59.77, 56.83, 56.19, 49.50, 37.26

### 5-(4-Fluorphenyl)-2-p-tosyl-2,5-diazabicyclo[2.2.1]heptan (134):

¹H-NMR (CDCI₃) :
7.68 (2H, d), 7.27 (2H, d), 6.82-6.95 (2H, m), 6.40-6.29 (2H, m), 4.49 (H, s), 4.23 (H, s), 3.52 (H, d), 3.46 (H, dd), 3.25 (H, dd), 3.13 (H, d) 2.41 (3H, s), 1.86 (H, d), 1.41 (H, d).

¹³C-NMR (CDCI₃)
157.63, 152.96, 143.56, 142.80,142.77, 135.21,129.65, 127.17, 115.73, 115.29, 113.20, 113.05, 59.97, 57.35, 56.93, 51.79, 36.60,21.34.

### 2-(4-Fluorphenyl)-2,5-diazabicyclo[2.2.1]heptan (135):

¹H-NMR (CDCI₃) :
7.05-6.83 (2H, m), 6.52-6.28 (2H, m), 4.20 (H, s), 3.76 (H, s), 3. 64 (H, dd), 3. 10 (H, d), 3.00 (H, dd), 2.88 (H, d), 1.96 (H, d), 1.81 (H, d), 1.76 (H, b).

¹³C-NMR (CDCl₃):
157.27, 152.63, 143.61, 115.67, 115.32, 113.13, 112.98, 60.21, 57.04, 56.27,49.21, 37.29.

### 5-(3-ChIorpropyl)-%2-phenyi-2,5-diazabicyclo[2.2.1]heptan (136) :

1.0 g (5.7 mmol) (129), 0.23 g (5.7 mmol) Natriumamid und 20 ml Toluol werden 1 Stunde auf Rückflusstemperatur erwärmt. Nun werden 0.93 g (5.7 mmol) 1-Brom-3-chlorpropan in 10 ml Toluol über 20 Min zugetropft und 2 Stunden bei Rückflusstemperatur gekocht, nach Abkühlen wird mit 2n HCl (2 x 50 ml) extrahiert, die wässrige Phase mit 30% NaOH alkalisch gemacht und mit Toluol (3 x 40 ml) extrahiert. Eindampfen und Kugelrohrdestillation (Kp: 0.05 mbar, 120-130 °C) ergaben 0.97 g (70.4% d.Th.) (136) als farbloses Öl

DC:CHCl₃/Methanol(9:1)

¹H-NMR (CDCl₃) :
7. 19 (2H, m), 6.69 (3H, m), 4.27 (H, b), 3.68 (H, b), 3.60 (H, dd), 3.18-2.89 (5H, m), 2.36-1.36 (7H,m).

### 5-(2-Chlorethyl)-2-(4-fluorphenyl)-2,5-diazabicyclo[2.2.1]heptan (137):

1.0 g (5.2 mmol) (135), 0.21 g (5.3 mmol) Natriumamid und 20 ml Toluol werden 1 Stunde auf Rückflusstemperatur erwärmt. Nun werden 0.77 g (5.2 mmol) 1-Brom-3-chlorethan in 10 ml Toluol über 20 Min zugetropft und 2 Stunden bei Rückflusstemperatur gekocht, nach Abkühlen wird mit 2 n HCl (2 x 50 ml) extrahiert, die wässrige Phase mit 30% NaOH alkalisch gemacht und mit Toluol (3 x 40 ml) extrahiert. Eindampfen und Kugelrohrdestillation (Kp: 0.05 mbar, 100-120°C) ergaben 0.76 g (56.7% d.Th.) (137) als farbloses Öl

DC: CHCl_{3/}Methanol (9:1)

¹H-NMR (CDCl₃):
7.05-6.83 (2H, m), 6.52-6.28 (2H, m), 4.20 (H, s), 3.76 (H, s), 3.64 (H, dd), 3.10 (H, d), 3.00 (H, dd), 2.88 (H, d), 2.66-2.28 (2H, m), 2.20-1.90 (2H, m) 1.96 (H, d), 1.81 (H, d), 1.76 (H.b).

### 2-t-Boc-5-(2-hydroxyemyl)-2,5-diazabicyclo[2.2.1]heptan (138):

In eine Lösung von 2.5 g 2-t-Boc-2,5-diazabicyclo[2.2.1]heptan in 50 ml Methanol werden unter Rühren bei 20°C 1.5 Stunden lang gasförmiges Ethylenoxyd langsam eingeleitet, wobei die Temperatur auf 35°C ansteigt. Die Lösung wurde eingedampft und das ölige Rohprodukt kugelrohrdestilliert (Kp: 0.05 mbar, 90-100°C): 1.60 g (138) als farbloses Öl (52.5% d.Th.).

¹H-NMR (CDCl₃) :
4.31 (H, d), 3.54 (2H, t), 3.40 (H, d), 3.18 (H, dd), 2.92 (H, dd), 2.73 (2H, m), 2.56 (H, d), 1.84 (H, d), 1.72 (H, d), 1.54 (9H, s)

¹³C-NMR (CDCl₃)
157.80, 79.21, 61.76, 61.24, 59.82, 59.68, 56.40, 56.09, 55.73, 55.43, 49,95, 49.21, 36.01,35.36,28.27

### 2-Benzyl-5-(2-hydroxyethyl)-2,5-diazabicyclo[2.2. 1]heptan (139):

Arbeitsvorschrift: siehe (138).
Ausbeute: 83.3% d.Th. (139) als farbloses Öl, Kp (0.005 mbar, 120-130°C)

¹H-NMR (CDCl₃) :
7.30 (5H, m), 3.67-3.74 (2H, d), 3.55 (2H, m), 3.30 (2H, b), 3.20 (H, b), 2.87 (H, dd), 2.75 (H, dd), 2.71 (H, t), 2.67 (2H, m), 1.78 (H, m), 1.68 (H, m).

13_{C-NMR} (CDCl₃) :
139.63, 128.29, 128.09, 126.65, 62.49, 61.16, 59.80, 58.19, 56.45, 56.26, 56.19, 33.64

### 2-Benzyl-5-cyanomethyl-2,5-diazabicyclo[2.2.1]heptan (140):

Zu einer Lösung von 3 g 2-Benzyl-2,5-diazabicyclo[2.2.1]heptan in 40 ml wasserfreiem Toluol wurden 3 g getrocknetes, fein verriebenes K₂CO₃ sowie 1.3 ml frisch destilliertes Chloracetonitril zugegeben und 10 Stunden unter heftigem Rühren auf Rückflusstemperatur erwärmt. Die Lösung wurde abgekühlt, filtriert und eingedampft. Kugelrohrdestillation (Kp: 0.01 mbar, 110-120°C) ergab 3.57 g (140) als farbloses Öl (97% d.Th.).

¹H-NMR (CDCl₃) :
7.41-7.18 (5H, m), 3.65, 3.75 (2H, d), 3.53, 3.46 (2H, d), 3.45 (H, b), 3.37 (H, b), 3.04 (H, d), 2.73 (H, d), 2.71 (H, dd), 2.68 (H, d), 1.82 (H, d), 1.77 (H, d).

¹³C-NMR (CDCI₃) :
139.41, 128.08, 127.92, 126.51, 117.03,62.47,61.39,57.97,57.09,55.97,41.23, 33.00.

### 2-Benzyl-5-(2-aminoethyl)-2,5-diazabicyclo[2.2.1]heptan (141):

Eine Lösung von 5.74 g (25.3 mmol) (140) und 50 ml NH₃ wurden in Methanol mit 2 g Raney-Nickel im Stahlautoklaven bei 100 Bar H₂ und 100°C 2 Stunden hydriert. Katalysator wurde abgesaugt, die Lösung eingedampft und kugelrohrdestilliert (Kp: 0.01 mbar, 135-145 °C): 5.02 g (141) als farbloses Öl (87% d.Th.).

¹H-NMR (CDCI₃) :
7.18 (5H, m), 3.70 (2H, d), 3.23 (2H, b), 2.69-2,,42 (8H, m), 1.71 (H, ddd), 1.65 (H, dddl 1.70 (2H,b).

¹³C-NMR (CDCl₃):
139.62,127.92,127.65,126.19,62.15,61.15,57.92,57.30,56.19,55.91,40.80,33.32

### 2-Benzyl-5-cyanoethyl-2,5-diazabicyclo[2.2.1]heptan (142):

Zu einer Lösung von 3 g 2-Benzyl-2,5-diazabicyclo[2.2.1]heptan in 40 ml wasserfreiem Toluol wurden 2.5 g frisch destilliertes Acrylnitril zugegeben und 24 Stunden unter heftigem Rühren auf Rückflusstemperatur erwärmt. Die Lösung wurde abgekühlt, filtriert und eingedampft. Kugelrohrdestillation (Kp: 0.01 mbar, 120-130°C) ergab 3.43 g (142) als farbloses Öl (88% d.Th.).

¹H-NMR (CDCl₃) :
7.39-7.17 (5H, m), 3.70 (2H, d), 3.30 (H, b), 3.26 (H, b), 2.88-2.59 (4H, m), 2.74 (H, d) 2.63 (H, dd), 2.42 (2H, t), 1.75 (H, dd), 1.64 (H, dd).

¹³C-NMR (CDCl₃):
139.50, 128.17, 127.99, 126.57,118.64, 62.40, 61.15, 58.68, 56.59, 55.91, 49.77, 33.66, 18.21

### 2-Benzyl-5-(2-aminopropyl)-2,5-diazabicyclo[2.2.1]heptan (143):

analog: (141)
Ausbeute: 83.7% d.Th. farbloses Öl, Kp (0.01): 120-130°C.

¹H-NMR (CDCI₃) :
7.18 (5H, m), 3.70 (2H, d), 3.31 (H, b), 3.16 (H, b), 2.91-2.48 (8H, m), 2.22 (2H, b), 1.71 (2H, m), 162 (H, d), 149 (H, d)

¹³C-NMR (CDCI3) :
139.46, 127.76, 127.54, 126.03, 61.55, 60.90, 57.76, 56.08, 55.32, 51.58, 39.99, 33.05, 31.97

### 2-(5-Benzyl-2,5-diazabicyclo[2.2.1]heptan)-essigsäureethylester (144):

Zu einer Lösung von 3 g 2-Benzyl-2.5-diazabicyclo[2.2.1]heptan in 40 ml wasserfreiem Toluol wurden 2.5 g Bromessigsäureethylester und 3 g getrocknetes, fein verriebenes K₂CO₃ zugegeben und 8 Stunden unter heftigem Rühren auf Rückflusstemperatur erwärmt. Die Lösung wurde abgekühlt, filtriert und eingedampft. Kugelrohrdestillation (Kp: 0.01 mbar, 125-130°C) ergab 1.79 g (144) als farbloses Öl (40 % d.Th.)

### ¹³C-NMR (CDCI₃):

170.96, 139.44, 128.14, 128.03,126.62, 62.31, 61.64, 60.36, 58.06, 56.90, 55.47, 55.33,33.74, 14.03

### 2-tBoc-5-diphenylmethyl-2-5-diazabicyclo][2.2.1]heptan (145):

Zu einer Lösung von 1.5 g 2-t-Boc-2,5-diazabicyclo[2.2.1]heptan in wasserfreiem THF wurden 0.8 g Triethylamin undl.55 g Diphenylmethylchlorid zugegeben und 4 Stunden bei Rückflusstemperatur gerührt. Nun wurde THF abgedampft, mit 50 ml ges. NaHCO₃ Lösung aufgenommen und 3 x mit 30 ml Ether extrahiert. Eindampfen ergab 2.2 g gelbliche Kristalle (145) (78% d.Th.).

### ¹H-NMR (CDCl₃) :

7. 48-7. 11 (10 H, m), 4 . 81 (H, b), 4 . 31 (H, d), 3. 40 (H, d), 3.18 (H, dd), 2.92 (H, dd), 2.56 (H, d), 1 . 84 (H, d), 1.72 (H, d), 1.54 (9H, s)

### Literaturliste

- 1.: S. Y.Han, J.E. Sweeney, E.S. Bachmann, E.J. Schweiger, J.T. Coyle, B.M. Davis, M.M. Joullie. Eur.J.Med.Chem. 27, 673-687 (1992)
- 2.: T. Kametam, K. Yamaki, S. Yaki, K. Fukumoto, J.Chem.Soc. (C), 2602 (1969)
- 3.: T. Kametani, K. Shishido, E. Hayashi, J. Org. Chem., 36, 1259 (1971)
- 4.: T. Kametani, C. Seino, K. Yamaki, S. Shibuya, K. Fukumoto, J. Chem. Soc, 1043-1047 (1971)
- 5.: T. Kametani, K. Yamaki, T. Terui, S. Shibuya, K. Fukumotor, J. Chem. Soc. Perkin I, 1513-1516 (1972)
- 6.: T. Kametani, K. Yamaki, T. Terui, J. Het. Chem. 10, 35-37 (1973)
- 7.: J. Szewczyk, A. Lewin, F.I. CaroIl J. Het. Chem 25,1809-1811 (1988)
- 8.: R. Vlahov, D. Krikorian, G. Spassov, M. Chinova, I. Vlahov, G. Parushev, G. Snatzke, L. Ernst, K. Kieslich, W. Abraham, W. Shedrick, Tetrahedron 45, 3329 (1989)
- 9.: P. Strehlke, G.A. Hoyer, E. Schröder, E. Arch, Pharm. 388 (2), 94-109 (1975)
- 10.: M. Ishizaki, K. Ozakr, A. Kanematsu, T. Isoda, O. Hoshino, J. Org. Chem. 58, 3877-3885 (1993)
- 11.: C. Nogueiras, W. Döpke, G. Lehmann, tetrahedron Letters 35, 3249-3250 (1971)
- 12.: H.H. Wassermann, R.J. Gambale, Tertrahedron 48 (35), 7059-7070 (1992)
- 13.: J.M. Pons, A. Pommier, J. Lerpimere, P. Kocienski, J. Chem. Soc, Perkin Trans I 14, 1549-1551 (1993)
- 14.: T. Kioshi, Yakugaku Zhassi, 104, 1009 (1984)
- 15.: B.M. Davsis, Pat WO 88/08708 A1 (1988)
- 16.: Stichting Biomed. Res., Pat NL 88000350 A1 (1989)
- 17.: J. Bastida; F. Viladomat; J.M. Llabres, S. Quiroga, C. Codina, M. Rubiralta, Planta Med. 56, 123, 1990
- 18.: S.J. Han, S.C. Mayer, E.J. Schweiger, B.M. Davis, M.M. Joullie, Boorg. Med. Chem. Lett. 1, 579, 1991
- 19.: D. Albrigt, N. Goldmann, Pat. 64219 (1968)
- 20.: H. G. Boit, W. Döpke,A.W. Beitner, Chem. Ber. 90,2197 (1957)
- 21.: R. Matusch, M. Kreh, U. Müller, Helv. Chim. Acta 77, 1611 (1994)
- 22.: H.M. Fales, L.D. Gioffiida, W.C. Wildmann, J.Am. Chem. Soc. 78, 4145 (1956)
- 23.: S. Kobayashi, K. Satoh, S. Numata; T. Hingu, M. Kihara, Phytochemistry 30, 675 (1991)
- 24.: R.W. Kosley, L. Davis, V. Taberna, Pat. EP 653427 A1 (1995), US 93-137440
- 25.: R.W. Kosley, L. Davis, V. Taberna, Pat. EP 649846 A1(1995), US 93-137444
- 26.: R.W. Kosley, L. Davis, V. Taberna, Pat. EP 648771 A1 (1995), US 93-137443
- 27.: W.S.K. Kaisha,EP 393400 (1990), JP 82321 (1989), JP 238064 (1989)
- 28.: DL. Romero e.y., Pat WO 91-09849 (1991), US 90-07390 (1990)
- 29.: J.E. Arrowsmith, Pat. EP 324543 (1989), GB 8800694 (1988)
- 30.: D.G. Hutchinson, Pat. WO 9323384 (1993), US 92-880432 (1992)
- 31.: P.S. Portoghese, A.A. Mikhail, J.Org. Chem. 31, 1059 (1966)
- 32.: T.F. Braish, D.E. Fox, J. Org. Chem., 55, 1684-1687 (1990)
- 33.: S. Ziklova, K. Ninov, Tr. Nauchniozsled. Khim. Farm. Inst. 12, 35-46 (1982)
- 34.: K. Fujii, K. Tomino, H. Watanabe, J. Pharm. Soc. Japan 74, 1049-51 (1954)
- 35.: HG. Morren, R. Danayer, R. Linz, J. Mathieu, H. Strubbe, S. Trolin, Ind. Chim. Belge 22, 409-416 (1957)
- 36.: D.C. Jones, M.A. Winter, K.S. Hirsch, N. Stamm, H.M. Taylor, J. Med. Chem. 33 (1), 416-429 (1990)
- 37.: G.L.Regnier, CG. Guillonneau, J.L. Duhault, F.P. Tisserand, G. Saint-Romas, SM. Holstorp, Eur. J. Med. Chem 22, 243-250 (1987)
- 38.: G.E. Martin e.a., J. Med. Chem. 32, 1056 (1989)
- 39.: Z. Budai, Hpat. HU 61737 (1991)
- 40.: D.W. Smith, Pat. EP 345808 (1988), US 204845 (1988), US 338253 (1989)
- 41.: J.P. Yevich, EP 400661 (1990), US 360657 (1989), US 503197 (1990)
- 42.: T.F. Braish, Pat.EP 397351 (1990), US 350423 (1989), US 423063 (1989)
- 43.: am 15. Oktober 1995 bekanntgemachte österr. Patentanmeldung 1980/94 vom 21. Oktober 1994
- 44.: GL. Ellmann K.D. Courtney, V. Andres, R.M. Featherstone, Biochem, Pharmacol. 7, 88 (1961).

## Patentansprüche

1. Verbindungen der folgenden Formeln:
| BEISPIEL | FORMELBILD | FORSCHUNGS-CODE | IC50 AChEI | IC50 BuChEI |
|---|---|---|---|---|
| 42 | 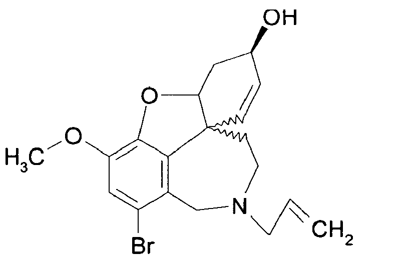 | SPH-1097 | 200 | 2.6 |
| 48 | 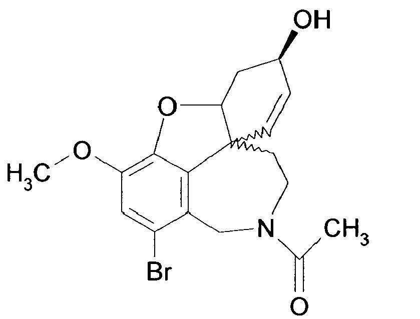 | SPH-1071 | 49 | 14 |
| 49 | 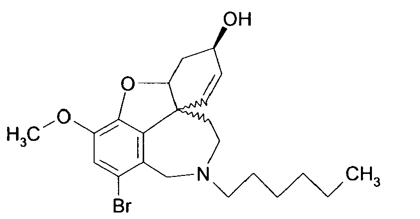 | SPH-1054 | 12 | 0.2 |
| 50 | 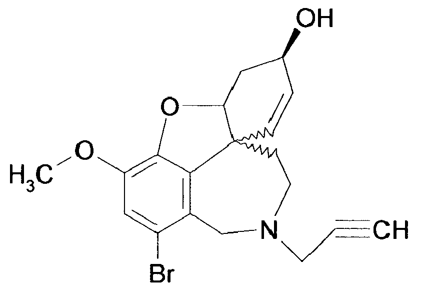 | SPH-1075 | 63 | 10 |
| 51 | 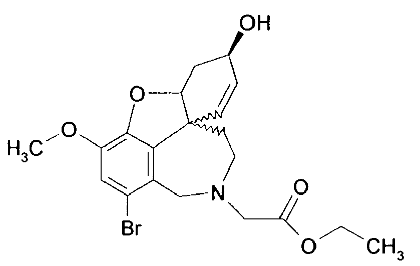 | SPH-1080 | 200 | 56 |
| 52 | 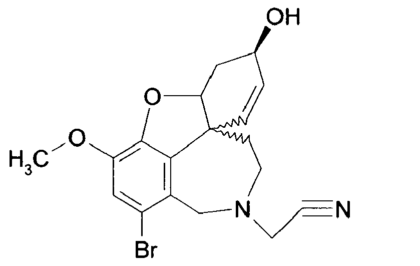 | SPH-1069 | 200 | 70 |
| 53 | 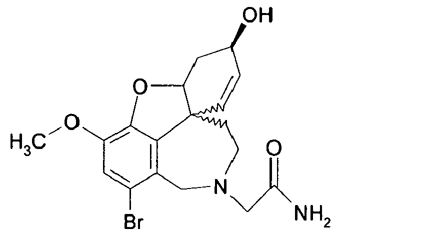 | SPH-1081 | 200 | 15 |
| 54 | 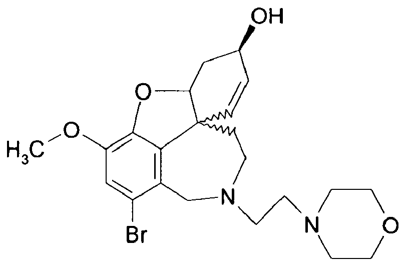 | SPH-1078 | 200 | 4.4 |
| 58 | 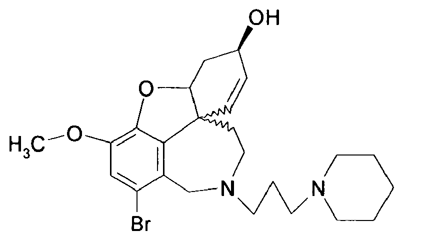 | SPH-1106 | 23 | 3.3 |
| 60 | 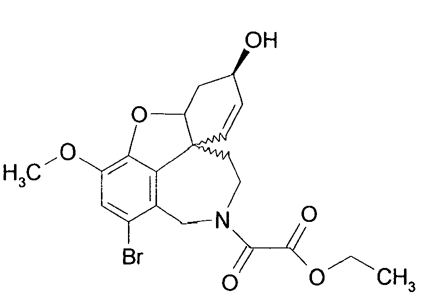 | SPH-1070 | 32.5 | 11 |
| 61 | 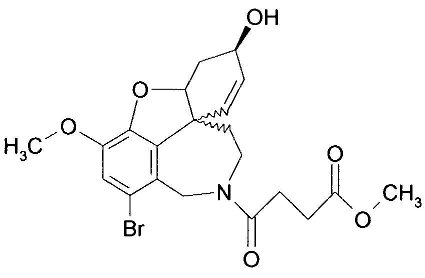 | SPH-1072 | 200 | 200 |
| 62 | 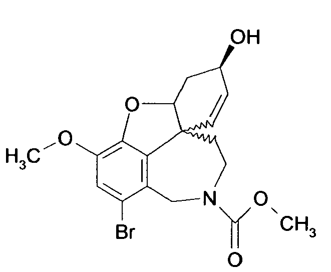 | SPH-1082 | 200 | 200 |
| 66 | 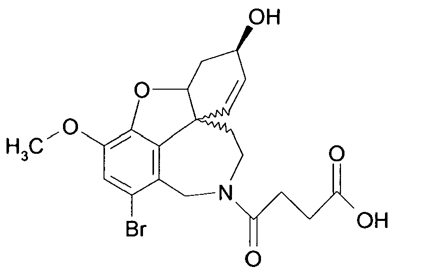 | SPH-1090 | 200 | 200 |
| 68 | 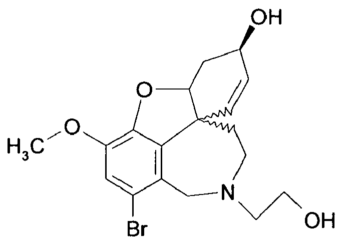 | SPH-1095 | 34 | 6.35 |
| 69 | 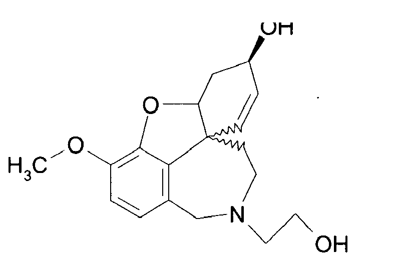 | SPH-1096 | 15 | 19 |
| 71 | 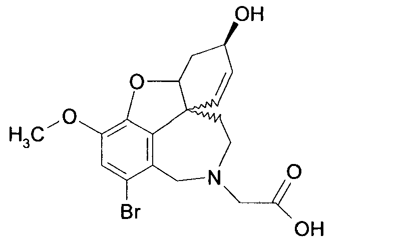 | SPH-1089 | 200 | 200 |
| 73 | 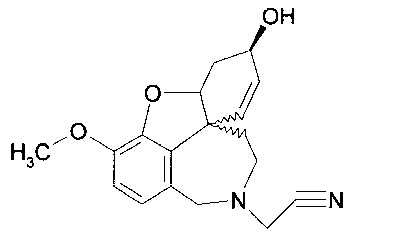 | SPH-1099 | 37 | 200 |
| 74 | 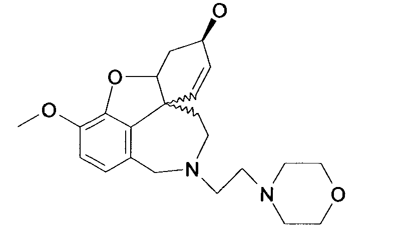 | SPH-1098 | 13 | 7 |
| 92 | 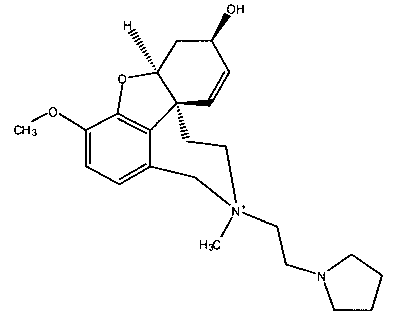 | SPH-1023 | 60 | 8 |
| 93 | 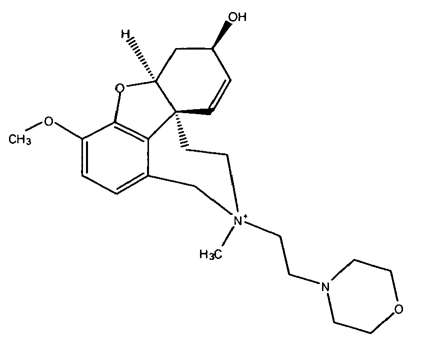 | SPH-1098 (rac) | 13 (rac) | 7 (rac) |
| 94 | 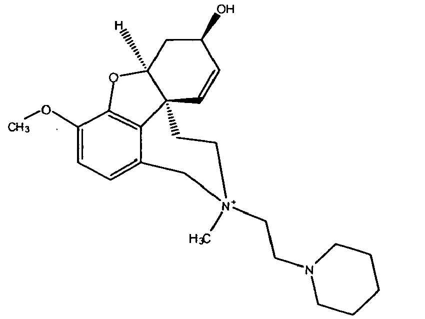 | SPH-1144 | 6,2 | 3,6 |
| 95 | 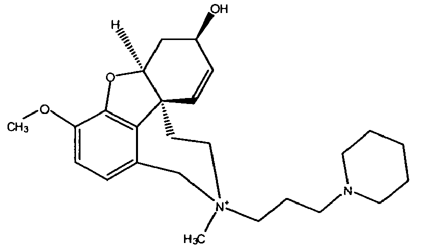 | SPH-1019 | 30 | 5,6 |
| 98 | 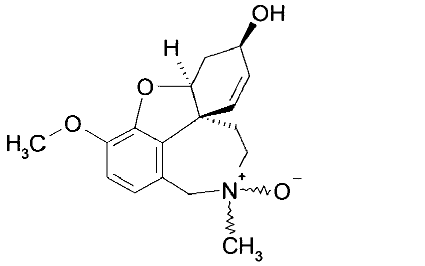 | SPH-1052 | 200 | 200 |
| 99 | 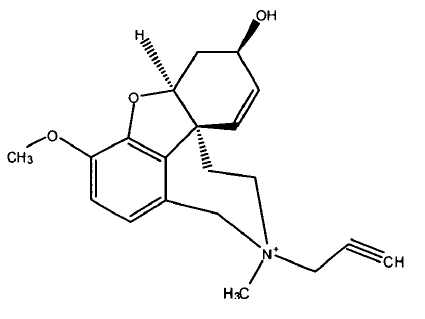 | SPH-1058 | 1,35 | 1,6 |
| 100 | 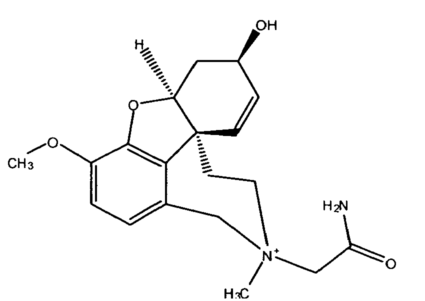 | SPH-1140 | 3,1 | 2,5 |
| 105 | 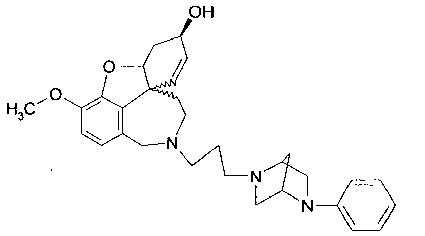 | SPH-1195 | 24.5 | 7.5 |
| 109 | 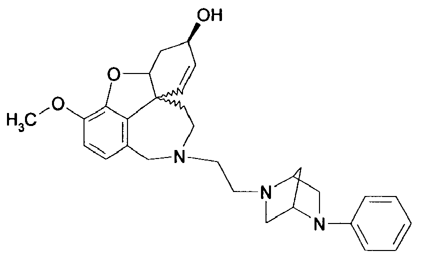 | SPH-1329 | 2.9 | 0.9 |

2. Arzneimittel, **dadurch gekennzeichnet, dass** es als pharmazeutischen Wirkstoff eine, zwei oder mehrere der Verbindungen gemäß Anspruch 1 enthält.

3. Verwendung einer, zweier oder mehrerer Verbindungen gemäß Anspruch 1, deren Enantiomere und/oder pharmazeutisch annehmbare Säureadditionssalze zum Herstellen eines Arzneimittels für die Behandlung der Alzheimer'schen Krankheit und verwandter Demenzzustände.

4. Verwendung einer, zweier oder mehrerer Verbindungen gemäß Anspruch 1, deren Enantiomere und/oder pharmazeutisch annehmbare Säureadditionssalze zum Herstellen eines Arzneimittels für die Behandlung von Trisomie 21 oder verwandter Trisomiesyndrome.
